# EUROPEAN PATENT SPECIFICATION

(11) **EP 2 114 863 B1**
(45) Date of publication and mention of the grant of the patent: **05.10.2016**
(21) Application number: 08725968.5
(22) Date of filing: 21.02.2008
(51) Int. Cl.: C07C 213/10, C07C 215/64

(54) **SOLID FORMS COMPRISING (-)-O-DESMETHYLVENLAFAXINE AND USES THEREOF**
FESTE FORMEN MIT (-)-O-DESMETHYLVENLAFAXIN UND VERWENDUNGEN DAVON
FORMES SOLIDES COMPRENANT DU (-)-O-DESMETHYLVENLAFAXINE ET UTILISATIONS DE CELLES-CI

(30) Priority: 21.02.2007 US 902950 P
(43) Date of publication of application: 11.11.2009
(60) Divisional application: 12164795.2
(73) Proprietor: Sepracor Inc., Marlborough, Massachusetts 01752 (US)
(72) Inventor: SIZENSKY, Michael, South Grafton, MA 01560 (US); WILKINSON, Harold, S., Westborough, MA 01581 (US); SNOONIAN, John, Ayer, MA 01432 (US); KIM, Norman, Westford, MA 01886 (US); LAUGHLIN, Sharon, M., Hudson, MA 01749 (US); BAKALE, Roger, P., Shrewsbury, MA 01545 (US); PLUNKETT, Kevin, Walpole, MA 02081 (US); MOUSAW, Patrick, Granger, Indiana 46530 (US)
(74) Representative: Tostmann, Holger Carl
(86) International application number: PCT/US2008/002379
(87) International publication number: WO 2008/103461

(56) References cited:
- WO-A-00/76955
- WO-A-2007/005961
- US-A1- 2006 199 860
- US-B1- 6 342 533

## Description

This application claims the benefit of U.S. provisional application 60/902,950, filed February 21, 2007.

### 1. FIELD OF THE INVENTION

The present invention relates to the solid form according to claim 1 comprising stereomerically pure (-)-O-desmethylvenlafaxine, including salts thereof, compositions comprising the solid form, methods of making the solid use in the manufacture of a medicament for the form and its treatment of various diseases and/or disorders.

### 2. BACKGROUND OF THE INVENTION

Each pharmaceutical compound has an optimal therapeutic blood concentration and a lethal concentration. The bioavailability of the compound determines the dosage strength in the drug formulation necessary to obtain the ideal blood level. If the drug can crystallize as two or more crystal forms differing in bioavailability, the optimal dose will depend on the crystal form present in the formulation. Some drugs show a narrow margin between therapeutic and lethal concentrations. Chloramphenicol-3-palmitate (CAPP), for example, is a broad-spectrum antibiotic known to crystallize in at least three polymorphic crystal forms and one amorphous form. The most stable form, A, is marketed. The difference in bioactivity between this polymorph and another form, B, is a factor of eight, thus creating the possibility of fatal overdosages of the compound if unwittingly administered as Form B due to alterations during processing and/or storage. Therefore, regulatory agencies, such as the United States Food and Drug Administration, have begun to place tight controls on the polymorphic content of the active component in solid dosage forms. In general, for drugs that exist in polymorphic forms, if anything other than the pure, thermodynamically preferred polymorph is to be marketed, the regulatory agency may require batch-by-batch monitoring. Thus, it becomes important for both medical and commercial reasons to produce and market the pure drug in its most thermodynamically stable polymorph, substantially free of other kinetically favored polymorphs.

New solid forms of a pharmaceutical agent can further the development of formulations for the treatment of illnesses. For instance, solid forms of salts of a compound are known in the pharmaceutical art to affect, for example, the solubility, dissolution rate, bioavailability, chemical and physical stability, flowability, fractability, and compressibility of the compound as well as the safety and efficacy of drug products based on the compound (see, e.g., Byrn, S.R., Pfeiffer, R.R., and Stowell, J.G. (1999) Solid-State Chemistry of Drugs, 2nd ed., SSCl, Inc.: West Lafayette, IN).

Accordingly, identification of a solid form comprising a salt or free base of a compound with optimal physical and chemical properties will advance the development of the compound as a pharmaceutical. Useful physical and chemical properties include: reproducible preparation, non-hygroscopicity, aqueous solubility, stability to visible and ultraviolet light, low rate of degradation under accelerated stability conditions of temperature and humidity, low rate of isomerization of between isomeric forms, and safety for long-term administration to humans. Crystallinity is often desirable, although in some instances enhanced dissociation profiles may be attained via preparation of an amorphous form.

O-desmethylvenlafaxine, chemically named 1-[2-(dimethylamino)-1-(4-hydroxyphenyl)ethyl]cyclohexanol, is a metabolite of the compound venlafaxine, a hydrochloride salt of which is currently commercially available under the trade name Effexor^{®}. Effexor^{®}, which is a racemic mixture of the (+) and (-) enantiomers of venlafaxine, is indicated for the treatment of depression. Racemic O-desmethylvenlafaxine has been exemplified as a fumarate salt in U.S. Patent No. 4,535,186, and a succinate and formate salts were disclosed in U.S. Patent Nos. 6,673,838 and 7,001,920, respectively. Stereomerically pure (-)-O-desmethylvenlafaxine and its pharmaceutically acceptable salts have been disclosed in U.S. Patent Nos. 6,342,533 B1, 6,441,048 B1 and 6,911,479 B2 as well as in US 2006/199869,WO 2007/005961 and WO 00/76955

We have discovered that not all of the solid forms comprising (-)-O-desmethylvenlafaxine, including salts thereof, are equally useful, as assessed by the list of properties described above. Thus, the present invention addresses the need for improved solid forms comprising (-)-O-desmethylvenlafaxine for, *e.g.,* manufacturing and formulation.

### 3. SUMMARY OF THE INVENTION

The present invention provides a novel solid form in accordance with claim 1, having particular utility in the manufacture of a medicament for use in the treatment, prevention or management of conditions and disorders including, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4th edition (DSM-IV).

Without intending to be limited by any particular theory, the storage stability, compressibility, bulk density or dissolution properties of the solid form is believed to be beneficial for manufacturing, formulation and bioavailability of (-)-O-desmethylvenlafaxine in accordance with claim 1. The invention also provides a pharmaceutical composition comprising the solid form in accordance with claim 1 and its use in the manufacture of a medicament for bioavailability of (-)-O-desmethylvenlafaxine and salts thereof. In certain embodiments, the form and its treatment, prevention and/or management of conditions and disorders including, but not limited to, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4^{th} edition (DSM-IV).

In certain embodiments, the form in accordance with claim 1 is used to treat, prevent and/or manage the above-described conditions and disorders while reducing or avoiding adverse effects including, sustained hypertension, headache, asthenia, sweating, nausea, constipation, somnolence, dry mouth, dizziness, insomnia, nervousness, anxiety, blurred or blurry vision, and abnormal ejaculation/orgasm or impotence in males.

The solid form is prepared from (-)-O-desmethylvenlafaxine, which is described in U.S. Patent Nos. 6,342,533 B1, 6,441,048 B1 and 6,911,479 B2. (-)-O-desmethylvenlafaxine has the following structure (I):

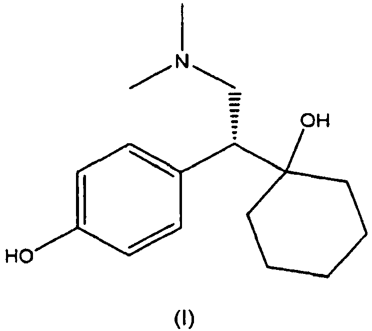

The present invention provides crystalline salts of (-)-O-desmethylvenlafaxine in particular crystalline hydrochloride salts of (-)-O-desmethylvenlafaxine. In certain embodiments, crystalline hydrochloride salts of (-)-O-desmethylvenlafaxine possess unexpected excellent properties, described in detail below. The present invention provides the polymorph of claim 1 of the hydrochloric acid salts of (-)-O-desmethylvenlafaxine. In accordance with claim 1, the present invention provides hydrates of the hydrochloride salts of (-)-O-desmethylvenlafaxine.

In certain embodiments, the present invention provides pharmaceutical compositions comprising a crystal form of claim 1 and/or a pharmaceutically acceptable diluent, excipient or carrier. In certain embodiments, the present invention further provides the pharmaceutical composition for use in the treatment, prevention and/or management of one or more of the following conditions or disorders: affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4th edition (DSM-IV), wherein such uses comprise administering to a subject, e.g., a human, in need of such treatment, prevention and/or management a therapeutically and/or prophylactically effective amount of solid form of the invention. The present invention also provides the pharmaceutical composition for use in the treatment, prevention and/or management of conditions and disorders including, but not limited to, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4^{th} edition (DSM-IV), comprising administering to a subject, e.g., a human, in need of such treatment, prevention or management a /or and prophylactically effective amount of a solid form of the invention.

In certain embodiments, the present disclosure provides methods of making, isolating and/or characterizing the solid forms of the invention.

In certain embodiments, the novel solid form of the invention is useful as active pharmaceutical ingredients for the preparation of formulations for use in animals or humans. In certain embodiments, the present invention encompasses the use of this solid form as a final drug product. In certain embodiments, the solid form of the invention is useful, for example, for the treatment, prevention or management of conditions and disorders listed above.

### 4. BRIEF DESCRIPTION OF THE DRAWINGS

FIG. 1 provides a thermal gravimetric analysis thermogram of a sample comprising Form A of the hydrochloride salt of (-)-O-desmethylvenlafaxine;
FIG. 2 provides a differential scanning calorimetry thermogram of a sample comprising Form A of the hydrochloride salt of (-)-O-desmethylvenlafaxine;
FIG. 3 provides an X-ray powder diffraction pattern of a sample comprising Form A of the hydrochloride salt of (-)-O-desmethylvenlafaxine;
FIG. 4 provides an infrared spectrum of a sample comprising Form A of the hydrochloride salt of (-)-O-desmethylvenlafaxine;
FIG. 5 provides a Raman spectrum of a sample comprising Form A of the hydrochloride salt of (-)-O-desmethylvenlafaxine;
FIG. 6 provides a moisture sorption isotherm of a sample comprising Form A of the hydrochloride salt of (-)-O-desmethylvenlafaxine;
FIG. 7 provides the asymmetric unit of the crystal structure of Form A obtained by single-crystal X-ray diffraction on a sample comprising Form A of the hydrochloride salt of (-)-O-desmethylvenlafaxine;
FIG. 8 provides an X-ray powder diffraction pattern simulated from single crystal X-ray diffraction data obtained on a sample comprising Form A of the hydrochloride salt of (-)-O-desmethylvenlafaxine;
FIG. 9 provides a thermal gravimetric analysis thermogram of a sample comprising Form B of the hydrochloride salt of (-)-O-desmethylvenlafaxine;
FIG. 10 provides a differential scanning calorimetry thermogram of a sample comprising Form B of the hydrochloride salt of (-)-O-desmethylvenlafaxine;
FIG. 11 provides an X-ray powder diffraction pattern of a sample comprising Form B of the hydrochloride salt of (-)-O-desmethylvenlafaxine;
FIG. 12 provides an infrared spectrum of a sample comprising Form B of the hydrochloride salt of (-)-O-desmethylvenlafaxine;
FIG. 13 provides a Raman spectrum of a sample comprising Form B of the hydrochloride salt of (-)-O-desmethylvenlafaxine;
FIG. 14 provides a moisture sorption isotherm of a sample comprising Form B of the hydrochloride salt of (-)-O-desmethylvenlafaxine;
FIG. 15 provides a thermal gravimetric analysis thermogram of a sample comprising Form C of the hydrochloride salt of (-)-O-desmethylvenlafaxine;
FIG. 16 provides a differential scanning calorimetry thermogram of a sample comprising Form C of the hydrochloride salt of (-)-O-desmethylvenlafaxine;
FIG. 17 provides an X-ray powder diffraction pattern of a sample comprising Form C of the hydrochloride salt of (-)-O-desmethylvenlafaxine;
FIG. 18 provides an infrared spectrum of a sample comprising Form C of the hydrochloride salt of (-)-O-desmethylvenlafaxine;
FIG. 19 provides a Raman spectrum of a sample comprising Form C of the hydrochloride salt of (-)-O-desmethylvenlafaxine;
FIG. 20 provides a moisture sorption isotherm of a sample comprising Form C of the hydrochloride salt of (-)-O-desmethylvenlafaxine;
FIG. 21 provides a thermal gravimetric analysis thermogram of a sample comprising Form D of the hydrochloride salt of (-)-O-desmethylvenlafaxine;
FIG. 22 provides a differential scanning carlorimetry thermogram of a sample comprising Form D of the hydrochloride salt of (-)-O-desmethylvenlafaxine;
FIG. 23 provides an X-ray powder diffraction pattern of a sample comprising Form D of the hydrochloride salt of (-)-O-desmethylvenlafaxine;
FIG. 24 provides a thermal gravimetric analysis thermogram of a sample comprising Form E of the hydrochloride salt of (-)-O-desmethylvenlafaxine;
FIG. 25 provides a differential scanning calorimetry thermogram of a sample comprising Form E of the hydrochloride salt of (-)-O-desmethylvenlafaxine;
FIG. 26 provides an X-ray powder diffraction pattern of a sample comprising Form E of the hydrochloride salt of (-)-O-desmethylvenlafaxine;
FIG. 27 provides an infrared spectrum of a sample comprising Form E of the hydrochloride salt of (-)-O-desmethylvenlafaxine;
FIG. 28 provides a Raman spectrum of a sample comprising Form E of the hydrochloride salt of (-)-O-desmethylvenlafaxine;
FIG. 29 provides a moisture sorption isotherm of a sample comprising Form E of the hydrochloride salt of (-)-O-desmethylvenlafaxine;
FIG. 30 provides a thermal gravimetric analysis thermogram of a sample comprising Form F of the hydrochloride salt of (-)-O-desmethylvenlafaxine;
FIG. 31 provides a differential scanning calorimetry thermogram of a sample comprising Form F of the hydrochloride salt of (-)-O-desmethylvenlafaxine;
FIG. 32 provides an X-ray powder diffraction pattern of a sample comprising Form F of the hydrochloride salt of (-)-O-desmethylvenlafaxine;
FIG. 33 provides an X-ray powder diffraction pattern simulated from single crystal X-ray diffraction data obtained on a sample comprising Form F of the hydrochloride salt of (-)-O-desmethylvenlafaxine;
FIG. 34 provides an infrared spectrum of a sample comprising Form F of the hydrochloride salt of (-)-O-desmethylvenlafaxine;
FIG. 35 provides a Raman spectrum of a sample comprising Form F of the hydrochloride salt of (-)-O-desmethylvenlafaxine;
FIG. 36 provides moisture sorption isotherm of a sample comprising Form F of the hydrochloride salt of (-)-O-desmethylvenlafaxine;
FIG. 37 provides the asymmetric unit of the crystal structure of Form F obtained by single-crystal X-ray diffraction on a sample comprising Form F of the hydrochloride salt of (-)-O-desmethylvenlafaxine;
FIG. 38 provides a thermal gravimetric analysis thermogram of a sample comprising Form G of the hydrochloride salt of (-)-O-desmethylvenlafaxine;
FIG. 39 provides a differential scanning calorimetry thermogram of a sample comprising Form G of the hydrochloride salt of (-)-O-desmethylvenlafaxine;
FIG. 40 provides an X-ray powder diffraction pattern of a sample comprising Form G of the hydrochloride salt of (-)-O-desmethylvenlafaxine;
FIG. 41 provides a moisture sorption isotherm of a sample comprising Form G of the hydrochloride salt of (-)-O-desmethylvenlafaxine;
FIG. 42 provides a thermal gravimetric analysis thermogram of a sample comprising Form H of the hydrochloride salt of (-)-O-desmethylvenlafaxine;
FIG. 43 provides a differential scanning calorimetry thermogram of a sample comprising Form H of the hydrochloride salt of (-)-O-desmethylvenlafaxine;
FIG. 44 provides an X-ray powder diffraction pattern of a sample comprising Form H of the hydrochloride salt of (-)-O-desmethylvenlafaxine;
FIG. 45 provides a thermal gravimetric analysis thermogram of a sample comprising Form I of the hydrochloride salt of (-)-O-desmethylvenlafaxine;
FIG. 46 provides a differential scanning calorimetry thermogram of a sample comprising Form I of the hydrochloride salt of (-)-O-desmethylvenlafaxine;
FIG. 47 provides an X-ray powder diffraction pattern of a sample comprising Form I of the hydrochloride salt of (-)-O-desmethylvenlafaxine;
FIG. 48 provides an X-ray powder diffraction pattern of a sample comprising Form J of the hydrochloride salt of (-)-O-desmethylvenlafaxine;
FIG. 49 provides an X-ray powder diffraction pattern of a sample comprising Form K of the hydrochloride salt of (-)-O-desmethylvenlafaxine;
FIG. 50 provides an X-ray powder diffraction pattern simulated from single crystal X-ray diffraction data obtained on a sample comprising Form K of the hydrochloride salt of (-)-O-desmethylvenlafaxine;
FIG. 51 provides a thermal gravimetric analysis thermogram of a sample comprising Form L of the hydrochloride salt of (-)-O-desmethylvenlafaxine;
FIG. 52 provides a differential scanning calorimetry thermogram of a sample comprising Form L of the hydrochloride salt of (-)-O-desmethylvenlafaxine;
FIG. 53 provides an X-ray powder diffraction pattern of a sample comprising Form L of the hydrochloride salt of (-)-O-desmethylvenlafaxine;
FIG. 54 provides an X-ray powder diffraction pattern of a sample comprising a desolvated solvate belonging to isostructural family 1 of the hydrochloride salt of (-)-O-desmethylvenlafaxine;
FIG. 55 provides a thermal gravimetric analysis thermogram of a sample comprising an amorphous form of the hydrochloride salt of (-)-O-desmethylvenlafaxine;
FIG. 56 provides a modulated differential scanning calorimetry thermogram of a sample comprising an amorphous form of the hydrochloride salt of (-)-O-desmethylvenlafaxine;
FIG. 57 provides an X-ray powder diffraction pattern of a sample comprising an amorphous form of the hydrochloride salt of (-)-O-desmethylvenlafaxine;
FIG. 58 provides a moisture sorption isotherm of a sample comprising an amorphous form of the hydrochloride salt of (-)-O-desmethylvenlafaxine.

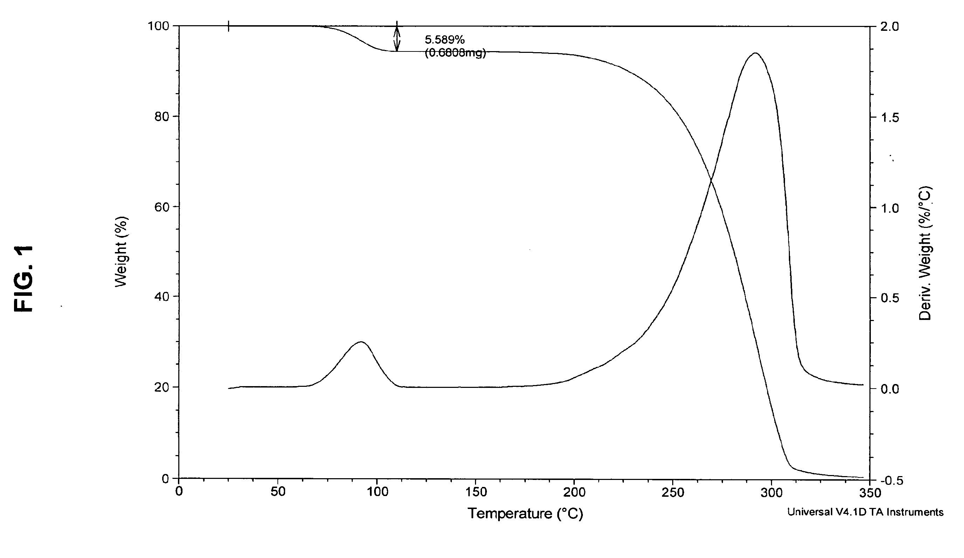

Figures 1 through 8 illustrate embodiments in accordance with the present invention, while the other Figures are for reference or comparison.

### 5. DETAILED DESCRIPTION OF THE INVENTION

### 5.1 DEFINITIONS

As used herein, the term (-)-O-desmethylvenlafaxine means the compound that is chemically named (-)-1-[2-(dimethylamino)-1-(4-hydroxyphenyl)ethyl] cyclohexanol.

As used herein, the term "pharmaceutically acceptable salts" refers to salts prepared from pharmaceutically acceptable, relatively non-toxic acids, including inorganic acids and organic acids. Suitable acids include acetic, benzenesulfonic, benzoic, camphorsulfonic, carbonic, citric, dihydrogenphosphoric, ethenesulfonic, fumaric, galactunoric, gluconic, glucuronic, glutamic, hydrobromic, hydrochloric, hydriodic, isobutyric, isethionic, lactic, maleic, malic, malonic, mandelic, methanesulfonic, monohydrogencarbonic, monohydrogenphosphoric, monohydrogensulfuric, mucic, nitric, pamoic, pantothenic, phosphoric, phthalic, propionic, suberic, succinic, sulfuric, tartaric, toluenesulfonic, including p-toluenesulfonic m-toluenesulfonic and o-toluenesulfonic acids, and the like (see, e.g., Berge et al., J. Pharm. Sci., 66:1-19 (1977); Stahl and Wermuth, Handbook of Pharmaceutical Salts, Wiley VCH, (2002)). Also included are salts of other relatively non-toxic compounds that possess acidic character, including amino acids, such as arginine and the like, and other compounds, such as aspirin, ibuprofen, saccharin, and the like. Particularly preferred are hydrochloric, hydrobromic, methanesulfonic, and sulfuric acids, and most particularly preferred is the hydrochloride salt. Acid addition salts can be obtained by contacting the neutral form of such compounds with a sufficient amount of the desired acid, either neat or in a suitable inert solvent. As solids, salts can exist in crystalline and/or amorphous modifications.

Particular salts described below include "hydrochloride salts," "hydrochloric acid salts," and "HCl salts" of (-)-O-desmethylvenlafaxine of the invention. A hydrochloride salt, hydrochloric acid salt or HCl salt is an acid addition salt formed using hydrochloric acid.

The term "solid forms" and related terms used herein, unless otherwise specified, refers to crystal forms and amorphous forms comprising (-)-O-desmethylvenlafaxine, and specifically includes crystal forms and amorphous forms comprising salts of (-)-O-desmethylvenlafaxine.

The term "crystalline" and related terms used herein, when used to describe a substance, component or product, means that the substance, component or product is crystalline as determined by X-ray diffraction. See, *e.g.,* Remington's Pharmaceutical Sciences, 18th ed., Mack Publishing, Easton PA, 173 (1990); The United States Pharmacopeia, 23rd ed., 1843-1844 (1995).

The term "crystal forms" and related terms herein refers to the various crystalline modifications of a given substance, including, but not limited to, polymorphs, solvates, hydrates, co-crystals and other molecular complexes, as well as salts, solvates of salts, hydrates of salts, other molecular complexes of salts, and polymorphs thereof. Crystal forms of a substance can be obtained by a number of methods, as known in the art. Such methods include, but are not limited to, melt recrystallization, melt cooling, solvent recrystallization, recrystallization in confined spaces such as, e.g., in nanopores or capillaries, recrystallization on surfaces or templates such as, e.g., on polymers, recrystallization in the presence of additives, such as, e.g., co-crystal counter-molecules, desolvation, dehydration, rapid evaporation, rapid cooling, slow cooling, vapor diffusion, sublimation, grinding and solvent-drop grinding.

The terms "polymorphs," "polymorphic forms" and related terms herein refer to two or more crystal forms that are composed of the same molecule, molecules or ions. Different polymorphs may have different physical properties such as, for example, melting temperatures, heats of fusion, solubilities, dissolution rates and/or vibrational spectra as a result of the arrangement or conformation of the molecules or ions in the crystal lattice (see, e.g., Byrn, S.R., Pfeiffer, R.R., and Stowell, J.G. (1999) Solid-State Chemistry of Drugs, 2nd ed., SSCI, Inc.: West Lafayette, IN). The differences in physical properties exhibited by polymorphs affect pharmaceutical parameters such as storage stability, compressibility and density (important in formulation and product manufacturing), and dissolution rate (an important factor in bioavailability). Differences in stability can result from changes in chemical reactivity (e.g., differential oxidation, such that a dosage form discolors more rapidly when comprised of one polymorph than when comprised of another polymorph) or mechanical changes (e.g., tablets crumble on storage as a kinetically favored polymorph converts to thermodynamically more stable polymorph) or both (e.g., tablets of one polymorph are more susceptible to breakdown at high humidity). As a result of solubility/dissolution differences, in the extreme case, some polymorphic transitions may result in lack of potency or, at the other extreme, toxicity. In addition, the physical properties of the crystal may be important in processing, for example, one polymorph might be more likely to form solvates or might be difficult to filter and wash free of impurities (*i*.*e*., particle shape and size distribution might be different between polymorphs).

The term "solvate" and "solvated," as used herein, refer to a crystal form of a substance which contains solvent. The term "hydrate" and "hydrated" refer to a solvate wherein the solvent is water. "Polymorphs of solvates" refers to the existence of more than one crystal form for a particular solvate composition. Similarly, "polymorphs of hydrates" refers to the existence of more than one crystal form for a particular hydrate composition.

The term "desolvated solvate," as used herein, refers to a crystal form of a substance which can be prepared by removing the solvent from a solvate.

The term "isostructural family," as used herein, refers to a series of two or more crystal forms of a substance which have a common structural similarity, including approximately similar interplanar spacing in the crystal lattice. (A more detailed account of crystal lattices can be found in Chapters 2 and 3 of Stout and Jensen, X-Ray Structure Determination: A Practical Guide, MacMillan Co., New York (1968)). Due to their common structural similarity, members of an isostructural family of crystal forms typically have similar, but not necessarily identical, X-ray powder diffraction patterns. An isostructural family may be based upon a substance that is a neutral molecule, a salt or a molecular complex. The series may be composed of solvates, including hydrates, and desolvated solvate crystal forms of the substance. Solvated members of an isostructural family of crystal forms typically contain one or more solvents, including water, in the crystal lattice. The solvent or solvents in the crystal lattice may be the solvent or solvents of crystallization used in preparing the crystal form. Typical solvents of crystallization include water and all classes of organic and other types of laboratory solvents, including, but not limited to: alcohols, such as methanol, ethanol, *n*-propanol, isopropanol, *n*-butanol, *sec*-butanol, *t*-butanol, hydroxyphenyl, glycerol, and the like; carbonyl-containing solvents, such as acetone, methyl ethyl ketone, formic acid, acetic acid, ethyl acetate, butyl acetate, *N*,*N*-dimethylformamide, and the like; hydrocarbons, such as pentane, hexane, cyclohexane, benzene, toluene, xylenes, and the like; halogenated solvents, such as dichlormethane, chloroform, carbon tetrachloride, and the like; and laboratory solvents containing other heteroatoms and/or functional groups, such as acetonitrile, tetrahydrofuran, diethyl ether, diisopropyl ether, carbon disulfide, dimethyl sulfoxide, 1,4-dioxane, nitrobenzene, nitromethane, pyridine, and the like.

The term "amorphous," "amorphous form," and related terms used herein mean that the material, substance, component or product under consideration is not crystalline as determined by X-ray diffraction. Amorphous forms of a substance can be obtained by a number of methods, as known in the art. Such methods include, but are not limited to, heating, melt cooling, rapid melt cooling, solvent evaporation, rapid solvent evaporation, desolvation, sublimation, grinding, cryo-grinding and freeze drying.

Techniques for characterizing crystal forms and amorphous forms include, but are not limited to, thermal gravimetric analysis (TGA), differential scanning calorimetry (DSC), X-ray powder diffractometry (XRPD), single crystal X-ray diffractometry, vibrational spectroscopy, e.g., infrared (IR) and Raman spectroscopy, solid-state NMR, optical microscopy, hot stage optical microscopy, scanning electron microscopy (SEM), electron crystallography and quantitative analysis, particle size analysis (PSA), surface area analysis, solubility studies and dissolution studies.

As used herein, and unless otherwise specified, the terms "about" and "approximately," when used in connection with doses, amounts, or weight percent of ingredients of a composition or a dosage form, mean a dose, amount, or weight percent that is recognized by those of ordinary skill in the art to provide a pharmacological effect equivalent to that obtained from the specified dose, amount, or weight percent. Specifically, the terms "about" and "approximately," when used in this context, contemplate a dose, amount, or weight percent within 15%, within 10%, within 5%, within 4%, within 3%, within 2%, within 1%, or within 0.5% of the specified dose, amount, or weight percent.

As used herein, and unless otherwise specified, the terms "about" and "approximately," when used in connection with a numeric value or range of values which is provided to describe a particular solid form, e.g., a specific temperature or temperature range, such as, for example, that describing a melting, dehydration, desolvation or glass transition; a mass change, such as, for example, a mass change as a function of temperature or humidity; a solvent or water content, in terms of, for example, mass or a percentage; or a peak position, such as, for example, in analysis by IR or Raman spectroscopy or XRPD; indicate that the value or range of values may deviate to an extent deemed reasonable to one of ordinary skill in the art while still describing the particular solid form. Specifically, the terms "about" and "approximately," when used in this context, indicate that the numeric value or range of values may vary by 20%, 10%, 9%, 8%, 7%, 6%, 5%, 4%, 3%, 2%, 1%, 0.9%, 0.8%, 0.7%, 0.6%, 0.5%, 0.4%, 0.3%, 0.2% or 0.1 % of the recited value or range of values while still describing the particular solid form.

As used herein and unless otherwise indicated, the term "stereomerically pure" means a composition that comprises one stereoisomer of a compound and is substantially free of other stereoisomers of that compound. For example, a stereomerically pure composition of a compound having one chiral center will be substantially free of the opposite enantiomer of the compound. A stereomerically pure composition of a compound having two chiral centers will be substantially free of other diastereomers of the compound. In certain embodiments, a stereomerically pure compound comprises greater than about 80 percent by weight of one stereoisomer of the compound and less than about 20 percent by weight of other stereoisomers of the compound, greater than about 90 percent by weight of one stereoisomer of the compound and less than about 10 percent by weight of the other stereoisomers of the compound, greater than about 95 percent by weight of one stereoisomer of the compound and less than about 5 percent by weight of the other stereoisomers of the compound, greater than about 97 percent by weight of one stereoisomer of the compound and less than about 3 percent by weight of the other stereoisomers or greater than about 99 percent by weight of one stereoisomer of the compound and less than about 1 percent by weight of the other stereoisomers of the compound.

As used herein and unless otherwise indicated, the term "enantiomerically pure" means a stereomerically pure composition of a compound having one chiral center.

As used herein to describe a compound, the term "substantially free of its (+) stereoisomer" means that the compound is made up of a significantly greater proportion of its (-) stereoisomer than of its optical antipode (*i*.*e*., its (+) stereoisomer). In certain embodiments of the invention, the term "substantially free of its (+) stereoisomer" means that the compound is made up of at least about 90% by weight of its (-) stereoisomer and about 10% by weight or less of its (+) stereoisomer. In certain embodiments of the invention, the term "substantially free of its (+) stereoisomer" means that the compound is made up of at least about 95% by weight of its (-) stereoisomer and about 5% by weight or less of its (+) stereoisomer. In certain embodiments, the term "substantially free of its (+) stereoisomer" means that the compound is made up of at least about 99% by weight of its (-) stereoisomer and about 1% or less of its (+) stereoisomer. In certain embodiments, the term "substantially free of its (+) stereoisomer" means that the compound is made up of approximately 100% by weight of its (-) stereoisomer. The above percentages are based on the total amount of the combined stereoisomers of the compound. The terms "substantially optically pure (-)-O-desmethylvenlafaxine," "optically pure (-)-O-desmethylvenlafaxine" and "(-) isomer of O-desmethylvenlafaxine" all refer to (-)-O-desmethylvenlafaxine that is substantially free of its (+) stereoisomer. The terms "substantially optically pure (-)-O-desmethylvenlafaxine," "optically pure (-)-O-desmethylvenlafaxine" and "(-) isomer of O-desmethylvenlafaxine" all refer to (-)-O-desmethylvenlafaxine that is substantially free of its (+) stereoisomer.

As used herein, a crystalline or amorphous form that is "pure," i.e., substantially free of other crystalline or amorphous forms, contains less than about 10 percent by weight of one or more other crystalline or amorphous form, less than about 5 percent by weight of one or more other crystalline or amorphous form, less than about 3 percent by weight of one or more other crystalline or amorphous form, or less than about 1 percent by weight of one or more other crystalline or amorphous form.

As used herein and unless otherwise indicated, a composition that is "substantially free" of a compound means that the composition contains less than about 20 percent by weight, less than about 10 percent by weight, less than about 5 percent by weight, less than about 3 percent by weight, or less than about 1 percent by weight of the compound.

As used herein, and unless otherwise specified, the terms "treat," "treating" and "treatment" refer to the eradication or amelioration of a disease or disorder, or of one or more symptoms associated with the disease or disorder. In certain embodiments, the terms refer to minimizing the spread or worsening of the disease or disorder resulting from the administration of one or more prophylactic or therapeutic agents to a subject with such a disease or disorder. In some embodiments, the terms refer to the administration of a compound provided herein, with or without other additional active agent, after the onset of symptoms of the particular disease.

As used herein, and unless otherwise specified, the terms "prevent," "preventing" and "prevention" refer to the prevention of the onset, recurrence or spread of a disease or disorder, or of one or more symptoms thereof. In certain embodiments, the terms refer to the treatment with or administration of a compound provided herein, with or without other additional active compound, prior to the onset of symptoms, particularly to patients at risk of disease or disorders provided herein. The terms encompass the inhibition or reduction of a symptom of the particular disease. Patients with familial history of a disease in particular are candidates for preventive regimens in certain embodiments. In addition, patients who have a history of recurring symptoms are also potential candidates for the prevention. In this regard, the term "prevention" may be interchangeably used with the term "prophylactic treatment."

As used herein, and unless otherwise specified, the terms "manage," "managing" and "management" refer to preventing or slowing the progression, spread or worsening of a disease or disorder, or of one or more symptoms thereof. Often, the beneficial effects that a subject derives from a prophylactic and/or therapeutic agent do not result in a cure of the disease or disorder. In this regard, the term "managing" encompasses treating a patient who had suffered from the particular disease in an attempt to prevent or minimize the recurrence of the disease.

As used herein, the term "affective disorder" includes depression, attention deficit disorder, attention deficit disorder with hyperactivity, bipolar and manic conditions, and the like. The terms "attention deficit disorder" (ADD) and "attention deficit disorder with hyperactivity" (ADDH), or attention deficit/hyperactivity disorder (AD/HD), are used herein in accordance with the accepted meanings as found in the Diagnostic and Statistical Manual of Mental Disorders, 4th ed., American Psychiatric Association (1997) (DSM-IV™).

As used herein, the term treating depression" means relief from the symptoms of depression which include, but are not limited to, changes in mood, feelings of intense sadness, despair, mental slowing, loss of concentration, pessimistic worry, agitation, and self-deprecation. Physical changes may also be relieved, including insomnia, anorexia, weight loss, decreased energy and libido, and abnormal hormonal circadian rhythms.

As used herein, the term treating, preventing or managing obesity or weight gain" means reduction of weight, or prevention of or relief from being overweight, gaining weight, or obesity; all of which are usually due to extensive consumption of food.

As used herein, the term "treating, preventing or managing disorders ameliorated by inhibition of neuronal monoamine reuptake" means prevention of or relief from symptoms of disease states associated with abnormal neuronal monoamine levels; such symptoms are reduced by way of neuronal monoamine reuptake inhibition. Monoamines, the reuptake of which are inhibited by the compounds or compositions of the present invention, include, but are not limited to, noradrenaline (or norepinephrine), serotonin and dopamine. Disorders treated by neuronal monoamine reuptake inhibition include, but are not limited to, Parkinson's disease and epilepsy.

As used herein, the term "treating, preventing or managing Parkinson's disease" means prevention of or relief from the symptoms of Parkinson's disease which include, but are not limited to, slowly increasing disability in purposeful movement, tremors, bradykinesia, rigidity, and a disturbance of posture in humans.

As used herein, the term "treating, preventing or managing cerebral function disorders" means prevention of or relief from the disease states associated with cerebral function disorders involving intellectual deficits which include but are not limited to, senile dementia, Alzheimer's type dementia, memory loss, amnesia/amnestic syndrome, disturbances of consciousness, coma, lowering of attention, speech disorders, Parkinson's disease, Lennox syndrome, autism, hyperkinetic syndrome and schizophrenia. Also within the meaning of cerebral function disorders are disorders caused by cerebrovascular diseases including, but not limited to, cerebral infarction, cerebral bleeding, cerebral arteriosclerosis, cerebral venous thrombosis, head injuries, and the like and where symptoms include disturbances of consciousness, senile dementia, coma, lowering of attention, speech disorders, and the like.

The terms "obsessive-compulsive disorder," "substance abuse," "pre-menstrual syndrome," "anxiety," "eating disorders" and "migraine" are used herein in a manner consistent with their accepted meanings in the art. See, e.g., DSM-IV™. The terms "method of treating, preventing or managing," "method of treating," "method of preventing" and "method of managing" when used in connection with these disorders mean the amelioration, prevention or relief from the symptoms and/or effects associated with these disorders. Without being limited by any theory, the treatment, prevention or management of certain of these disorders may be related to the activity of the active ingredient(s) as inhibitors of serotonin uptake.

As used herein, the term "treating, preventing or managing incontinence" means prevention of or relief from the symptoms of incontinence including involuntary voiding of feces or urine, and dribbling or leakage or feces or urine which may be due to one or more causes including but not limited to pathology altering sphincter control, loss of cognitive function, overdistention of the bladder, hyper-reflexia and/or involuntary urethral relaxation, weakness of the muscles associated with the bladder or neurologic abnormalities.

As used herein, and unless otherwise specified, a "therapeutically effective amount" of a compound is an amount sufficient to provide a therapeutic benefit in the treatment or management of a disease or disorder, or to delay or minimize one or more symptoms associated with the disease or disorder. A therapeutically effective amount of a compound means an amount of therapeutic agent, alone or in combination with other therapies, which provides a therapeutic benefit in the treatment or management of the disease or disorder. The term "therapeutically effective amount" can encompass an amount that improves overall therapy, reduces or avoids symptoms or causes of disease or disorder, or enhances the therapeutic efficacy of another therapeutic agent.

As used herein, and unless otherwise specified, a "prophylactically effective amount" of a compound is an amount sufficient to prevent a disease or disorder, or prevent its recurrence. A prophylactically effective amount of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

The term "composition" as used herein is intended to encompass a product comprising the specified ingredients (and in the specified amounts, if indicated), as well as any product which results, directly or indirectly, from combination of the specified ingredients in the specified amounts. By "pharmaceutically acceptable" it is meant the diluent, excipient or carrier must be compatible with the other ingredients of the formulation and not deleterious to the recipient thereof.

The term "therapeutically and/or prophylactically effective amount" refers to the amount of the subject solid form that will elicit the biological or medical response of a tissue, system, animal or human that is being sought by the researcher, veterinarian, medical doctor or other clinician or that is sufficient to prevent development of or alleviate to some extent one or more of the symptoms of the disease being treated.

The term "subject" is defined herein to include animals such as mammals, including, but not limited to, primates (e.g., humans), cows, sheep, goats, horses, dogs, cats, rabbits, rats, mice and the like. In specific embodiments, the subject is a human.

Prodrugs of the compounds are structurally modified forms of the compound that readily undergo chemical changes under physiological conditions to provide the compound. Additionally, prodrugs can be converted to the compound by chemical or biochemical methods in an ex *vivo* environment. For example, prodrugs can be slowly converted to a compound when placed in a transdermal patch reservoir with a suitable enzyme or chemical reagent. Prodrugs are often useful because, in some situations, they may be easier to administer than the compound, or parent drug. They may, for instance, be bioavailable by oral administration whereas the parent drug is not. The prodrug may also have improved solubility in pharmaceutical compositions over the parent drug. A wide variety of prodrug derivatives are known in the art, such as those that rely on hydrolytic cleavage or oxidative activation of the prodrug. An example, without limitation, of a prodrug would be a compound which is administered as a carbamate (the "prodrug"), but then is metabolically hydrolyzed to the phenol, the active entity. Additional examples include petidyl derivatives of a compound.

In certain embodiments, the compounds of the present invention may also contain unnatural proportions of atomic isotopes at one or more of the atoms. For example, the compound may be labeled with radioactive and/or nonradioactive isotopes, such as for example deuterium (²H), tritium (³H), iodine-125 (¹²⁵I), sulfur-35 (³⁵S), carbon-13 (¹³C) or carbon-14 (¹⁴C). Radiolabeled compounds are useful as therapeutic agents, e.g., cancer therapeutic agents, research reagents, e.g., binding assay reagents, and diagnostic agents, *e.g., in vivo* imaging agents. All isotopic variations of the compound of the present invention, whether radioactive or not, are intended to be encompassed within the scope of the present invention.

### 5.2 EMBODIMENTS OF THE INVENTION

The present invention is directed to solid forms comprising stereomerically pure (-)-O-desmethylvenlafaxine and salts thereof, in accordance with claim 1 and compositions comprising the solid form alone or in combination with other active ingredients, its use in the manufacture of a medicament for the treatment, prevention and or management of conditions and disorders including and compositions comprising the solid form its affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4^{th} edition (DSM-IV). While not intending to be bound by any particular theory, the storage stability, compressibility, density or dissolution properties of the solid form is beneficial for manufacturing, formulation and bio-availability of the present invention.

In one embodiment, the condition or disorder is an affective disorder. In another embodiment, the condition or disorder is depression. In another embodiment, the condition or disorder is an anxiety disorder. In another embodiment, the condition or disorder is a cerebral function disorder. In another embodiment, the condition or disorder is fibromyalgia. In another embodiment, the condition or disorder is pain. In another embodiment, the condition or disorder is neuropathic pain.

In certain embodiments, solid forms are those that are characterized by physical properties, e.g., stability, solubility and dissolution rate, appropriate for clinical and therapeutic dosage forms. Certain solid forms are characterized by physical properties, e.g., crystal morphology, compressibility and hardness, suitable for manufacture of a solid dosage form. Such properties can be determined using techniques such as X-ray diffraction, microscopy, IR spectroscopy and thermal analysis, as described herein and known in the art.

### 5.2.1 Salts of Stereomerically Pure (-)-O-desmethylvenlafaxine

Claim 1 provides a particular pharmaceutically acceptable salt of (-)-O-desmethylvenlafaxine, having utility for the treatment, prevention or management of conditions and disorders including, but not limited to, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4th edition (DSM-IV).

The present invention provides a hydrochloride salt of stereomerically pure (-)-O-desmethylvenlafaxine. As shown above, (-)-O-desmethylvenlafaxine has the general formula (I): 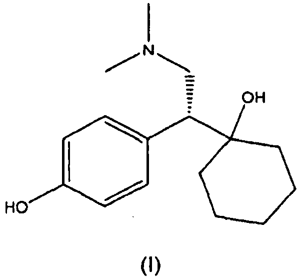 In the hydrochloride salts of (-)-O-desmethylvenlafaxine, the acid is according to the formula HCl.

A preferred hydrochloric acid salt of (-)-O-desmethylvenlafaxine is the monohydrochloric acid salt, provided by formula (II):

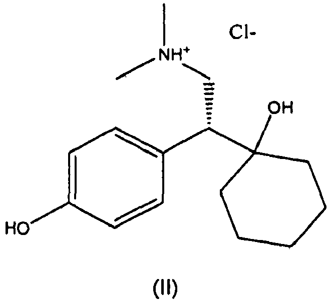

Each salt of the invention can be made from a preparation of stereomerically pure (-)-O-desmethylvenlafaxine or from an addition salt of (-)-O-desmethylvenlafaxine. (-)-O-desmethylvenlafaxine can be synthesized or obtained according to any method apparent to those of skill in the art. In preferred embodiments, (-)-O-desmethylvenlafaxine is prepared according to the methods described in detail in the examples below, in U.S. Patent Nos. 6,342,533 B1, 6,441,048 B1 and 6,911,479 B2.

In some embodiments, (-)-O-desmethylvenlafaxine prepared by any method can be contacted with an appropriate acid, either neat or in a suitable solvent, to yield the salts of the invention. For example (-)-O-desmethylvenlafaxine can be contacted with hydrochloric acid to yield the hydrochloride salt of the invention.

In some embodiments, an (-)-O-desmethylvenlafaxine addition salt prepared by any method known in the art can be contacted with an appropriate acid, either neat or in a suitable solvent, to yield the salts of the invention. For example, (-)-O-desmethylvenlafaxine cyclohexylphenylycolic acid salt can be contacted with hydrochloric acid to yield the hydrochloride salt of the invention.

As shown below, certain forms comprising the hydrochloride salt of (-)-O-desmethylvenlafaxine display superior stability, solubility and hygroscopicity properties in comparison to other forms comprising (-)-O-desmethylvenlafaxine.

### 5.2.2 Solid Forms Comprising Stereomerically Pure (-)-O-desmethylvenlafaxine and Salts Thereof

The present invention also provides crystal forms comprising stereomerically pure (-)-O-desmethylvenlafaxine salts in acordance with claim 1, having particular utility for the treatment, prevention or management of conditions and disorders including, but not limited to, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4th edition (DSM-IV). The solid forms of the invention are crystal forms comprising the hydrochloride salt of (-)-O-desmethylvenlafaxine described above.

In certain embodiments, crystal forms of the invention can be made from a preparation of (-)-O-desmethylvenlafaxine. For instance, a salt of (-)-O-desmethylvenlafaxine can be dissolved and then crystallized to yield crystal forms of the invention. In particular embodiments of the invention, a hydrochloride salt of (-)-O-desmethylvenlafaxine can be crystallized from particular solvent mixtures, such as those described below, to yield the crystal forms of the invention.

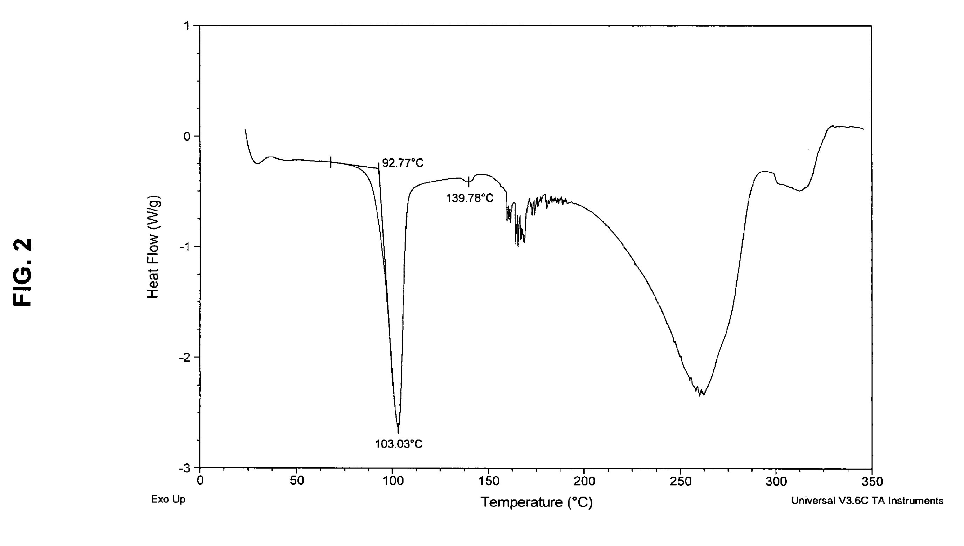

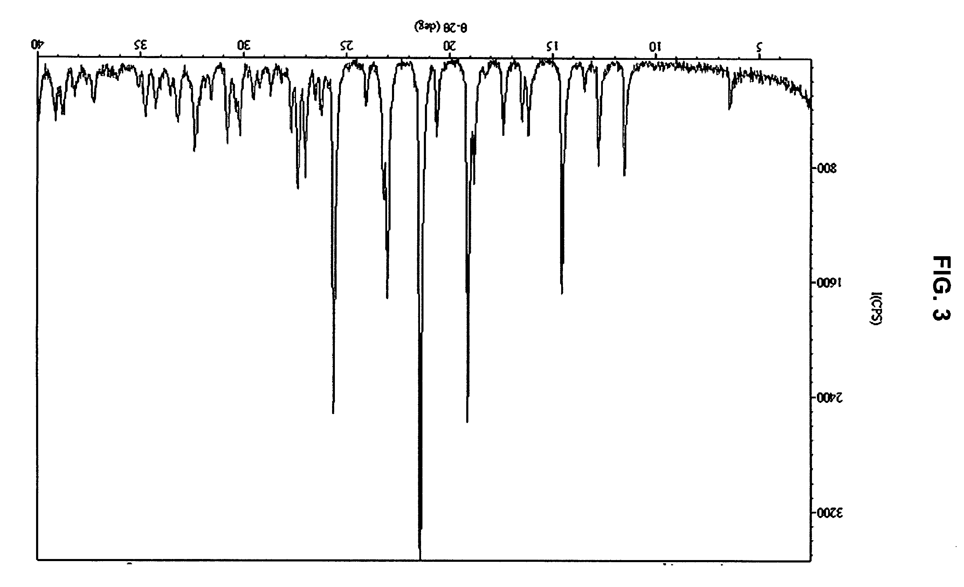

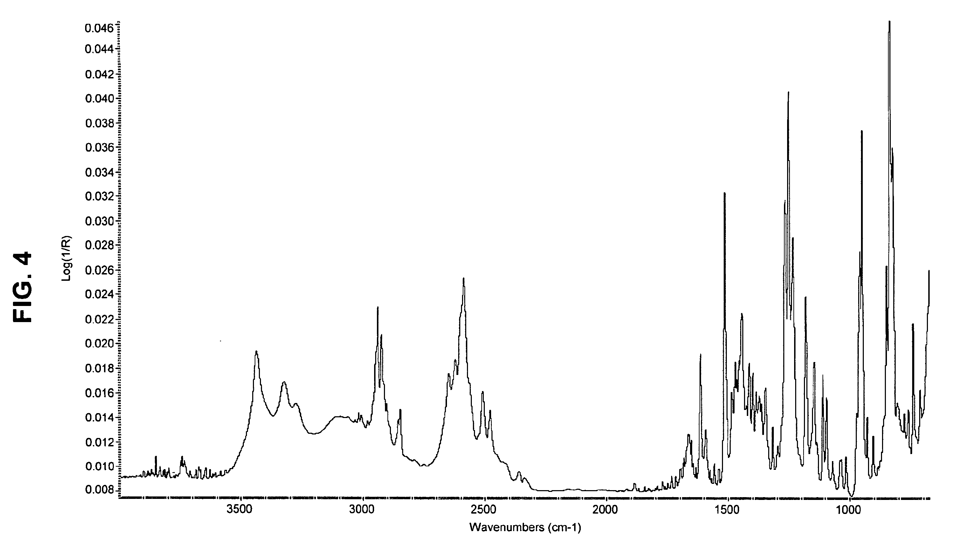

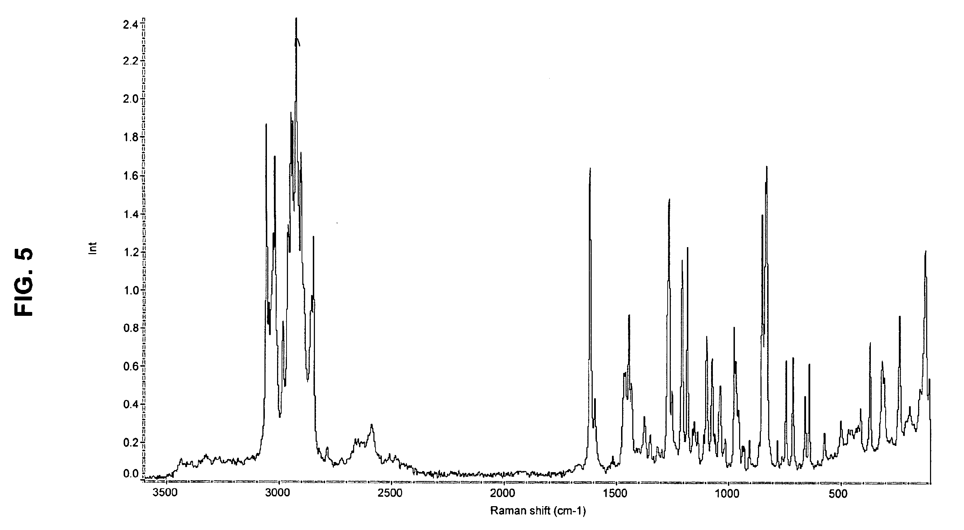

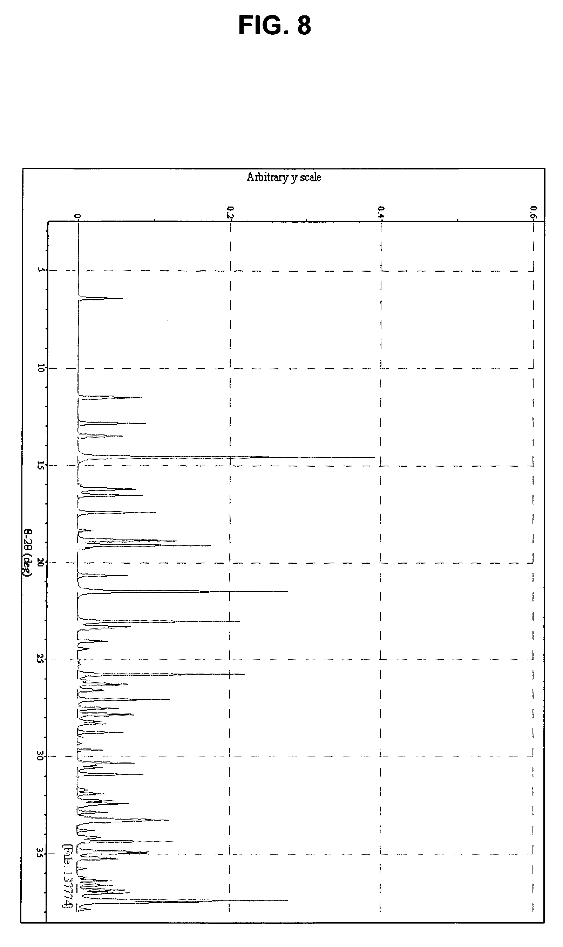

The present invention provides Form A, a crystal form of a hydrochloride salt of (-)-O-desmethylvenlafaxine ((-)-1-[2-(dimethylamino)-1-(4-hydroxyphenyl)ethyl]cyclohexanol hydrochloride salt). Form A is a crystal form of the monohydrochloride salt of (-)-O-desmethylvenlafaxine. In certain embodiments, Form A has a water content of about 6 % of the total mass of the sample, which in accordance with claim 1, is equal to about one molar equivalent of water per mole of (-)-O-desmethylvenlafaxine. In certain embodiments, when examined by Karl Fisher titration according to the methods described herein, Form A has a water content of about 5.7% by mass. In further embodiments, Form A has a thermal gravimetric analysis thermogram similar to that of FIG. 1. In certain embodiments, when examined by thermal gravimetric analysis according to the methods described herein, Form A has a weight loss corresponding to about 5.6% of the total mass of the sample occurring between about 25 and about 110 °C. In certain embodiments, Form A has a differential scanning calorimetry thermogram similar to that of FIG. 2. In certain embodiments, when examined by differential scanning calorimetry according to the methods described herein, Form A has an endotherm with an onset temperature at about 93 °C. In certain embodiments, the Form A crystal form of the hydrochloride salt of (-)-O-desmethylvenlafaxine has an X-ray powder diffraction pattern similar to that of FIG. 3 using Cu Kα radiation. In certain embodiments, the Form A crystal form of the hydrochloride salt of (-)-O-desmethyvenlafaxine has an X-ray powder diffraction pattern similar to that of FIG. 8, which was simulated for Cu Kα radiation using single-crystal X-ray diffraction structural data obtained on Form A. In accordance with claim 1, Form A crystal forms of the invention have major X-ray powder diffraction pattern peaks at about 12.7, 14.5, 19.1, 21.4, 23.0, 25.5, 27.3 °2*θ* using Cu Kα radiation. In certain embodiments, the Form A crystal form of the invention has both a water content of about 5.7 % of the total mass of the sample and major X-ray powder diffraction pattern peaks at about 12.7, 14.5, 19.1, 21.4, 23.0, 25.5, 27.3 °2*θ* using Cu Kα radiation. In certain embodiments of the invention, Form A has an infrared spectrum similar to that of FIG. 4. In certain embodiments of the invention, Form A has a Raman spectrum similar to that of FIG. 5. In certain embodiments, when analyzed at approximately 150 K according to a method capable of determining unit cell parameters, e.g. single crystal X-ray diffraction, Form A has the following approximate unit cell parameters: a = 6.78 *A; b* = 9.29 A; c = 27.65 A; α = 90°; β = 90°; γ = 90°; *V* = 1741.39 Å³. In certain embodiments, Form A crystallizes in space group *P*2₁2₁2₁.

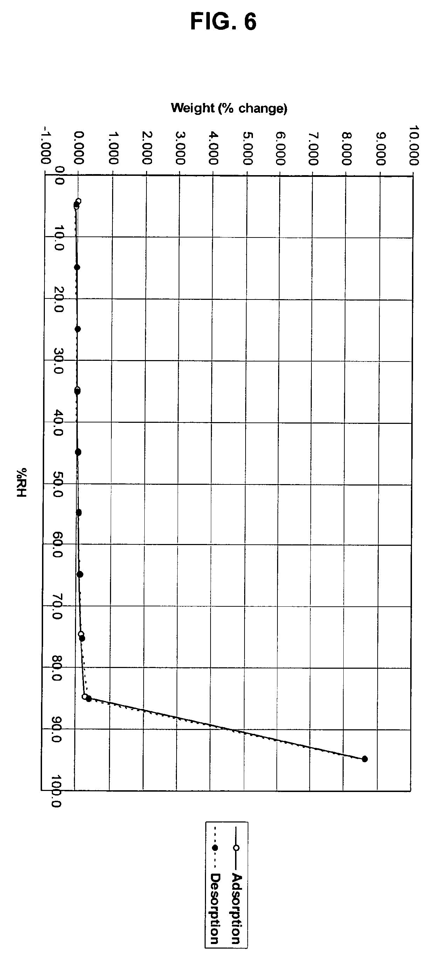

Without being limited by a particular theory, it has been found that the Form A crystal form of the hydrochloride salt of (-)-O-desmethylvenlafaxine has excellent hygroscopicity properties. For example, without being limited by a particular theory, when examined by dynamic vapor sorption according to the methods described herein, Form A gains <1% in mass upon increasing the sample from 5% to 90% relative humidity. Further, the mass gain of Form A as a function of relative humidity is reversible, such that, for example, the sample loses about 1% in mass upon decreasing from 90% to 5% relative humidity. In certain embodiments, the Form A crystal form provided herein has a moisture sorption isotherm similar to that of FIG. 6.

In addition, without being limited by a particular theory, it has been found that the Form A crystal form of the hydrochloride salt of (-)-O-desmethylvenlafaxine also has excellent stability properties.

Form A of the hydrochloride salt of (-)-O-desmethylvenlafaxine can be made by any method of making Form A apparent to those of skill in the art based upon the teachings herein. In certain embodiments, Form A can be prepared by crystallization of the hydrochloride salt of (-)-O-desmethylvenlafaxine from a solvent system containing one or more solvents, such as, but not limited to, water, acetone, acetonitrile, ethanol, isopropanol, methanol, methyl ethyl ketone, methyl t-butyl ether, heptane, hexanes toluene and mixtures thereof. In certain embodiments, Form A may be obtained by crystal form conversion from another crystal or amorphous form of the hydrochloride salt of (-)-O-desmethylvenlafaxine, for instance, via a solvent-mediated and/or water-mediated form conversion process.

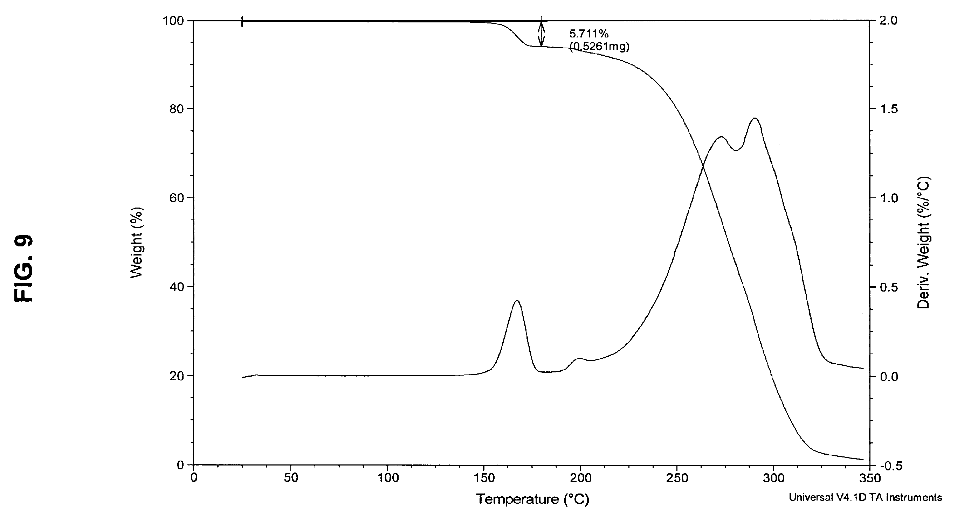

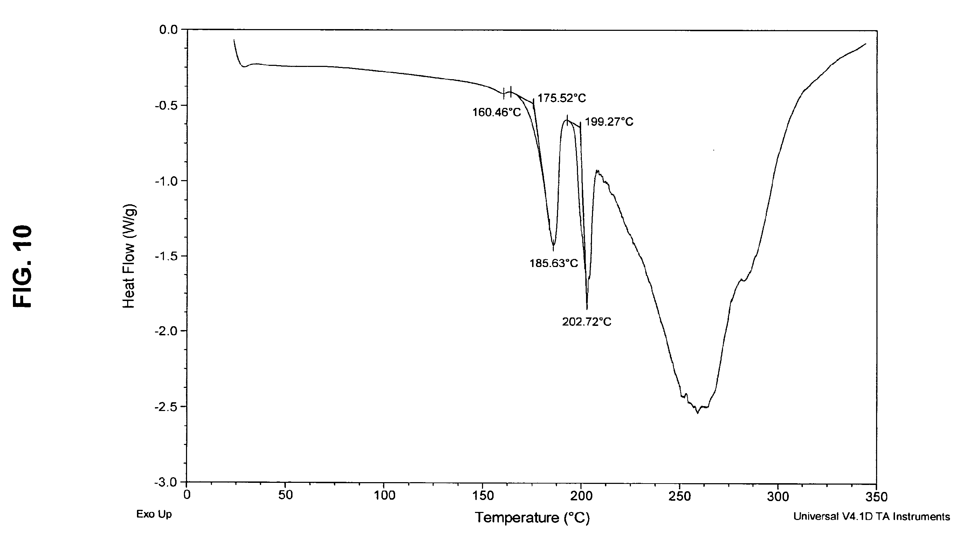

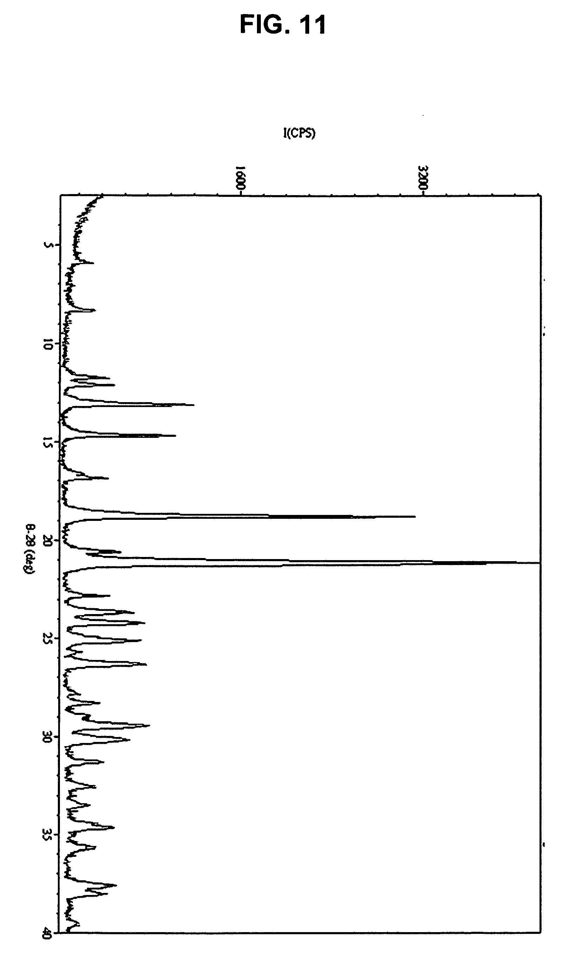

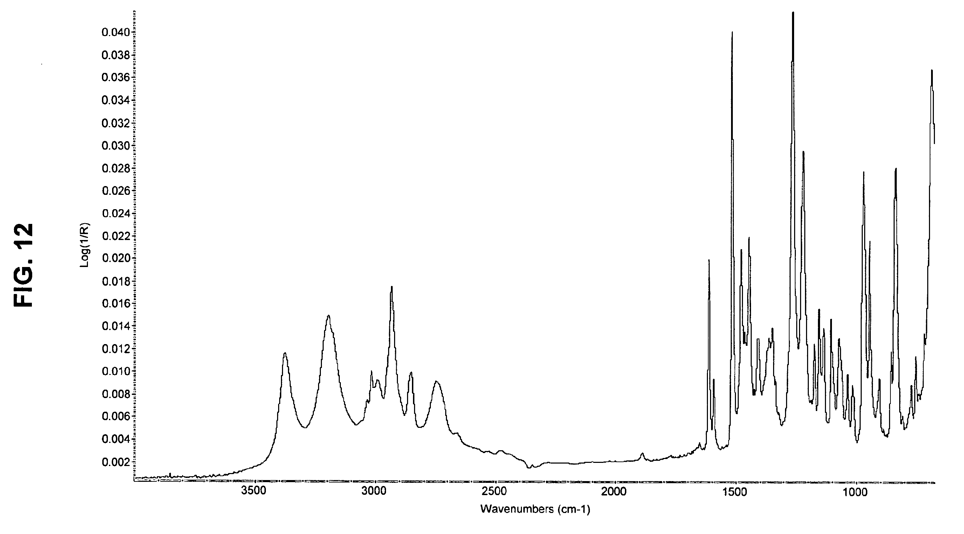

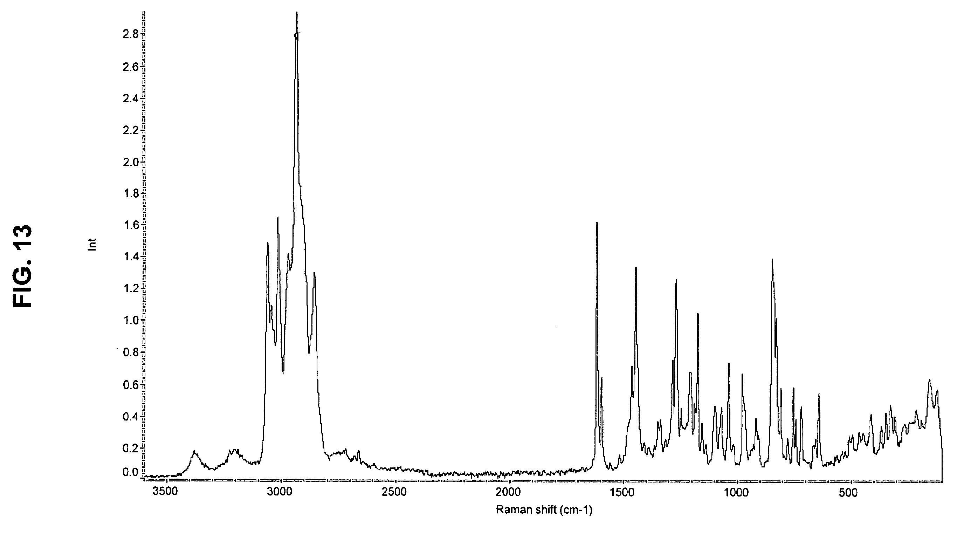

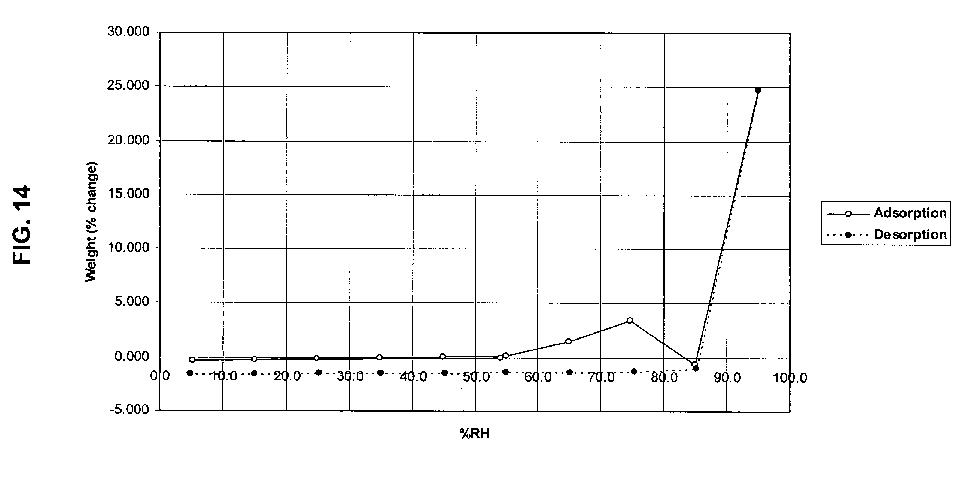

Also disclosed is Form B, a crystal form of the monohydrochloride salt of (-)-O-desmethylvenlafaxine that contains the solvent tetrahydrofuran (THF) in the crystal lattice. In a certain embodiment, the THF is present in the approximate ratio of 0.25 molar equivalents of THF per mole of the hydrochloride salt of (-)-O-desmethylvenlafaxine. In terms of mass, this equates to a THF content of approximately 6% of the total mass of a sample of Form B. In a certain embodiment, the THF content of Form B ranges from about 4% to about 8% of the total mass of the sample of Form B. In certain embodiments, Form B has a thermal gravimetric analysis thermogram similar to that of FIG. 9. In certain embodiments, when examined by thermal gravimetric analysis according to the methods described herein, Form B has a weight loss corresponding to about 5.7% of the total mass of the sample occurring between about 25 and about 180 °C. In certain embodiments, the Form B crystal form has a differential scanning calorimetry thermogram similar to that of FIG. 10. In certain embodiments, when examined by differential scanning calorimetry according to the methods described herein, Form B has an endotherm with an onset temperature of about 176 °C and another endotherm with an onset temperature of about 199 °C. In certain embodiments, Form B has an additional endotherm with a peak temperature at about 160 °C. In certain embodiments, the Form B crystal form of the hydrochloride salt of (-)-O-desmethylvenlafaxine has an X-ray powder diffraction pattern similar to that of FIG. 11 using Cu Kα radiation. In certain embodiments, Form B crystal forms have major X-ray powder diffraction pattern peaks at about 13.1, 14.7, 18.8, 21.1, 24.2, 26.3, 29.4 °2*θ* using Cu Kα radiation. In certain embodiments, the Form B crystal form has major X-ray powder diffraction pattern peaks at one, two, three, four, five, six or seven of the X-ray powder diffraction pattern positions of about 13.1, 14.7, 18.8, 21.1, 24.2, 26.3, 29.4 °2*θ* using Cu Kα radiation. In certain embodiments, the Form B crystal form has both a THF content of about 6% of the total mass of the sample and major X-ray powder diffraction pattern peaks at one, two, three, four, five, six or seven of the X-ray powder diffraction pattern positions of about 13.1,14.7, 18.8, 21.1, 24.2, 26.3, 29.4 °2*θ* using Cu Kα radiation. In certain embodiments, the Form B has an infrared spectrum similar to that of FIG. 12. In certain embodiments, the Form B crystal form has a Raman spectrum similar to that of FIG. 13. In a certain embodiment, Form B has a dynamic vapor sorption isotherm similar to that of FIG. 14. In certain embodiments, when examined by dynamic vapor sorption according to the methods described herein, Form B exhibits a gain in mass of about 25% when increased from 5% to 95% relative humidity, followed by a loss in mass of about 26% when decreased from 95% to 5% relative humidity.

Form B of the hydrochloride salt of (-)-O-desmethylvenlafaxine can be made by any method of making Form B apparent to those of skill in the art based upon the teachings herein. In certain embodiments, Form B can be prepared by crystallization from solutions of the hydrochloride salt of (-)-O-desmethylvenlafaxine in THF.

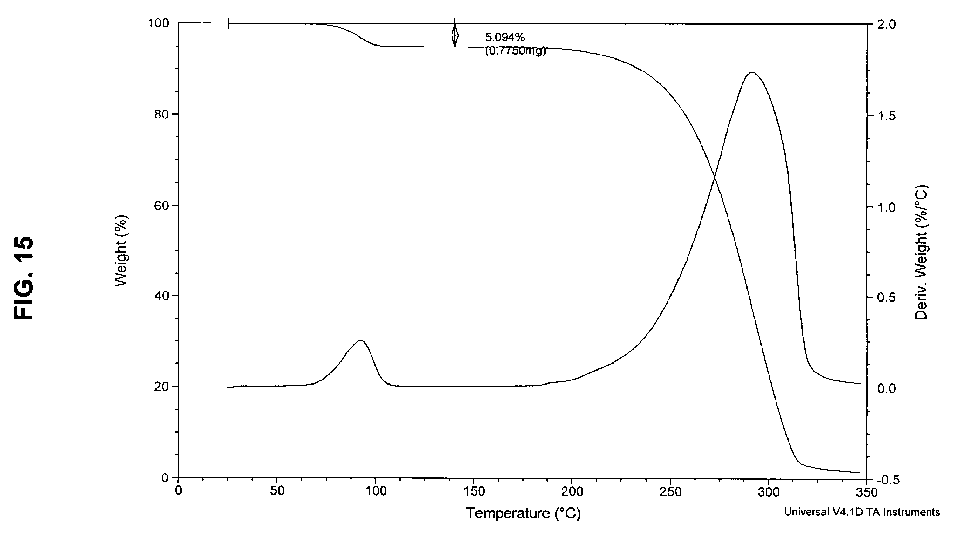

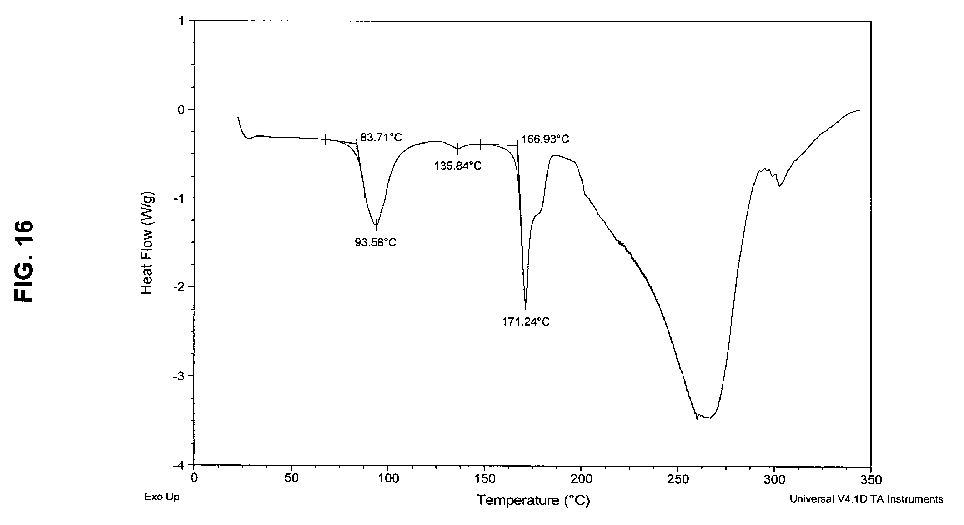

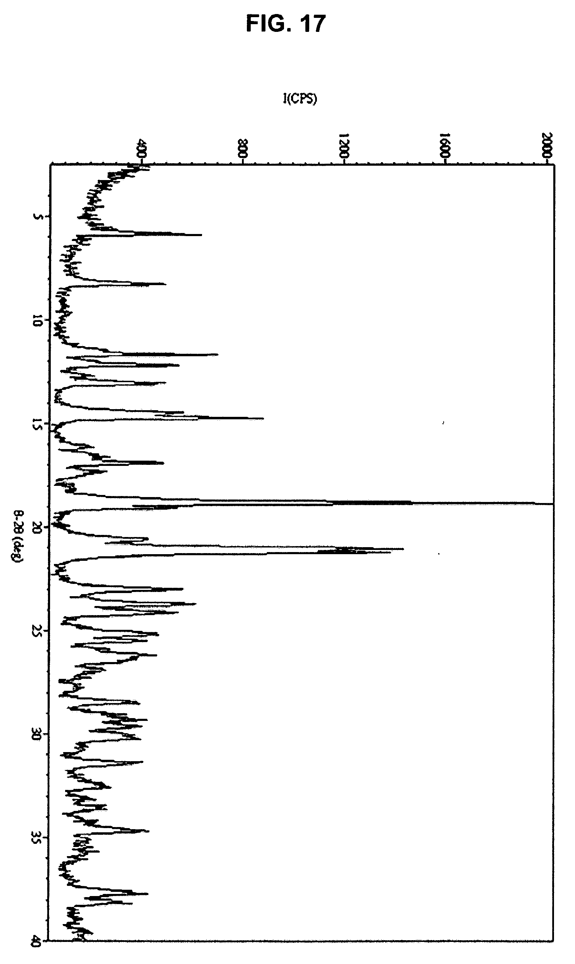

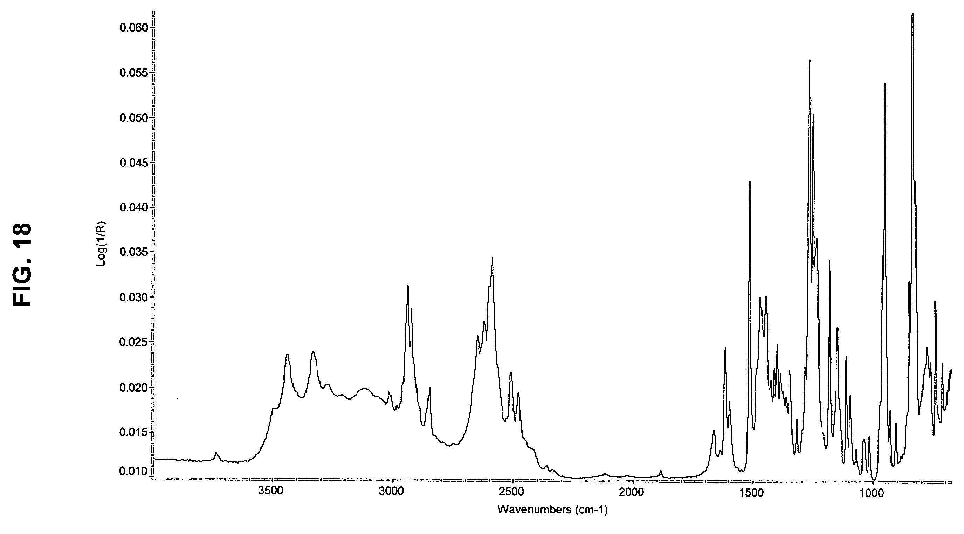

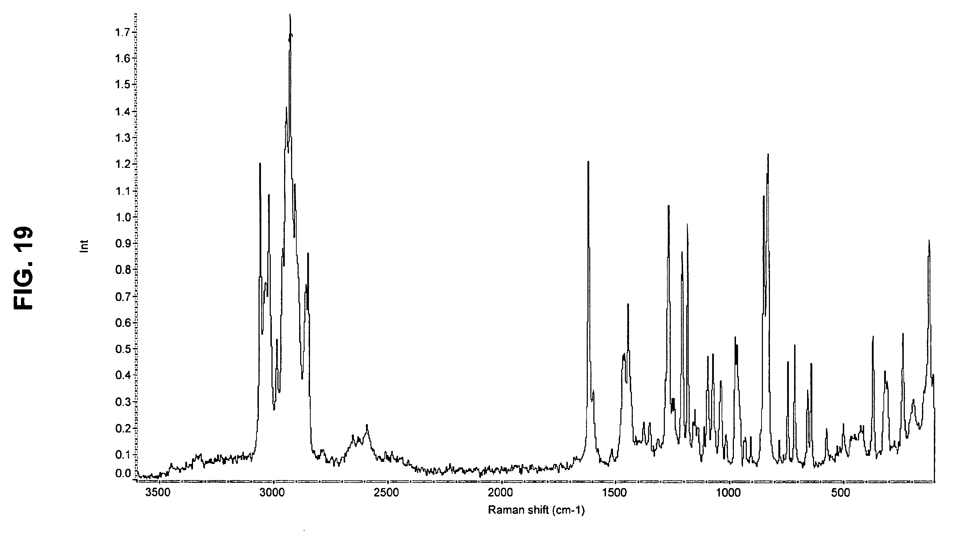

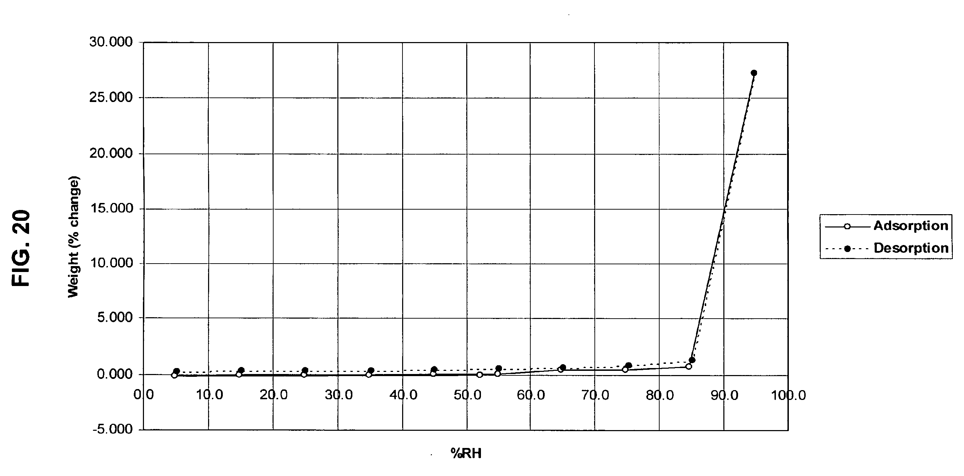

The disclosure present provides Form C, a crystal form of the monohydrochloride salt of (-)-O-desmethylvenlafaxine that contains one or more of the solvents ethyl acetate, ethyl ether and water in the crystal lattice. In a particular embodiment, ethyl acetate is present in the approximate ratio of 0.2 molar equivalents of ethyl acetate per mole of the hydrochloride salt of (-)-O-desmethylvenlafaxine. In terms of mass, this equates to an ethyl acetate content of approximately 6% of the total mass of a sample of Form C. In a particular embodiment, ethyl ether is present in the approximate ratio of 0.2 molar equivalents of ethyl ether per mole of the hydrochloride salt of (-)-O-desmethylvenlafaxine. In terms of mass, this equates to an ethyl ether content of approximately 5% of the total mass of a sample of Form C. In a particular embodiment, the combined content of ethyl acetate, ethyl ether and water ranges from about 3% to about 8% of the total mass of the sample of Form C. In certain embodiments, Form C has a thermal gravimetric analysis thermogram similar to that of FIG. 15. In certain embodiments, when examined by thermal gravimetric analysis according to the methods described herein, Form C has a weight loss corresponding to about 5.1% of the total mass of the sample occurring between about 25 and about 110 °C. In certain embodiments, the Form C crystal form has a differential scanning calorimetry thermogram similar to that of FIG. 16. In certain embodiments, when examined by differential scanning calorimetry according to the methods described herein, Form C has an endotherm with an onset temperature of about 84 °C, another endotherm with a peak temperature at about 136 °C, and another endotherm with an onset temperature at about 167 °C. In certain embodiments, the Form C crystal form of the hydrochloride salt of (-)-O-desmethylvenlafaxine has an X-ray powder diffraction pattern similar to that of FIG. 17 using Cu Kα radiation. Particular Form C crystal forms have major X-ray powder diffraction pattern peaks at about 5.8, 11.7, 14.7, 18.8, 21.0, 21.2 °2*θ* using α radiation. In certain embodiments, the Form C crystal form has major X-ray powder diffraction pattern peaks at one, two, three, four, five or six of the X-ray powder diffraction pattern positions of about 5.8, 11.7, 14.7, 18.8, 21.0, 21.2 °2*θ* using Cu Kα radiation. In certain embodiments, the Form C crystal form has a combined content of ethyl acetate, ethyl ether and water amounting to between about 3% and about 8% of the total mass of the sample and major X-ray powder diffraction pattern peaks at one, two, three, four, five or six of the X-ray powder diffraction pattern positions of about 5.8, 11.7, 14.7, 18.8, 21.0, 21.2 °2*θ* using Cu Kα radiation. In certain embodiments, the Form C crystal form has an infrared spectrum similar to that of FIG. 18. In certain embodiments, the Form C crystal form has a Raman spectrum similar to that of FIG. 19. In certain embodiments, Form C has a dynamic vapor sorption isotherm similar to that of FIG. 20. In certain embodiments, when examined by dynamic vapor sorption according to the methods described herein, Form C exhibits a gain in mass of about 27% when increased from 5% to 95% relative humidity, followed by a loss in mass of about 27% when decreased from 95% to 5% relative humidity.

Form C of the hydrochloride salt of (-)-O-desmethylvenlafaxine can be made by any method of making Form C apparent to those of skill in the art based upon the teachings herein. In certain embodiments, Form C can be prepared by crystallization from solutions of the hydrochloride salt of (-)-O-desmethylvenlafaxine in ethyl acetate, ethyl ether, water, a mixture of two or more of these solvents.

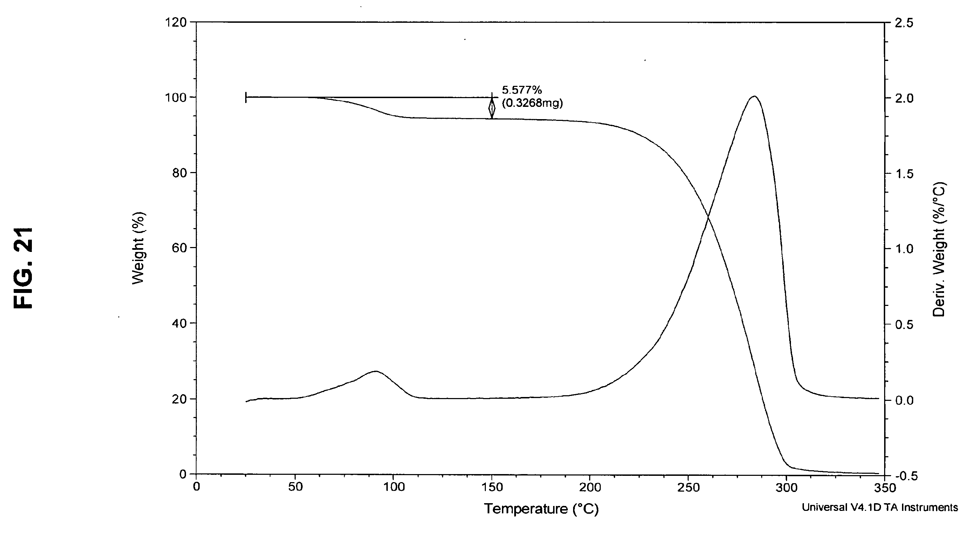

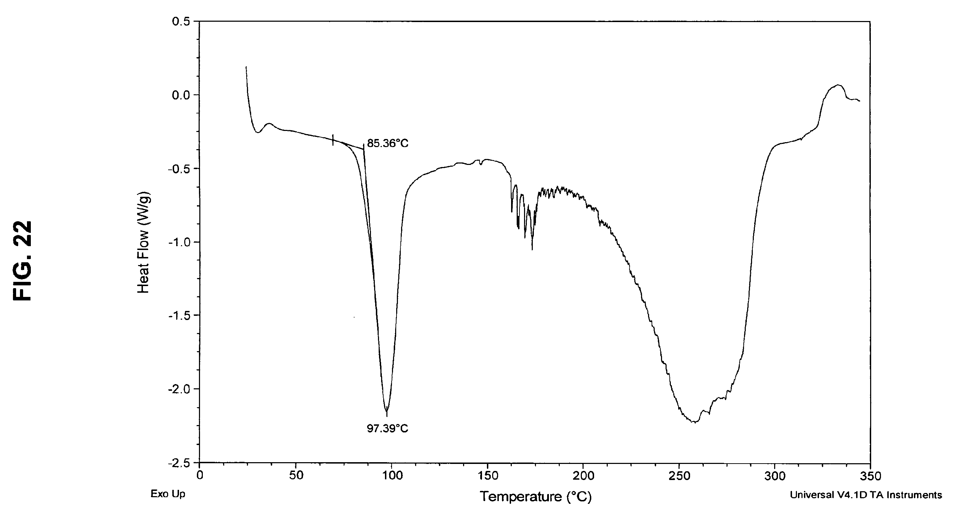

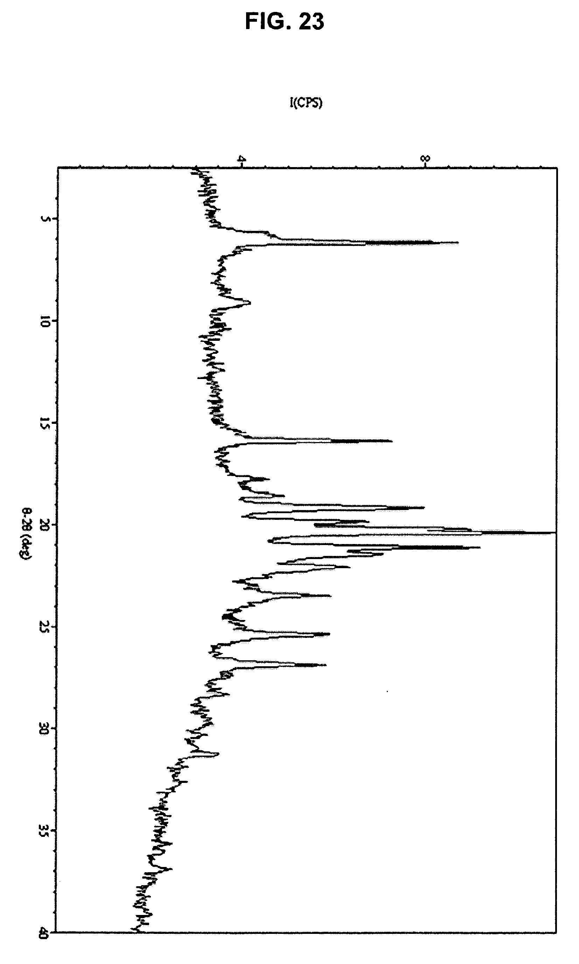

The present disclosure provides Form D, a crystal form of the monohydrochloride salt of (-)-O-desmethylvenlafaxine that contains isopropyl alcohol (IPA) and/or water in the crystal lattice. In one embodiment, a sample of the Form D crystal form of the hydrochloride salt of (-)-O-desmethylvenlafaxine has a combined IPA and water content ranging between about 2% and about 8% of the total mass of the sample. In certain embodiments, the Form D crystal form has a thermal gravimetric analysis thermogram similar to that of FIG. 21. In certain embodiments, when examined by thermal gravimetric analysis according to the methods described herein, Form D has a weight loss corresponding to about 5.6% of the total mass of the sample occurring in the range of about 25 to about 150 °C. In certain embodiments, the Form D crystal form has a differential scanning calorimetry thermogram similar to that of FIG. 22. In certain embodiments, when examined by differential scanning calorimetry according to the methods described herein, Form D has an endotherm with an onset temperature at about 85 °C. In certain embodiments, the Form D crystal form of the hydrochloride salt of (-)-O-desmethylvenlafaxine has an X-ray powder diffraction pattern similar to that of FIG. 23 using Cu Kα radiation. Particular Form D crystal forms have major X-ray powder diffraction pattern peaks at about 2.4, 5.7, 6.0, 15.9, 19.1, 19.8, 20.3 °2*θ* using Cu Kα radiation. In certain embodiments, the Form D crystal form has major X-ray powder diffraction peaks at one, two, three, four, five, six or seven of the X-ray powder diffraction pattern positions of about 2.4, 5.7, 6.0, 15.9, 19.1, 19.8, 20.3 °2*θ* using Cu Kα radiation. In certain embodiments, the Form D crystal form has both a combined IPA and water content ranging between about 2% and about 8% of the total mass of the sample and major X-ray powder diffraction peaks at one, two, three, four, five, six or seven of the X-ray powder diffraction pattern positions of about 2.4, 5.7, 6.0, 15.9, 19.1, 19.8, 20.3 °2*θ* using Cu Kα radiation.

Form D of the hydrochloride salt of (-)-O-desmethylvenlafaxine can be made by any method of making Form D apparent to those of skill in the art based upon the teachings herein. In certain embodiments, Form D can be prepared by crystallization from solutions of the hydrochloride salt of (-)-desmethylvenlafaxine in IPA.

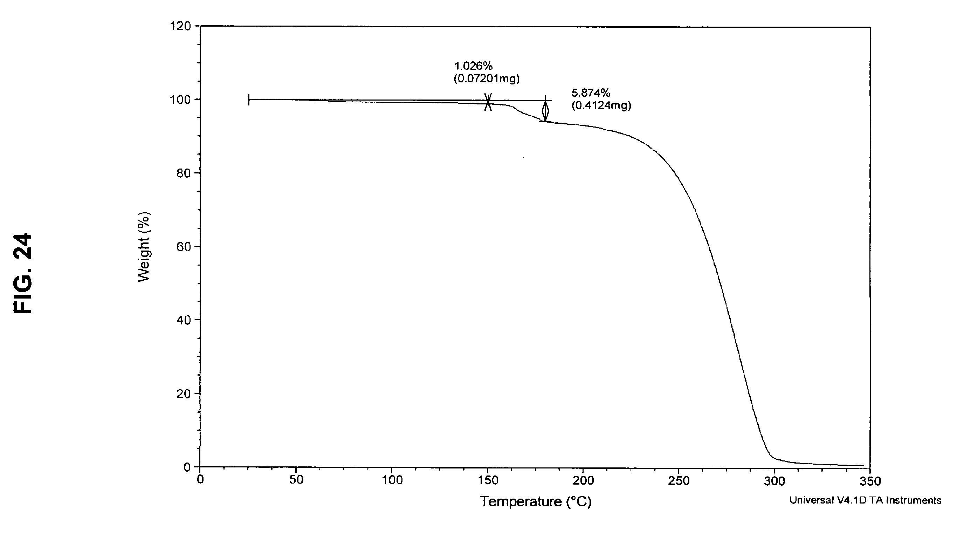

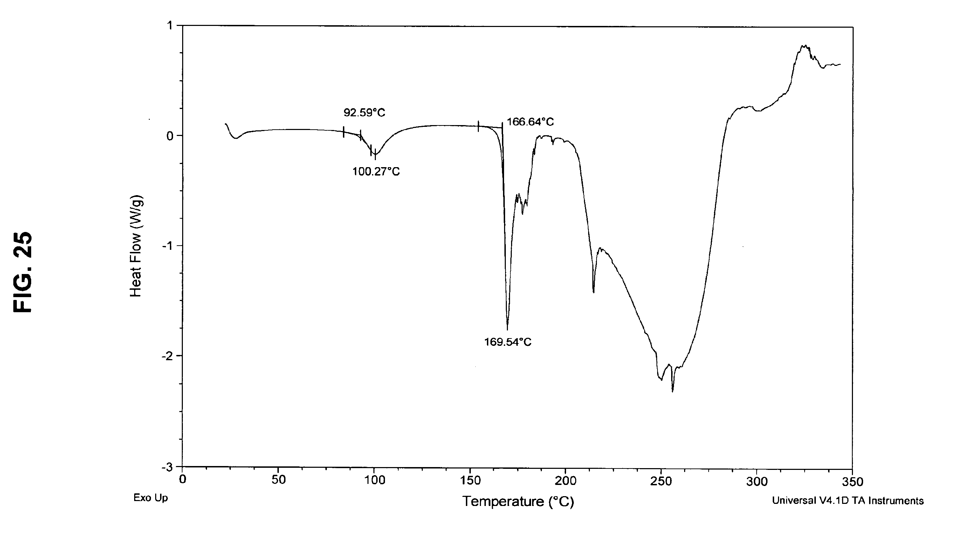

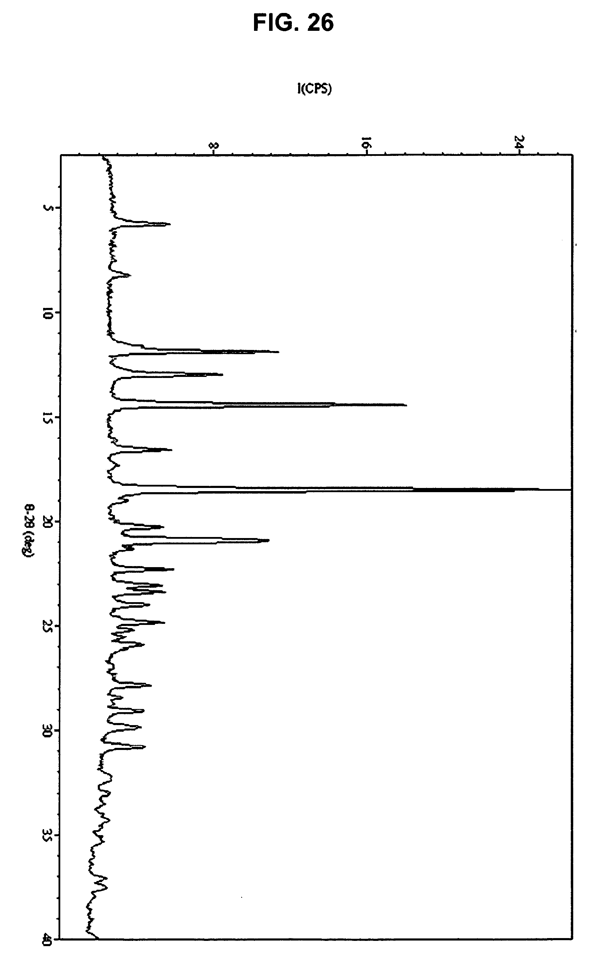

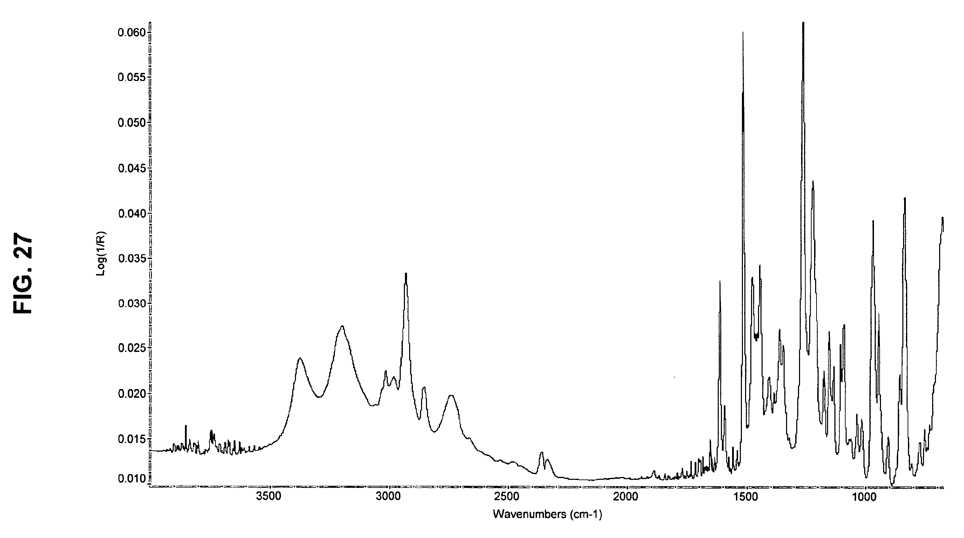

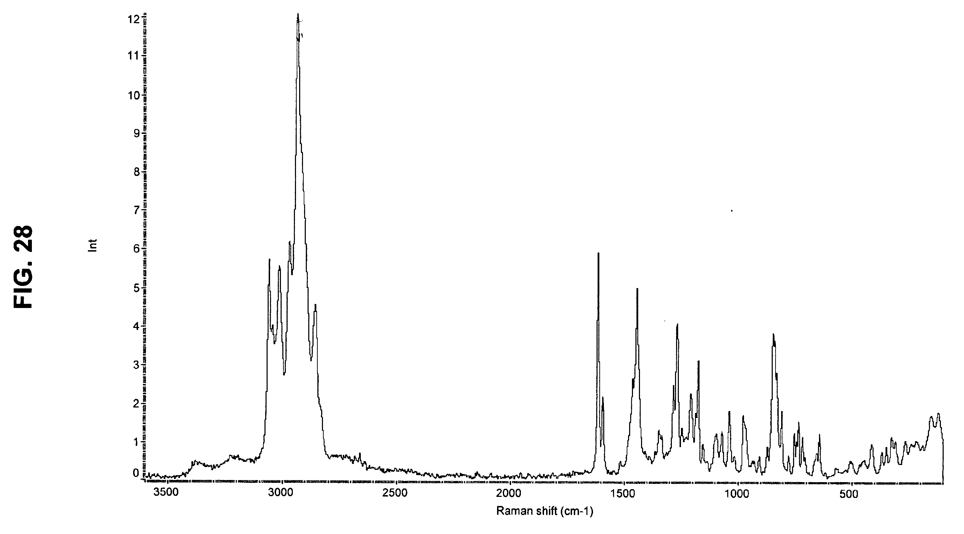

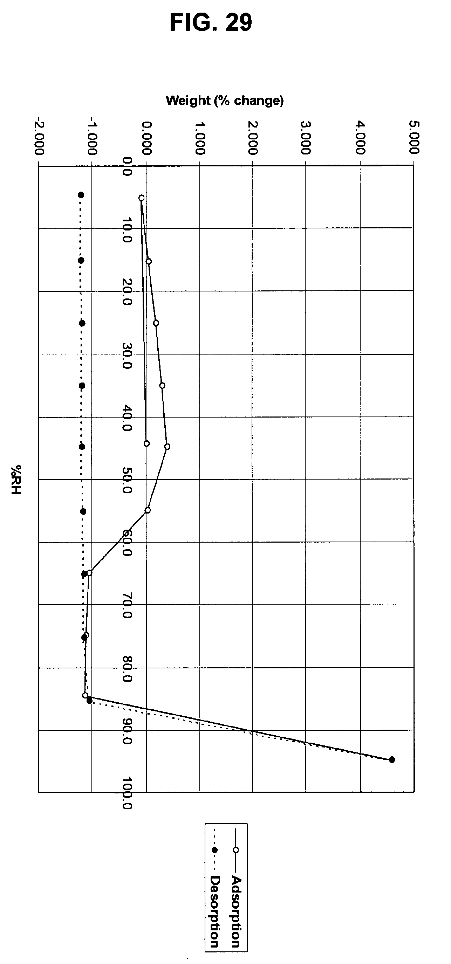

The present disclosure provides Form E, a crystal form of the hydrochloride salt of (-)-O-desmethylvenlafaxine IPA disclosure monohydrochloride salt of (-)-O-desmethylvenlafaxine that contains methyl t-butyl ether (MTBE) and/or water in the crystal lattice. In one embodiment a sample of the Form E crystal form of the hydrochloride salt of (-)-O-desmethylvenlafaxine has a combined MTBE and water content ranging between about 4% and about 10% of the total mass of the sample. In certain embodiments, the Form E crystal form has a MTBE content of about 6% of the total mass of the sample, which is equal to about 0.2 molar equivalent of MTBE per mole of (-)-O-desmethylvenlafaxine. In certain embodiments, Form E has a thermal gravimetric analysis thermogram similar to that of FIG. 24. In certain embodiments, when examined by thermal gravimetric analysis according to the methods described herein, Form E has a weight loss corresponding to about 5.9% of the total mass of the sample occurring in the range of about 25 to about 180 °C. In certain embodiments, Form E has a differential scanning calorimetry thermogram similar to that of FIG. 25. In certain embodiments, when examined by differential scanning calorimetry according to the methods described herein, Form E has an endotherm with an onset temperature at about 93 °C, followed by an endotherm with an onset temperature at about 167 °C. In certain embodiments, the Form E crystal form of the hydrochloride salt of (-)-O-desmethylvenlafaxine has an X-ray powder diffraction pattern similar to that of FIG. 26 using Cu Kα radiation. Particular Form E crystal forms have major X-ray powder diffraction pattern peaks at about 5.8, 11.9, 13.0, 14.4, 18.5, 20.9 °2*θ* using Cu Kα radiation. In certain embodiments, the Form E crystal form has major X-ray powder diffraction peaks at one, two, three, four, five or six of the X-ray powder diffraction pattern positions of about 5.8, 11.9, 13.0, 14.4, 18.5, 20.9 °2*θ* using Cu Kα radiation. In certain embodiments, the Form E crystal form has both a solvent content ranging between about 4% and about 10% of the total mass of the sample and major X-ray powder diffraction peaks at one, two, three, four, five or six of the X-ray powder diffraction pattern positions of about 5.8, 11.9, 13.0, 14.4, 18.5, 20.9 °2*θ* using Cu Kα radiation. In certain embodiments, the Form E crystal form has an infrared spectrum similar to that of FIG. 27. In certain embodiments, the Form E crystal form has a Raman spectrum similar to that of FIG. 28. In certain embodiments. Form E has a dynamic vapor sorption isotherm similar to that of FIG. 29. In certain embodiments, when examined by dynamic vapor sorption according to the methods described herein, Form E exhibits a net gain in mass of about 4.7% when increased from 5% to 95% relative humidity, followed by a loss in mass of about 5.8% when decreased from 95% to 5% relative humidity.

Form E of the hydrochloride salt of (-)-O-desmethylvenlafaxine can be made by any method of making Form E apparent to those of skill in the art based upon the teachings herein. In certain embodiments, Form E can be prepared by the dissolution of a solid form comprising the hydrochloride salt of (-)-O-desmethylvenlafaxine in a solvent or solvent mixture containing, for example, methanol and water, followed by subsequent crystallization brought about by the addition of an anti-solvent, such as methyl t-butyl ether.

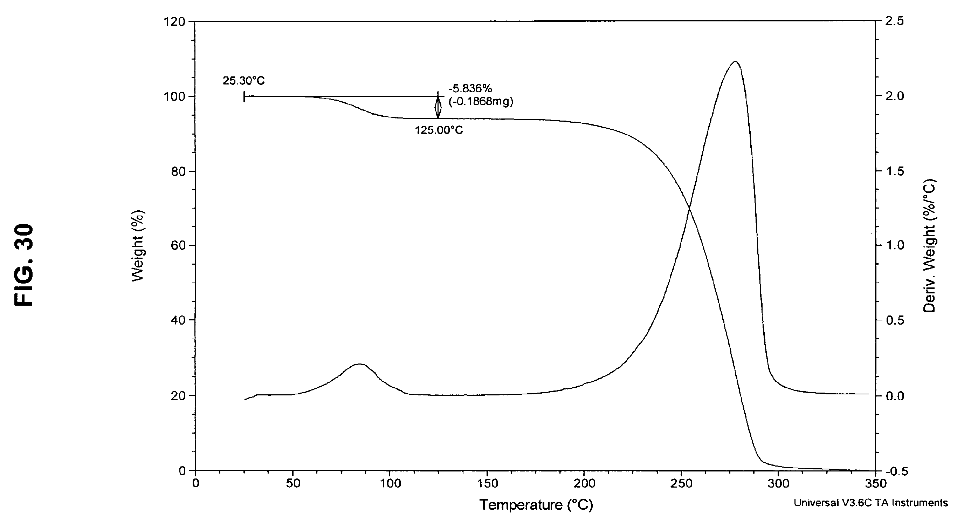

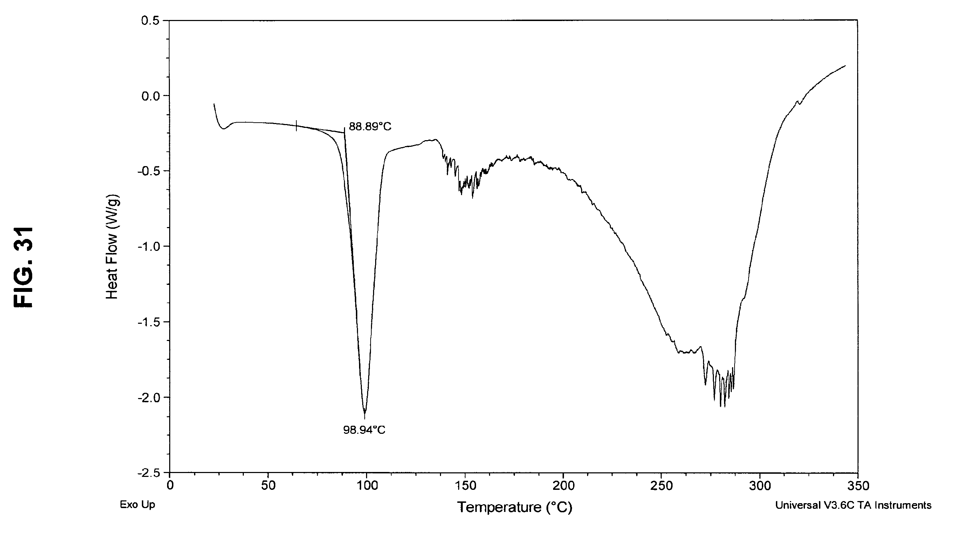

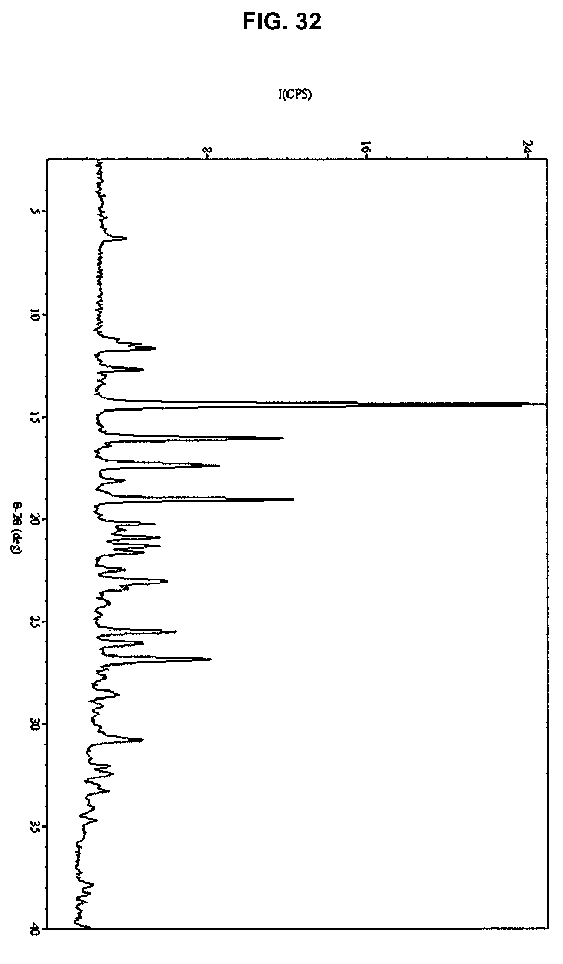

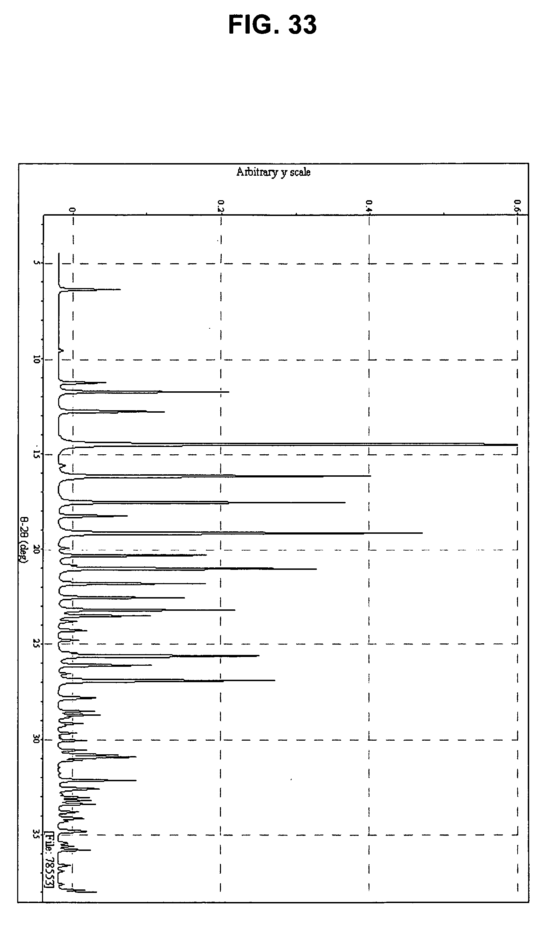

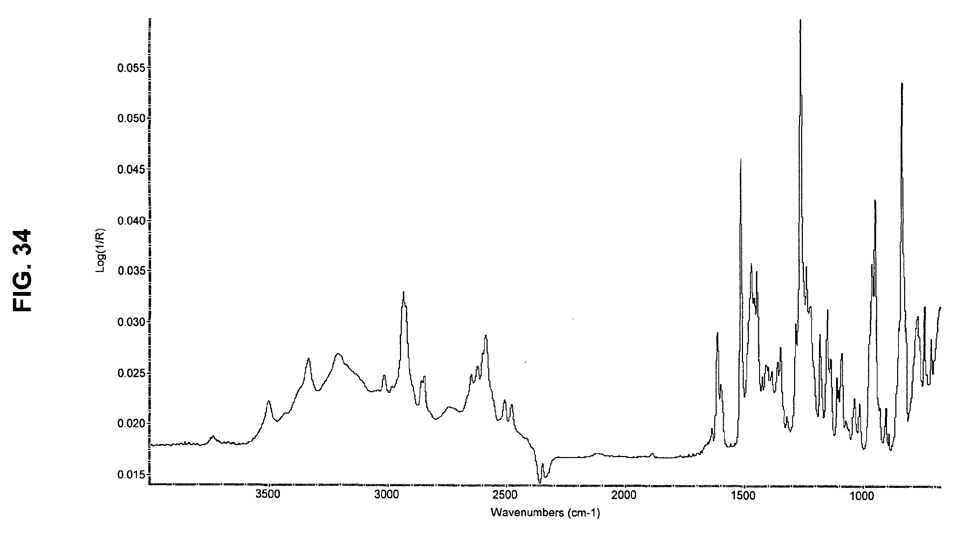

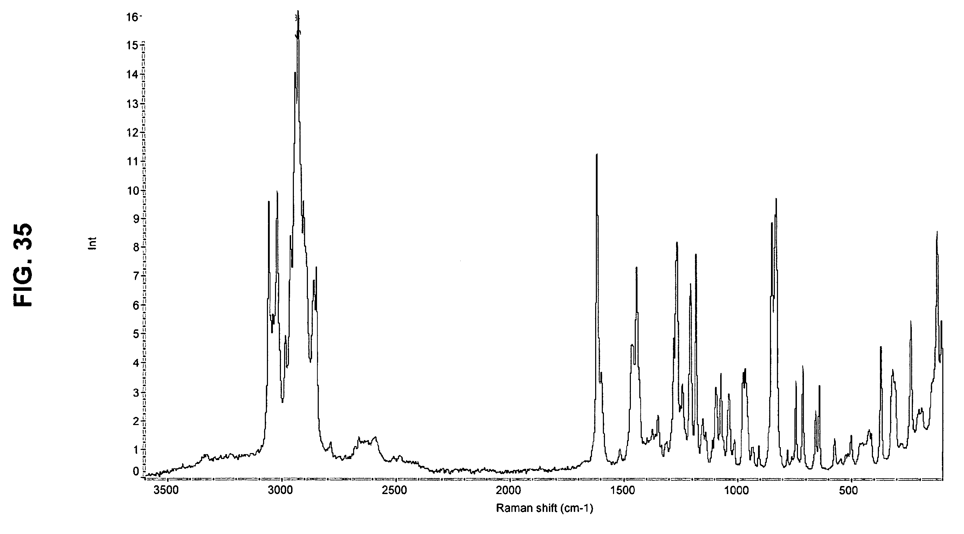

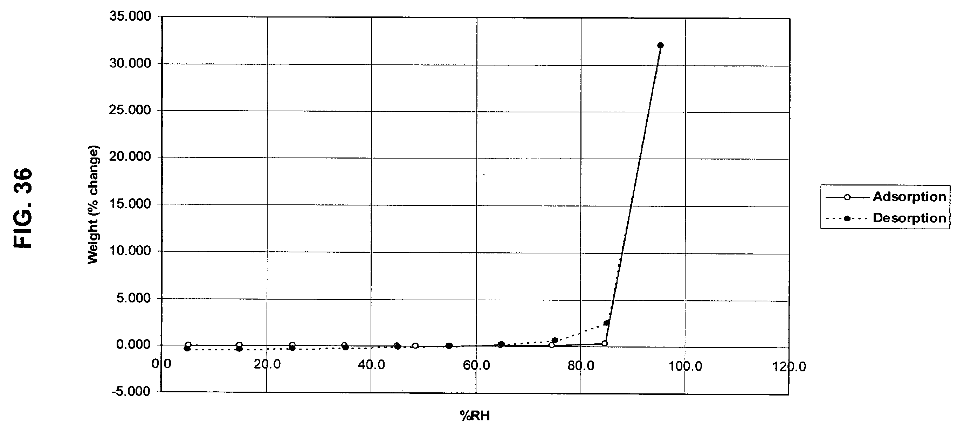

The present disclosure provides From F, a hydrate crystal about by the addition of an anti-solvent, such as methyl t-butyl ether. disclosure form of the monohydrochloride salt of (-)-O-desmethylvenlafaxine. In one embodiment, a sample of the Form F crystal form of the hydrochloride salt of (-)-O-desmethylvenlafaxine has a water content ranging between about 4% and about 8% of the total mass of the sample. In certain embodiments, the Form F crystal form has a water content of about 6% of the total mass of the sample, which is equal to about one molar equivalent of water per mole of (-)-O-desmethylvenlafaxine. In certain embodiments, Form F has a thermal gravimetric analysis thermogram similar to that of FIG. 30. In certain embodiments, when examined by thermal gravimetric analysis according to the methods described herein, Form F has a weight loss corresponding to about 5.8% of the total mass of the sample occurring in the range of about 25 to about 125 °C. In certain embodiments, Form F has a differential scanning calorimetry thermogram similar to that of FIG. 31. In certain embodiments, when examined by differential scanning calorimetry according to the methods described herein, Form F has an endotherm with an onset temperature at about 89 °C. In certain embodiments, the Form F crystal form of the hydrochloride salt of (-)-O-desmethylvenlafaxine has an X-ray powder diffraction pattern similar to that of FIG. 32. In certain embodiments, the Form F crystal form of the hydrochloride salt of (-)-O-desmethylvenlafaxine has an X-ray powder diffraction pattern similar to that of FIG. 33, which was simulated for Cu Kα radiation using single-crystal X-ray diffraction structural data obtained on Form F. Particular Form F crystal forms have major X-ray powder diffraction pattern peaks at about 14.4, 16.0, 17.4, 19.0, 25.5, 26.8 °2*θ* using Cu Kα radiation. In certain embodiments, the Form F crystal form has major X-ray powder diffraction peaks at one, two, three, four, five or six of the X-ray powder diffraction pattern positions of about 14.4, 16.0, 17.4, 19.0, 25.5, 26.8 °2*θ* using Cu Kα radiation. In certain embodiments, the Form F crystal form has both a water content of about 6% of the total mass of the sample and major X-ray powder diffraction peaks at one, two, three, four, five or six of the X-ray powder diffraction pattern positions of about 14.4, 16.0, 17.4, 19.0, 25.5, 26.8 °2*θ* using Cu Kα radiation. In certain embodiments, the Form F crystal form has an infrared spectrum similar to that of FIG. 34. In certain embodiments, the Form F crystal form has a Raman spectrum similar to that of FIG. 35. In certain embodiments, Form F has a dynamic vapor sorption isotherm similar to that of FIG. 36. In certain embodiments, when examined by dynamic vapor sorption according to the methods described herein, Form F exhibits a gain in mass of about 32% when increased from 5% to 95% relative humidity, followed by a loss in mass of about 33% when decreased from 95% to 5% relative humidity. In certain embodiments, when analyzed at approximately 173 K according to a method capable of determining unit cell parameters, e.g. single crystal X-ray diffraction, Form F has the following approximate unit cell parameters: a = 9.29 Å; *b* = 6.82 Å; c = 13.91 Å; α = 90°; β = 92.58°; γ = 90°; V = 879.95 Å³. In certain embodiments, Form F crystallizes in space group *P*2₁.

Form F of the hydrochloride salt of (-)-O-desmethylvenlafaxine can be made by any method of making Form F apparent to those of skill in the art based upon the teachings herein. In certain embodiments, Form F can be prepared by the dissolution of a solid form comprising the hydrochloride salt of (-)-O-desmethylvenlafaxine in a solvent mixture containing, for example, methanol and water, followed by subsequent crystallization brought about by the addition of an anti-solvent, such as methyl t-butyl ether.

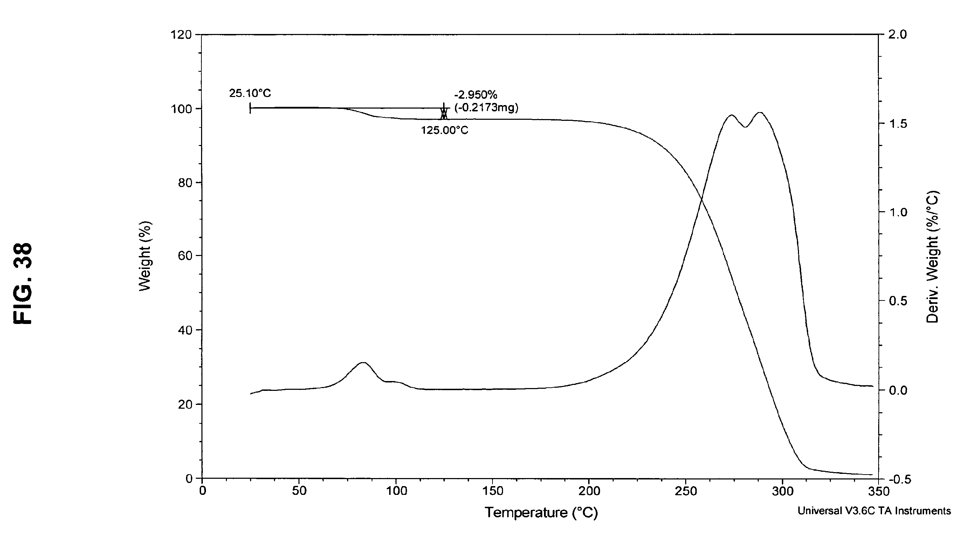

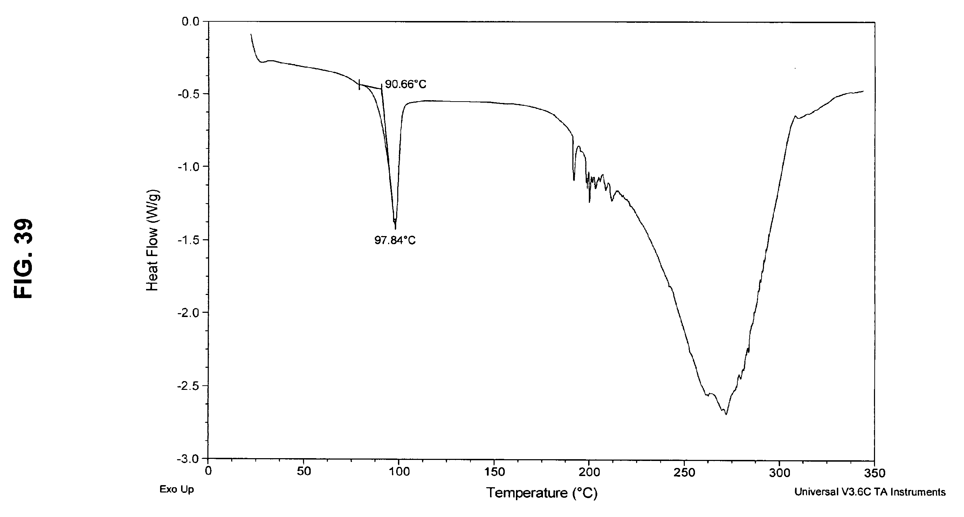

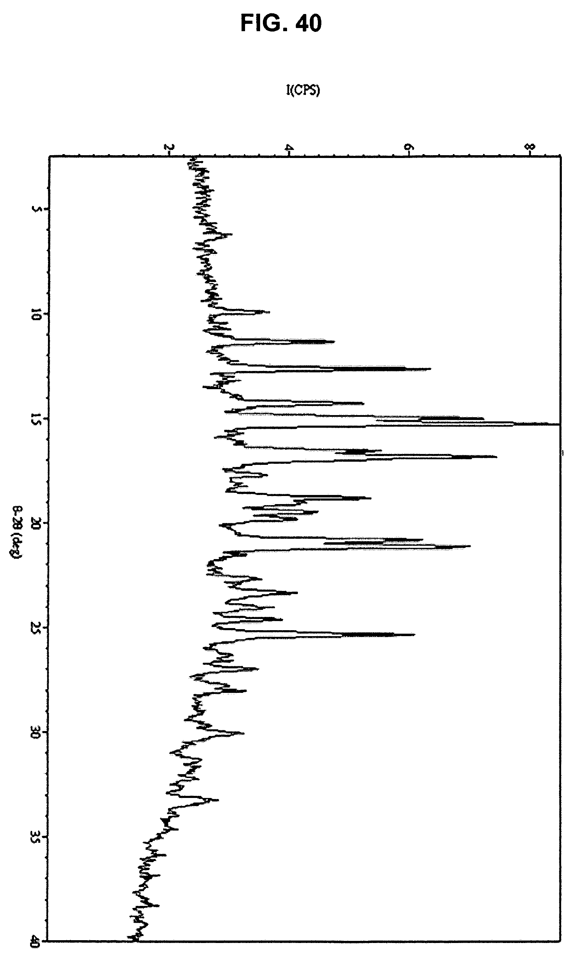

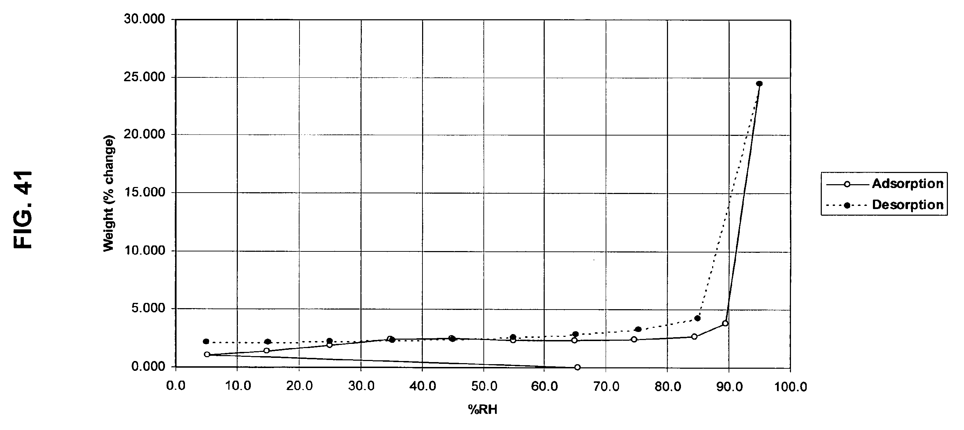

The present disclosure provides Form G, a crystal form of the addition of an anti-solvent, such as methyl t-butyl ether. disclosure monohydrochloride salt of (-)-O-desmethylvenlafaxine. In one embodiment, a sample of the Form G crystal form of the hydrochloride salt of (-)-O-desmethylvenlafaxine has a water content ranging between 0% and 6% of the total mass of the sample. In certain embodiments, the Form G crystal form has a water content of about 3% of the total mass of the sample, which is equal to about a half molar equivalent of water per mole of (-)-O-desmethylvenlafaxine. In certain embodiments, Form G has a thermal gravimetric analysis thermogram similar to that of FIG. 38. In certain embodiments, when examined by thermal gravimetric analysis according to the methods described herein, Form G has a weight loss corresponding to about 3.0% of the total mass of the sample occurring in the range of about 25 to about 125 °C. In certain embodiments, Form G has a differential scanning calorimetry thermogram similar to that of FIG. 39. In certain embodiments, when examined by differential scanning calorimetry according to the methods described herein, Form G has an endotherm with an onset temperature of about 91 °C. In certain embodiments, the Form G crystal form of the hydrochloride salt of (-)-O-desmethylvenlafaxine has an X-ray powder diffraction pattern similar to that of FIG. 40 using Cu Kα radiation. Particular Form G crystal forms have characteristic X-ray powder diffraction pattern peaks at about 12.6, 15.1, 16.7, 18.8, 21.0, 25.3 °2*θ* using Cu Kα radiation. In certain embodiments, the Form G crystal form has major X-ray powder diffraction peaks at one, two, three, four, five or six of the X-ray powder diffraction pattern positions of about 12.6, 15.1, 16.7, 18.8, 21.0, 25.3 °2*θ* using Cu Kα radiation. In certain embodiments, the Form G crystal form of the invention has both a water content of about 0 to 6% of the total mass of the sample and major X-ray powder diffraction peaks at one, two, three, four, five or six of the X-ray powder diffraction pattern positions of about 12.6, 15.1, 16.7, 18.8, 21.0, 25.3 °2*θ* using Cu Kα radiation. In certain embodiments, Form G has a dynamic vapor sorption isotherm similar to that of FIG. 41. In certain embodiments, when examined by dynamic vapor sorption according to the methods described herein, Form G exhibits a gain in mass of about 3% when increased from 5% to 90% relative humidity. In certain embodiments, when examined by dynamic vapor sorption according to the methods described herein, Form G exhibits a gain in mass of about 23% when increased from 5% to 95% relative humidity, and a loss in mass of about 22% when decreased from 95% to 5% relative humidity.

Form G of the hydrochloride salt of (-)-O-desmethylvenlafaxine can be made by any method of making Form G apparent to those of skill in the art based upon the teachings herein. In certain embodiments, Form G is prepared by drying the Form A crystal form of this invention, described above and in the examples below, over a suitable drying agent, such as, for example, P₂O₅.

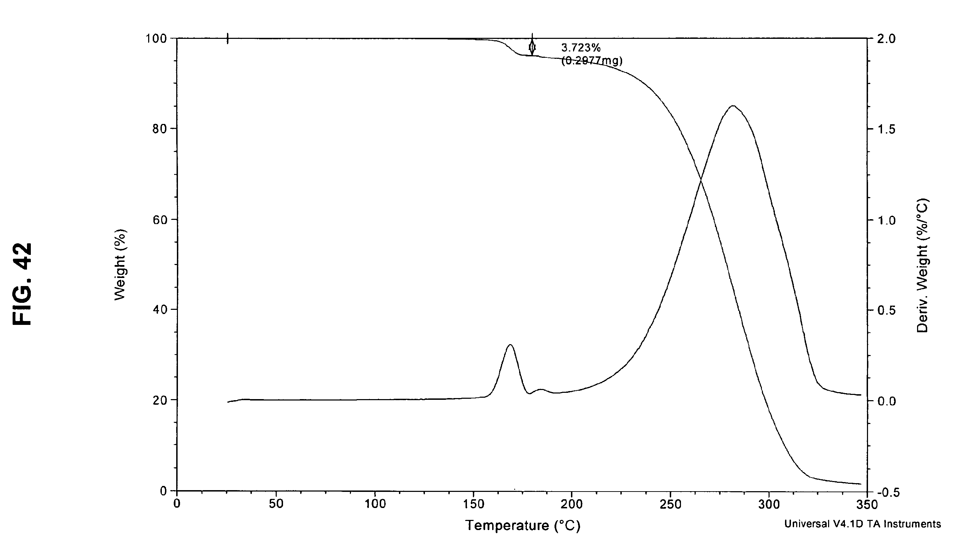

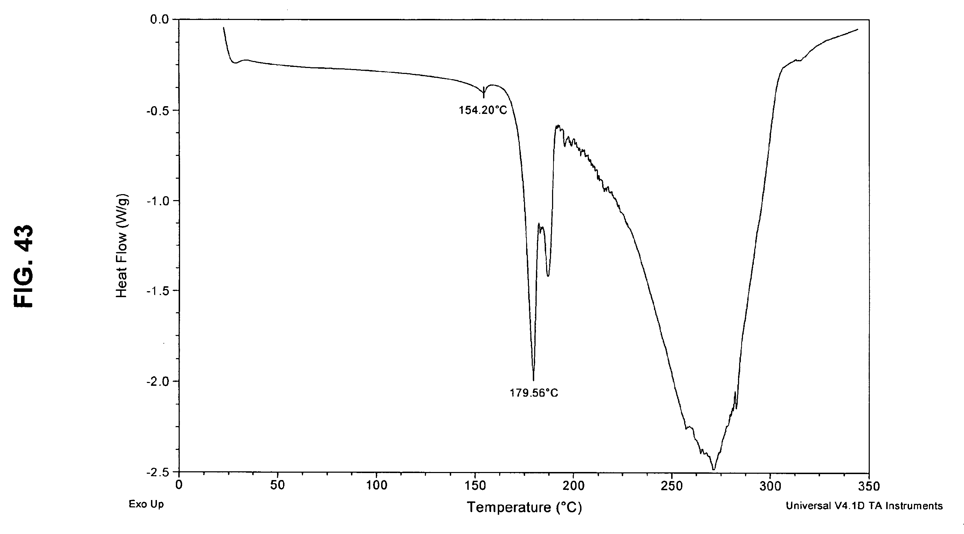

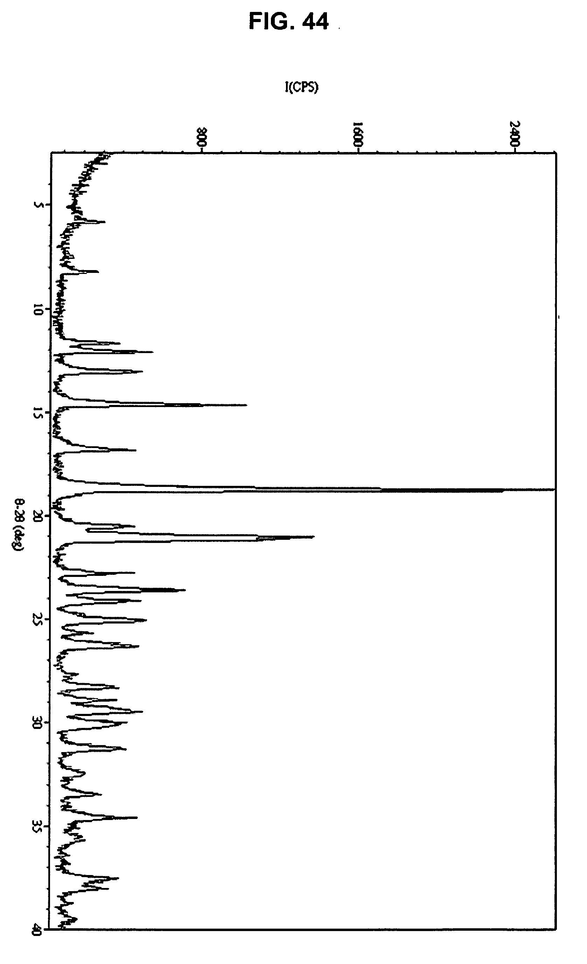

The present disclosure provides form H, a crystal form of example, P₂O₅. disclosure the monohydrochloride salt of (-)-O-desmethylvenlafaxine that contains the solvent acetone in the crystal lattice. In a particular embodiment, the acetone is present in the approximate ratio of 0.2 molar equivalents of acetone per mole of the hydrochloride salt of (-)-O-desmethylvenlafaxine. In terms of mass, this equates to an acetone content of approximately 4% of the total mass of a sample of Form H. In a certain embodiment, the acetone content of Form H ranges from about 2% to about 6% of the total mass of the sample of Form H. In certain embodiments, Form H has a thermal gravimetric analysis thermogram similar to that of FIG. 42. In certain embodiments, when examined by thermal gravimetric analysis according to the methods described herein, Form H has a weight loss corresponding to about 3.7% of the total mass of the sample occurring between about 25 and about 180 °C. In certain embodiments, the Form H crystal form has a differential scanning calorimetry thermogram similar to that of FIG. 43. In certain embodiments, when examined by differential scanning calorimetry according to the methods described herein, Form H has an endotherm with a peak temperature at about 180 °C. In certain embodiments, Form H has an additional endotherm with a peak temperature at about 154 °C. In certain embodiments, the Form H crystal form of the hydrochloride salt of (-)-O-desmethylvenlafaxine has an X-ray powder diffraction pattern similar to that of FIG. 44 using Cu Kα radiation. Particular Form H crystal forms have major X-ray powder diffraction pattern peaks at about 12.1, 14.6, 18.7, 21.1, 26.3 °2*θ* using Cu Kα radiation. In certain embodiments, the Form H crystal form has major X-ray powder diffraction pattern peaks at one, two, three, four or five of the X-ray powder diffraction pattern positions of about 12.1, 14.6, 18.7, 21.1, 26.3 °2*θ* using Cu Kα radiation. In certain embodiments, the Form H crystal form has both an acetone content of about 4% of the total mass of the sample and major X-ray powder diffraction pattern peaks at one, two, three, four, five or six of the X-ray powder diffraction pattern positions of about 12.1, 14.6, 18.7, 21.1, 26.3 °2*θ* using Cu Kα radiation.

Form H of the hydrochloride salt of (-)-O-desmethylvenlafaxine can be made by any method of making Form H apparent to those of skill in the art based upon the teachings herein. In certain embodiments, Form H can be obtained by stirring a slurry of the Form A crystal form of (-)-O-desmethylvenlafaxine in acetone at elevated temperature, followed by filtration.

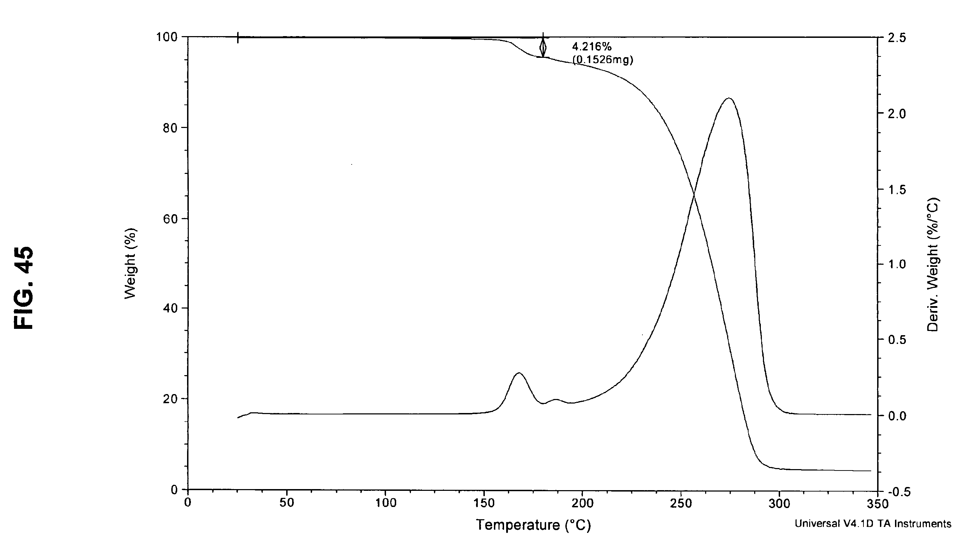

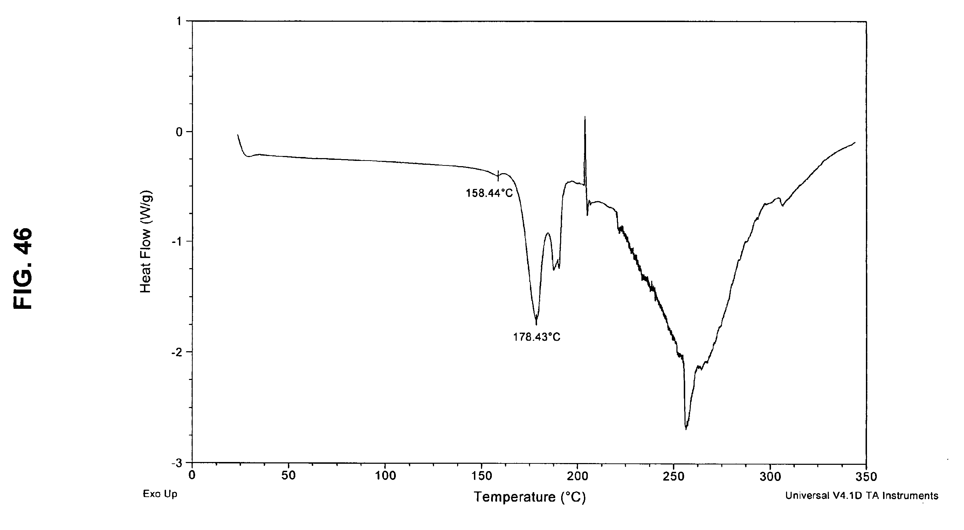

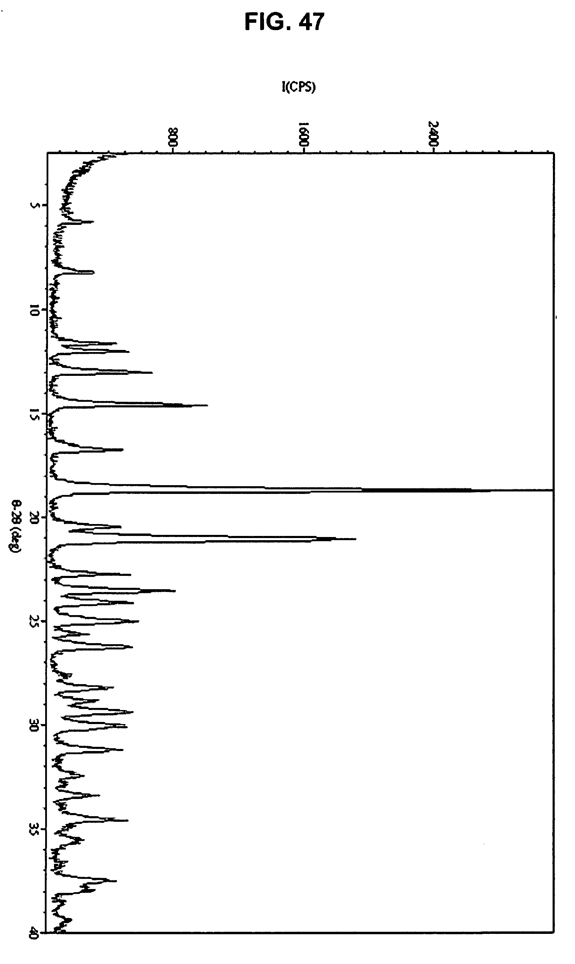

The present disclosure provides Form I, a crystal form of form of (-)-O-desmethylvenlafaxine in acetone at elevated temperature, followed by filtration. disclosure the monohydrochloride salt of (-)-O-desmethylvenlafaxine that contains the solvent isopropanol in the crystal lattice. In a particular embodiment, the isopropanol is present in Form I in the approximate ratio of 0.2 molar equivalents of isopropanol per mole of the hydrochloride salt of (-)-O-desmethylvenlafaxine. In terms of mass, this equates to an isopropanol content of approximately 4% of the total mass of a sample of Form I. In a certain embodiment, the isopropanol content of Form I ranges from about 2% to about 6% of the total mass of the sample of Form I. In certain embodiments, Form I has a thermal gravimetric analysis thermogram similar to that of FIG. 45. In certain embodiments, when examined by thermal gravimetric analysis according to the methods described herein, Form I has a weight loss corresponding to about 4.2% of the total mass of the sample occurring between about 25 and about 180 °C. In certain embodiments, the Form I crystal form has a differential scanning calorimetry thermogram similar to that of FIG. 46. In certain embodiments, when examined by differential scanning calorimetry according to the methods described herein, Form I has endotherm with a peak temperature at about 178 °C. In certain embodiments, Form I has an additional endotherm with a peak temperature at about 158 °C. In certain embodiments, the Form I crystal form of the hydrochloride salt of (-)-O-desmethylvenlafaxine has an X-ray powder diffraction pattern similar to that of FIG. 47 using Cu Kα radiation. Particular Form I crystal forms have major X-ray powder diffraction pattern peaks at about 13.0, 14.6, 18.7, 21.0, 23.5, 26.2 °2*θ* using Cu Kα radiation. In certain embodiments, the Form I crystal form has major X-ray powder diffraction pattern peaks at one, two, three, four, five or six of the X-ray powder diffraction pattern positions of about 13.0, 14.6, 18.7, 21.0, 23.5, 26.2 °2*θ* using Cu Kα radiation. In certain embodiments, the Form I crystal form has both an isopropanol content of about 4% of the total mass of the sample and major X-ray powder diffraction pattern peaks at one, two, three, four, five or six of the X-ray powder diffraction pattern positions of about 13.0, 14.6, 18.7, 21.0, 23.5, 26.2 °2*θ* using Cu Kα radiation.

Form I of the hydrochloride salt of (-)-O-desmethylvenlafaxine can be made by any method of making Form I apparent to those of skill in the art based upon the teachings herein. In certain embodiments, Form I can be obtained by precipitation from a solution of the hydrochloride salt of (-)-O-desmethylvenlafaxine in isopropanol followed by filtration. In certain embodiments, Form I can be obtained by fast evaporation of an isopropanol solution of the hydrochloride salt of (-)-O-desmethylvenlafaxine.

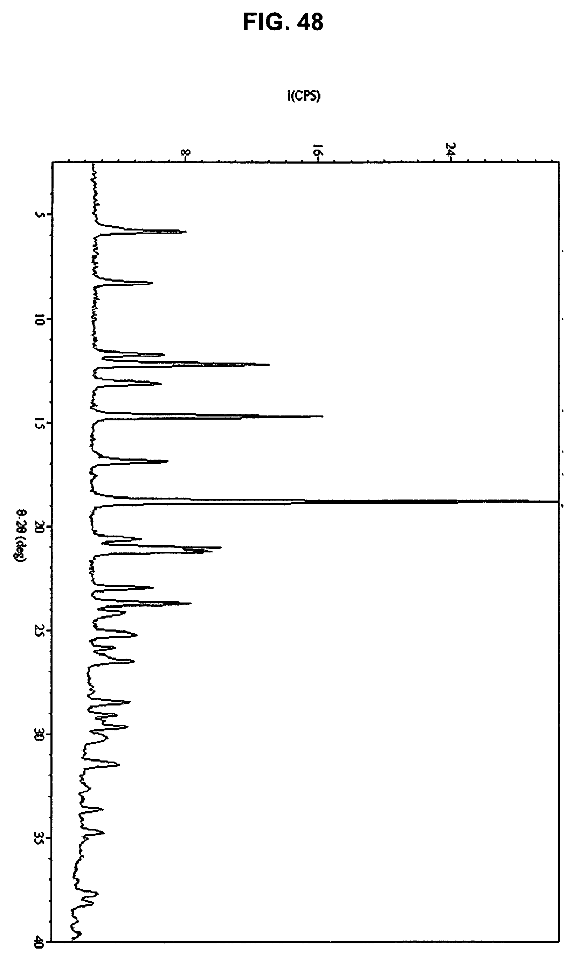

The present disclosure provides Form J, disclosure a crystal form of the monohydrochloride salt of (-)-O-desmethylvenlafaxine that contains the solvent acetonitrile in the crystal lattice. In a particular embodiment, the acetonitrile is present in Form J in the approximate ratio of 0.2 molar equivalents of acetonitrile per mole of the hydrochloride salt of (-)-O-desmethylvenlafaxine. In terms of mass, this equates to an acetonitrile content of approximately 3% of the total mass of a sample of Form J. In a certain embodiment, the acetonitrile content of Form J ranges from about 1% to about 5% of the total mass of the sample of Form J. In certain embodiments, the Form J crystal form of the hydrochloride salt of (-)-O-desmethylvenlafaxine has an X-ray powder diffraction pattern similar to that of FIG. 48 using Cu Kα radiation. Particular Form J crystal forms have major X-ray powder diffraction pattern peaks at about 12.2, 14.7, 16.9, 18.8, 21.0, 23.7 °2*θ* using Cu Kα radiation. In certain embodiments, the Form J crystal form major X-ray powder diffraction pattern peaks at one, two, three, four, five or six of the X-ray powder diffraction pattern positions of about 12.2, 14.7, 16.9, 18.8, 21.0, 23.7 °2*θ* using Cu Kα radiation. In certain embodiments, the Form J crystal form has both an acetonitrile content of about 3% of the total mass of the sample and major X-ray powder diffraction pattern peaks at one, two, three, four, five or six of the X-ray powder diffraction pattern positions of about 12.2, 14.7, 16.9, 18.8, 21.0, 23.7 "2*θ* using Cu Kα radiation.

Form J of the hydrochloride salt of (-)-O-desmethylvenlafaxine can be made by any method of making Form J apparent to those of skill in the art based upon the teachings herein. In certain embodiments, Form J can be obtained by precipitation from a solution of the hydrochloride salt of (-)-O-desmethylvenlafaxine in acetonitrile followed by filtration. In certain embodiments, Form J can be obtained by slurrying Form A of the hydrochloride salt of (-)-O-desmethylvenlafaxine in acetonitrile followed by filtration.

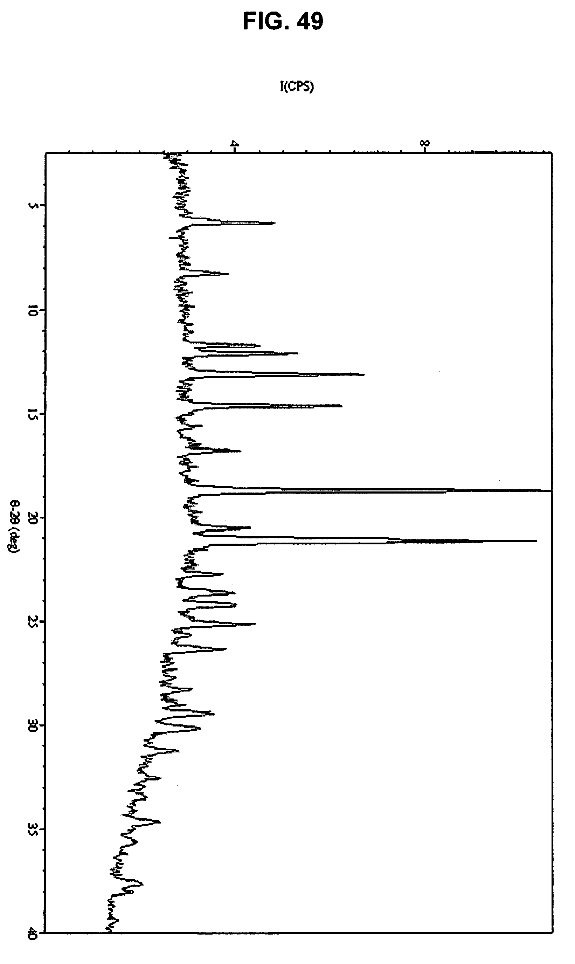

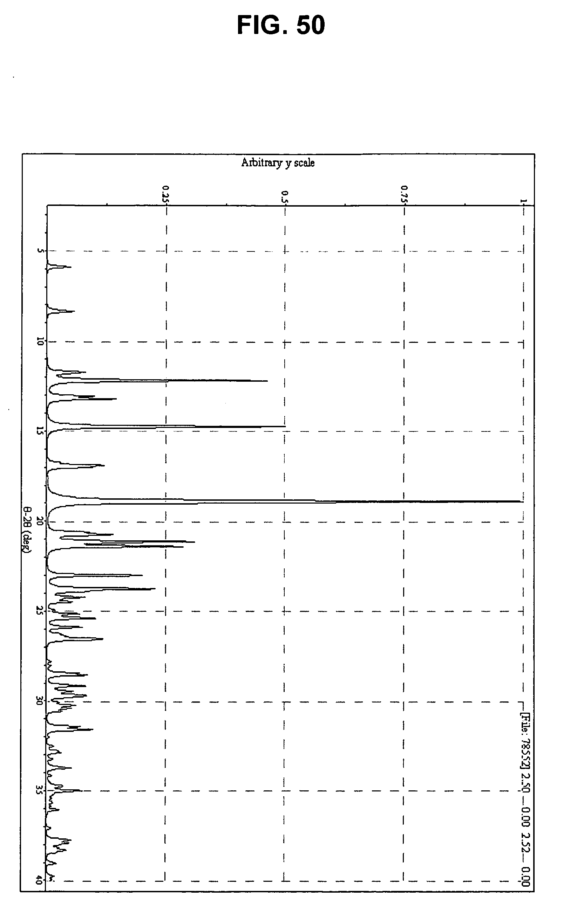

The present disclosure provides Form K, a crystal form of the monohydrochloride salt of (-)-O-desmethylvenlafaxine that contains the solvent ethanol in the crystal lattice. In a certain embodiment, the ethanol content of Form K is less than about 13% of the total mass of the sample of Form K, which is less than about one molar equivalent of ethanol per mole of the hydrochloride salt of (-)-O-desmethylvenlafaxine. In certain embodiments, the Form K crystal form of the hydrochloride salt of (-)-O-desmethylvenlafaxine has an X-ray powder diffraction pattern similar to that of FIG. 49. In certain embodiments, the Form K crystal form of the hydrochloride salt of (-)-O-desmethylvenlafaxine has an X-ray powder diffraction pattern similar to that of FIG. 50, which was simulated for Cu Kα radiation according to the methods described herein using single-crystal X-ray diffraction structural data obtained for Form K. Particular Form K crystal forms have major X-ray powder diffraction pattern peaks at about 12.1, 13.1, 14.6, 18.7, 21.0, 21.2 °2*θ* using Cu Kα radiation. In certain embodiments, the Form K crystal form has major X-ray powder diffraction pattern peaks at one, two, three, four, five or six of the X-ray powder diffraction pattern positions of about 12.1, 13.1, 14.6, 18.7, 21.0, 21.2 °2*θ* using Cu Kα radiation. In certain embodiments, the Form K crystal form has both an ethanol content of less than about 13% of the total mass of the sample and major X-ray powder diffraction pattern peaks at one, two, three, four, five or six of the X-ray powder diffraction pattern positions of about 12.1, 13.1, 14.6, 18.7, 21.0, 21.2 °2*θ* using Cu Kα radiation. In certain embodiments, when analyzed at approximately 173 K according to a method capable of determining unit cell parameters, e.g. single crystal X-ray diffraction, Form K has the following approximate unit cell parameters: a = 30.06 Å; *b* = 7.74 Å; *c* = 21.21 Å; α = 90°; β = 134.50°; γ = 90°; *V* = 3517.7 Å³. In certain embodiments, Form K crystallizes in space group C2.

Form K of the hydrochloride salt of (-)-O-desmethylvenlafaxine can be made by any method of making Form K apparent to those of skill in the art based upon the teachings herein. In certain embodiments, Form K can be obtained by crystallization from a solution of the hydrochloride salt of (-)-O-desmethylvenlafaxine in an ethanol/acetone solvent system.

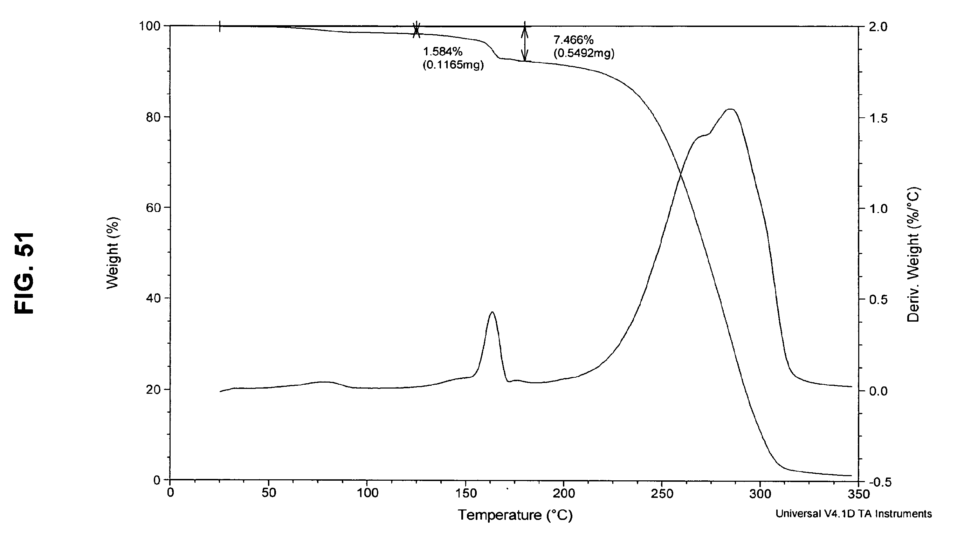

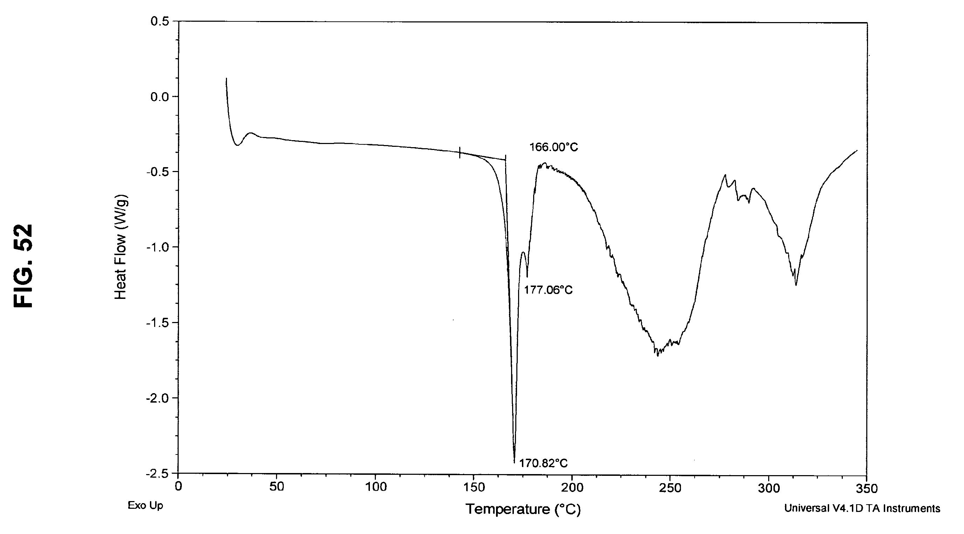

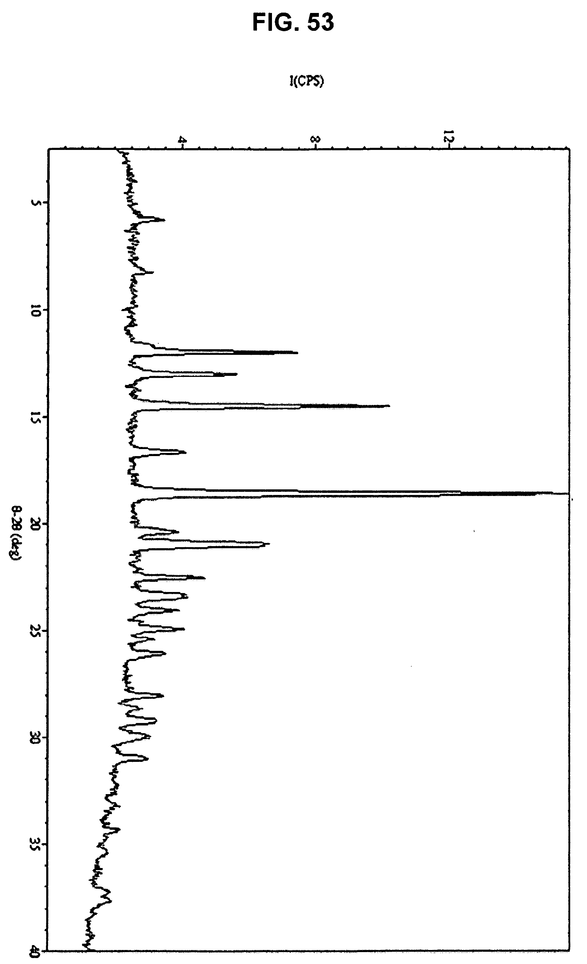

The present disclosure provides Form L, a crystal form of hydrochloride salt of (-)-O-desmethylvenlafaxine in an ethanol/acetone solvent system. disclosure the monohydrochloride salt of (-)-O-desmethylvenlafaxine that contains the solvent 2-methyl-tetrahydrofuran in the crystal lattice. In a particular embodiment, the 2-methyl-tetrahydrofuran is present in Form L in the approximate ratio of between 0.1 and 0.3 molar equivalents of 2-methyl-tetrahydrofuran per mole of the hydrochloride salt of (-)-O-desmethylvenlafaxine. In terms of mass, this equates to a 2-methyl-tetrahydrofuran content of between approximately 3% to 8% of the total mass of a sample of Form L. In a certain embodiment, the 2-methyl-tetrahydrofuran content of Form L ranges from about 1% to about 10% of the total mass of the sample of Form L. In certain embodiments, Form L has a thermal gravimetric analysis thermogram similar to that of FIG. 51. In certain embodiments, when examined by thermal gravimetric analysis according to the methods described herein, Form L has a weight loss corresponding to about 2% of the total mass of the sample occurring between about 25 and about 125 °C and a weight loss corresponding to about 7% of the total mass of the sample occurring between about 25 and about 180 °C. In certain embodiments, the Form L crystal form has a differential scanning calorimetry thermogram similar to that of FIG. 52. In certain embodiments, when examined by differential scanning calorimetry according to the methods described herein, Form L has an endotherm with an onset temperature at about 166 °C. In certain embodiments, the Form L crystal form of the hydrochloride salt of (-)-O-desmethyhienlafaxine has an X-ray powder diffraction pattern similar to that of FIG. 53 using Cu Kα radiation. Particular Form L crystal forms have major X-ray powder diffraction pattern peaks at about 12.0, 13.0, 14.5, 18.8, 21.0, 23.4 °2*θ* using Cu Kα radiation. In certain embodiments, the Form L crystal form has major X-ray powder diffraction pattern peaks at one, two, three, four, five or six of the X-ray powder diffraction pattern positions of about 12.0, 13.0, 14.5, 18.8, 21.0, 23.4 °2*θ* using Cu Kα radiation. In certain embodiments, the Form L crystal form has both an 2-methyl-tetrahydrofuran content of between about 1 % and about 10% of the total mass of the sample and major X-ray powder diffraction pattern peaks at one, two, three, four, five or six of the X-ray powder diffraction pattern positions of about 12.0, 13.0, 14.5, 18.8, 21.0, 23.4 °2*θ* using Cu Kα radiation.

Form L of the hydrochloride salt of (-)-O-desmethylvenlafaxine can be made by any method of making Form L apparent to those of skill in the art based upon the teachings herein. In certain embodiments, Form L can be obtained by slurrying Form A of the hydrochloride salt of (-)-O-desmethylvenlafaxine in 2-methyl-tetrahydrofuran followed by filtration.

The present disclosure provides crystal forms comprising the monohydrochloride salt of (-)-O-desmethylvenlafaxine that are members of an isostructural family of crystal forms. Members of a particular isostructural family of crystal forms share a certain structural similarity to the other members of that family with regard to features such as, for example, interplanar spacing in the crystal lattice. Structural similarity among members of a particular isostructural family results in some common characteristics of these crystal forms, for example, members of an isostructural family of crystal forms comprising the hydrochloric acid salt of (-)-O-desmethylvenlafaxine have similar X-ray powder diffraction patterns. Each member of a given isostructural family of crystal forms contains one or more types of organic solvents and/or water in the crystal lattice, or alternatively may be a desolvated solvate. Preferred solvents for inclusion in the crystal lattice of a member of a given isostructural family of crystal forms comprising the hydrochloric acid salt of (-)-O-desmethylvenlafaxine include common organic laboratory solvents and water. The disclosure provides desolvated forms comprising the hydrochloric acid salt of (-)-O-desmethylvenl include common disclosure solvate members of an isostructural family of crystal forms comprising the hydrochloric acid salt of (-)-O-desmethylvenlafaxine. A desolvated solvate is formed by the removal from the crystal lattice of one or more types of solvents and/or water; as a result, desolvated solvate crystal forms do not possess substantial quantities of solvent or water in the crystal lattice. Solvent removal may involve drying, heating and/or vacuum methods, as well as other methods apparent to those skilled in the art.

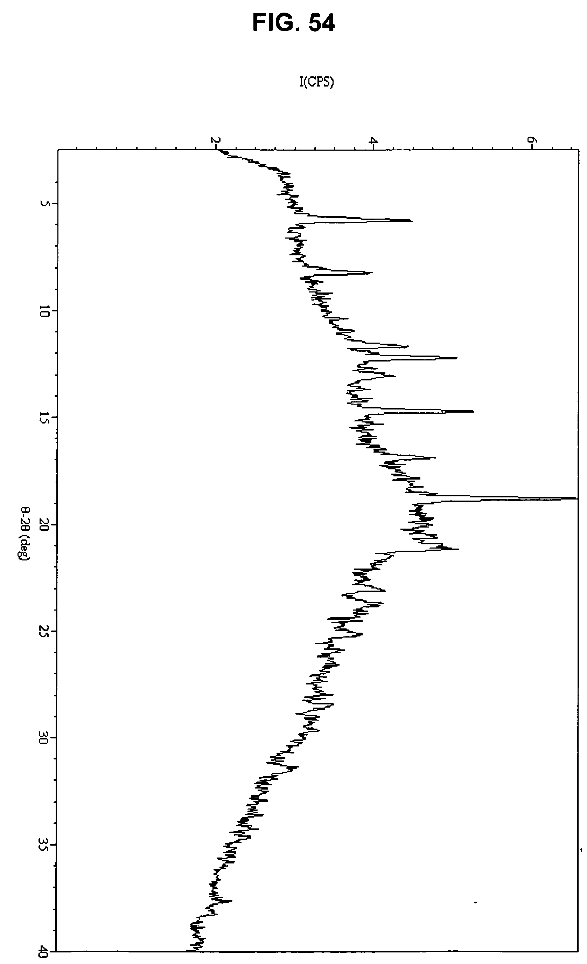

The disclosure provides crystal forms comprising the skilled in the art. disclosure phydrochloride salt of (-)-O-desmethylvenlafaxine belonging to isostructural family 1. In certain embodiments, members of isostructural family 1 are selected from the group consisting of Form B, Form C, Form H, Form I, Form J, Form K and Form L. In a certain embodiment, a member of isostructural family 1 of crystal forms has characteristic X-ray powder diffraction pattern peaks at about 5.8, 13.0, 14.6, 18.7, 21.1, 26.3°2θ using Cu Kα radiation. In certain embodiments, a member of isostructural family 1 of crystal forms comprising the hydrochloric acid salt of (-)-O-desmethylvenlafaxine has one, two, three, four, five or six characteristic X-ray powder diffraction pattern peaks at the positions of about 5.8, 13.0, 14.6. 18.7, 21.1 and 26.3 °2θ using Cu Kα radiation. Solvents for inclusion in the crystal lattice of a member of the isostructural family 1 of crystal forms comprising the hydrochloric acid salt of (-)-O-desmethylvenlafaxine include, but are not limited to, tetrahydrofuran, ethyl acetate, ethyl ether, acetone, isopropanol, acetonitrile, ethanol, water, and combinations thereof. Certain embodiments provide a member of the isostructural family 1 of crystal forms comprising the hydrochloric acid salt of (-)-O-desmethylvenlafaxine that is a desolvated solvate crystal form. In certain embodiments, a desolvated solvate belonging to isostructural family 1 may be prepared by the removal of any of the above-mentioned solvents from the crystal lattice of any solvated and/or hydrated crystal form member of isostructural family 1. In certain embodiments, the solvents ethyl acetate, diethyl ether and/or water are removed from the crystal lattice of Form C (a crystal form of the HCl salt of (-)-O-desmethylvenlafaxine, described herein) by way of heating in order to yield a desolvated solvate member of isostructural family 1. In certain embodiments, a desolvated solvate that is a member of isostructural family 1 has a XRPD pattern similar to that of FIG. 54 using Cu Kα radiation.

The disclosure provides crystal forms comprising disclosure the monohydrochloride salt of (-)-O-desmethylvenlafaxine belonging to isostructural family 2. In certain embodiments, members of isostructural family 2 are selected from the group consisting of Form E and Form L. In certain embodiments, a crystal form that is a member of isostructural family 2 has characteristic X-ray powder diffraction pattern peaks at about 11.9, 13.0, 14.4, 18.5, and 20.9 °2*θ* using Cu Kα radiation. In other embodiments , a crystal form of the hydrochloride salt of (-)-O-desmethylvenlafaxine that is a member of isostructural family 2 has one, two, three, four or five characteristic X-ray powder diffraction pattern peaks at the positions of about 11.9, 13.0, 14.4, 18.5, and 20.9 °2*θ* using Cu Kα radiation. Solvents for inclusion in the crystal lattice of a member of the isostructural family 2 of crystal forms comprising the hydrochloric acid salt of (-)-O-desmethylvenlafaxine include, but are not limited to, methyl t-butyl ether, 2-methyl-tetrahydrofuran, water and combinations thereof. Certain embodiments include desolvated solvate crystal forms that are members of isostructural family 2. In certain embodiments, desolvated solvate crystal forms that are members of isostructural family 2 are formed by the desolvation and/or dehydration of a crystal form that is a member of the isostructural family 2.

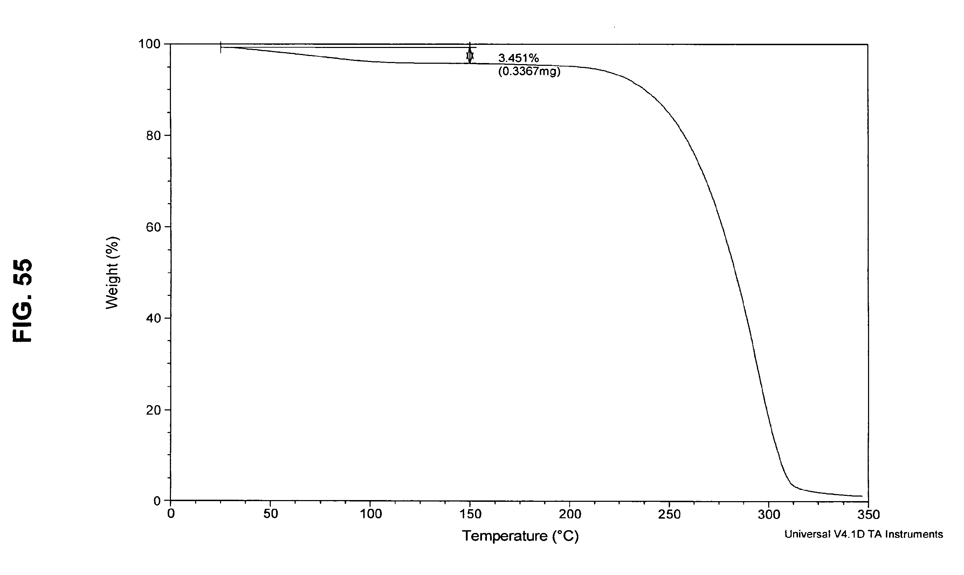

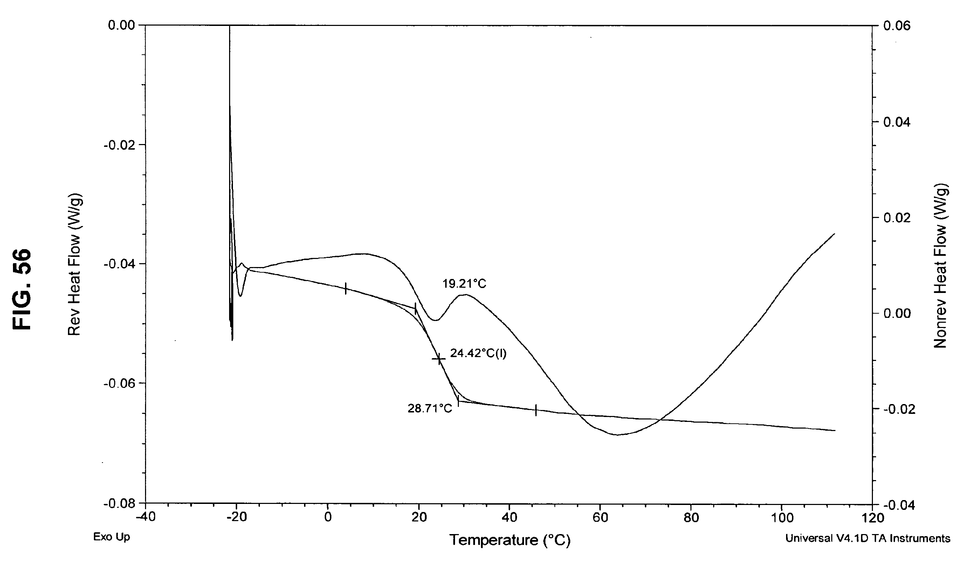

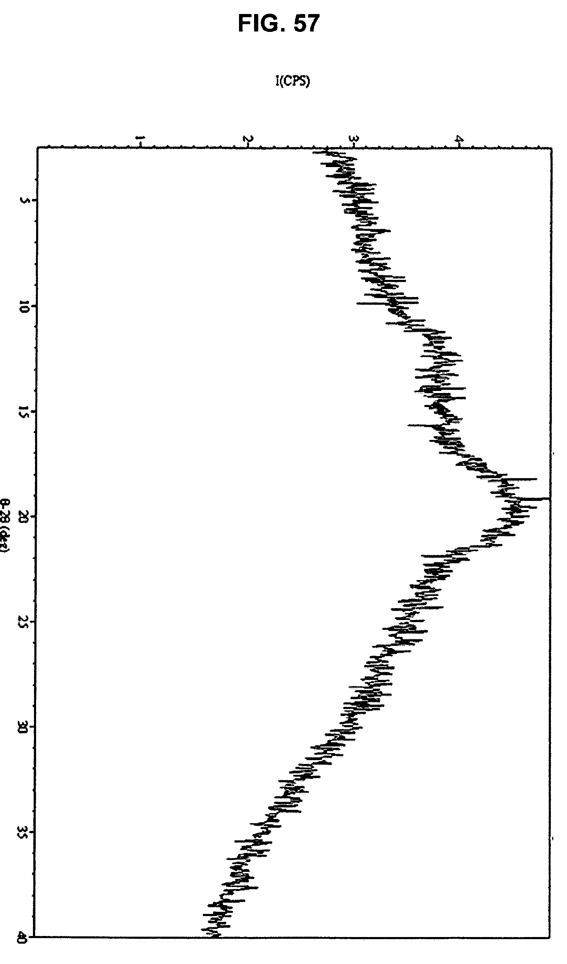

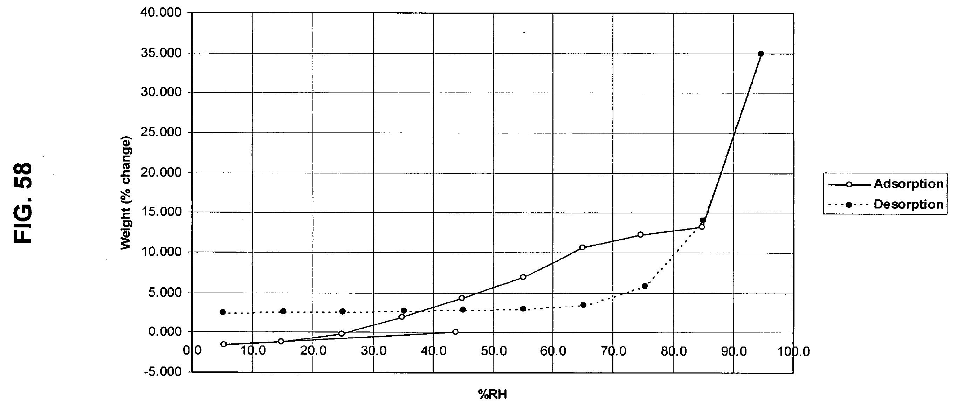

The present disclosure provides amorphous forms form that is a member of isostructural family 2. disclosure comprising (-)-O-desmethylvenlafaxine and salts thereof, having particular utility for the treatment, prevention or management of conditions and disorders including, but not limited to, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4^{th} edition (DSM-IV). Amorphous forms of the invention can be made following a preparation of (-)-O-desmethylvenlafaxine, as described herein. Particular embodiments include the amorphous form comprising either the free base or a salt of (-)-O-desmethylvenlafaxine. In certain embodiments, the amorphous forms are amorphous forms comprising pharmaceutically acceptable salts of (-)-=-desmethylvenlafaxine. amorphous forms comprising pharmaceutically acceptable salts of (-)-O-desmethylvenlafaxine. disclosure monohydrochloride salt of (-)-O-desmethylvenlafaxine ((-)-1-[2-(dimethylamino)-1-(4-hydroxyphenyl)ethyl]cyclohexanol monohydrochloride salt). In certain embodiments , the amorphous form of the hydrochloride salt of (-)-O-desmethylvenlafaxine has a thermal gravimetric analysis thermogram similar to that of FIG 55. In certain embodiments , the amorphous form of the hydrochloride salt of (-)-O-desmethylvenlafaxine has a modulated differential scanning calorimetry thermogram similar to that of FIG 56. In certain embodiments, when examined by modulated differential scanning calorimetry according to the methods described herein, the amorphous form of the hydrochloride salt of (-)-O-desmethylvenlafaxine has a glass transition temperature of approximately 24 °C. In certain embodiments, the amorphous form of the hydrochloride salt of (-)-O-desmethylvenlafaxine has an X-ray powder diffraction pattern similar to that of FIG. 57 using Cu Kα radiation. Particular embodiments of the amorphous form has an X-ray powder diffraction pattern do not contain sharp diffraction peaks in the range of about 2.5 to 40.0 °2*θ*, measured using Cu Kα radiation. In certain embodiments, the amorphous form of the hydrochloride salt of (-)-O-desmethylvenlafaxine has a dynamic vapor sorption isotherm similar to that of FIG. 58. In certain embodiments, when examined by dynamic vapor sorption according to the methods described herein, the amorphous form of the hydrochloride salt of the current invention exhibits a gain in mass of about 36% when increased from 5% to 95% relative humidity, followed by a loss in mass of about 32% when decreased from 95% to 5% relative humidity.

The amorphous form of the hydrochloride salt of (-)-O-desmethylvenlafaxine can be made by any method apparent to those of skill in the art to make the amorphous form based upon the teachings herein. In particular embodiments, a crystal form of a hydrochloride salt of (-)-O-desmethylvenlafaxine is dissolved in a solvent or solvent mixture, including solvents such as acetonitrile, isopropanol, ethyl acetate, ethanol, methanol, or the like, which is then evaporated to yield the amorphous forms of the invention. In certain embodiments, a solid form comprising a HCl salt of (-)-O-desmethylvenlafaxine is dissolved in a suitable solvent or solvents, e.g., water, then subjected to a freeze drying procedure to yield the amorphous forms of the invention. In certain embodiments, a hydrate, solvate, or anhydrous crystal form of the HCl salt of (-)-O-desmethylvenlafaxine, such as, for example, Form A, is heated to a temperature above its dehydration, desolvation, or melting temperature to yield the amorphous forms of the invention.

Certain embodiments provide mixtures, including physical mixtures and/or solid solutions, of solid forms comprising (-)-O-desmethylvenlafaxine or salts thereof. Certain embodiments provide mixtures of solid forms comprising the hydrochloride salt of (-)-O-desmethylvenlafaxine. Certain embodiments provide mixtures comprising an amorphous form of (-)-O-desmethylvenlafaxine HCl salt with one or more crystalline forms of (-)-O-desmethylvenlafaxine HCl salt. Certain embodiments provide mixtures comprising two or more, three or more, four or more, five or more, or six or more solid forms of the HCl salt of (-)-O-desmethylvenlafaxine comprising about 0.1%, 0.5%, 1%, 2%, 3%, 4%, 5%, 6%, 7%, 8%, 9%, 10%, 15%. 20%, 25%, 30%, 40%, 50%. 60%, 70%, 80%, 90%, 91%, 92%, 93%, 94%, 95%, 96%, 97%, 98%, 99%, 99.5% or 99.9% of an amorphous form of a HCl salt of (-)-O-desmethylvenlafaxine. Certain embodiments provide mixtures comprising two or more, three or more, four or more, five or more, or six or more crystal forms of a HCl salt of (-)-O-desmethylvenlafaxine, e.g., a mixture comprising Form A and Form F (-)-O-desmethylvenlafaxine HCl salt. Certain embodiments provide mixtures comprising two or more, three or more, four or more, five or more, or six or more crystal forms of the HCl salt of (-)-O-desmethylvenlafaxine comprising about 0.1%, 0.5%, 1%, 2%, 3%, 4%, 5%, 6%, 7%, 8%, 9%, 10%, 15%, 20%, 25%, 30%, 40%, 50%, 60%, 70%, 80%, 90%, 91%, 92%, 93%, 94%, 95%, 96%, 97%, 98%, 99%, 99.5% or 99.9% of one form, e.g. Form A, of a HCl salt of (-)-O-desmethylvenlafaxine. Certain embodiments provide a solid solution of crystal forms of a HCl salt of (-)-O-desmethylvenlafaxine, wherein characteristic structural features (e.g. lattice parameters, XRPD peak positions and/or one or more solvents of crystallization) comprising two or more, three or more, four or more, five or more, or six or more crystal forms which are members of a particular isostructural family of crystal forms, e.g. isostructural family 1, are exhibited upon analysis of the mixture.

### 5.2.3 Compositions

The present invention provides pharmaceutical compositions for use in the treatment, prevention or management of conditions and disorders including, but not limited to, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome; disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4th edition (DSM-IV). The compositions comprise the crystal form of claim 1 and a pharmaceutically acceptable diluent, excipient or carrier. In certain embodiments, a pharmaceutical composition of the invention comprises a pure crystal form of a salt of (-)-O-desmethylvenlafaxine. For example, a pharmaceutical composition of the invention comprises pure Form A monohydrate hydrochloric acid salt of (-)-O-desmethylvenlafaxine.

The pharmaceutical compositions for the administration of the crystalline forms of this invention may conveniently be presented in unit dosage form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the crystalline or amorphous form is included in an amount sufficient to produce the desired effect upon the process, condition or disease to be treated, prevented, or managed.

Any suitable route of administration can be employed for providing the patient with a therapeutically and/or prophylactically effective dose of the active ingredient of the present invention. For example, this invention comprises single unit dosage forms suitable for oral, mucosal (e.g., nasal, sublingual, vaginal, buccal, or rectal), parenteral (*e.g.,* subcutaneous, intravenous, bolus injection, intramuscular, or intraarterial), or transdermal administration to a patient. Examples of dosage forms include, but are not limited to: tablets; caplets; capsules, such as hard gelatin or soft elastic gelatin capsules; cachets; troches; lozenges; dispersions; suppositories; ointments; cataplasms (poultices); pastes; powders; dressings; creams; plasters; solutions; patches; aerosols (*e.g.,* nasal sprays or inhalers); gels; liquid dosage forms suitable for oral or mucosal administration to a patient, including suspensions (*e.g.,* aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

In practical use, an active ingredient can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, micro-crystalline cellulose, diluents, granulating agents, lubricants, binders, and disintegrating agents can be used in the case of oral solid preparations, preferably without employing the use of lactose. For example, suitable carriers include powders, capsules, and tablets, with the solid oral preparations being preferred over the liquid preparations.

The composition, shape, and type of dosage forms of the invention will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms encompassed by this invention will vary from one another will be readily apparent to those skilled in the art. *See, e.g.,* Remingfon's Pharmaceutical Sciences, 18th ed., Mack Publishing, Easton PA (1990).

### 5.2.3.1 Oral Dosage Forms

Pharmaceutical compositions of the invention that are suitable for oral administration can be presented as discrete dosage forms, such as, but are not limited to, tablets, chewable tablets, caplets, capsules, and liquids (e.g., flavored syrups). Such dosage forms contain predetermined amounts of active ingredients, and may be prepared by methods of pharmacy well known to those skilled in the art. *See generally,* Remington's Pharmaceutical Sciences, 18th ed., Mack Publishing, Easton PA (1990).

Typical oral dosage forms of the invention are prepared by combining the active ingredients in an intimate admixture with at least one excipient according to conventional pharmaceutical compounding techniques. Excipients can take a wide variety of forms depending on the form of preparation desired for administration.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

Disintegrants or lubricants can be used in pharmaceutical compositions and dosage forms of the invention. Production of pharmaceutical compositions or dosage forms in accordance with the present invention may require, in addition to the therapeutic drug ingredients, excipients or additives including, but not limited to, diluents, binders, lubricants, disintegrants, colorants, flavors, sweetening agents and the like or mixtures thereof. By the incorporation of these and other additives, a variety of dosage forms (*e.g*., tablets, capsules, caplets, troches and the like) may be made. These include, for example, hard gelatin capsules, caplets, sugar-coated tablets, enteric-coated tablets to delay action, multiple compressed tablets, prolonged-action tablets, tablets for solution, effervescent tablets, buccal and sublingual tablets, troches and the like.

Hence, unit dose forms or dosage formulation of a pharmaceutical composition of the present invention, such as a troche, a tablet or a capsule, may be formed by combining a desired amount of each of the active ingredients with one or more pharmaceutically compatible or acceptable excipients, as described below, in pharmaceutically compatible amounts to yield a unit dose dosage formulation the desired amount of each active ingredient. The dose form or dosage formulation may be formed by methods well known in the art.

Tablets are often a preferred dosage form because of the advantages afforded both to the patient (e.g., accuracy of dosage, compactness, portability, blandness of taste as well as ease of administration) and to the manufacturer (*e.g*., simplicity and economy of preparation, stability as well as convenience in packaging, shipping and dispensing). Tablets are solid pharmaceutical dosage forms containing therapeutic drug substances with or without suitable additives.

Tablets are typically made by molding, by compression or by generally accepted tablet forming methods. Accordingly, compressed tablets are usually prepared by large-scale production methods while molded tablets often involve small-scale operations. For example, there are three general methods of tablet preparation: (1) the wet-granulation method; (2) the dry-granulation method; and (3) direct compression. These methods are well known to those skilled in the art. *See, e.g.,* Remington's Pharmaceutical Sciences, 16th and 18th eds., Mack Publishing Co., Easton, Pa. (1980 and 1990). *See also* U.S. Pharmacopeia XXI, U.S. Pharmacopeial Convention, Inc., Rockville, Md. (1985).

Various tablet formulations may be made in accordance with the present invention. These include tablet dosage forms such as sugar-coated tablets, film-coated tablets, enteric-coated tablets, multiple-compressed tablets, prolonged action tablets and the like. Sugar-coated tablets (SCT) are compressed tablets containing a sugar coating. Such coatings may be colored and are beneficial in covering up drug substances possessing objectionable tastes or odors and in protecting materials sensitive to oxidation. Film-coated tablets (FCT) are compressed tablets which are covered with a thin layer or film of a water-soluble material. A number of polymeric substances with film-forming properties may be used. The film coating imparts the same general characteristics as sugar coating with the added advantage of a greatly reduced time period required for the coating operation. Enteric-coated tablets are also suitable for use in the present invention. Enteric-coated tablets (ECT) are compressed tablets coated with substances that resist dissolution in gastric fluid but disintegrate in the intestine. Enteric coating can be used for tablets containing drug substances which are inactivated or destroyed in the stomach, for those which irritate the mucosa or as a means of delayed release of the medication.

Multiple compressed tablets (MCT) are compressed tablets made by more than one compression cycle, such as layered tablets or press-coated tablets. Layered tablets are prepared by compressing additional tablet granulation on a previously compressed granulation. The operation may be repeated to produce multilayered tablets of two, three or more layers. Typically, special tablet presses are required to make layered tablets. *See*, for example, U.S. Pat. No. 5,213,738.

Press-coated tablets are another form of multiple compressed tablets. Such tablets, also referred to as dry-coated tablets, are prepared by feeding previously compressed tablets into a tableting machine and compressing another granulation layer around the preformed tablets. These tablets have all the advantages of compressed tablets, *i.e.,* slotting, monogramming, speed of disintegration, etc., while retaining the attributes of sugar coated tablets in masking the taste of the drug substance in the core tablet. Press-coated tablets can also be used to separate incompatible drug substances. Further, they can be used to provide an enteric coating to the core tablets. Both types of tablets (*i.e.,* layered tablets and press-coated tablets) may be used, for example, in the design of prolonged-action dosage forms of the present invention.

Pharmaceutical compositions or unit dosage, forms of the present invention in the form of prolonged-action tablets may comprise compressed tablets formulated to release the drug substance in a manner to provide medication over a period of time. There are a number of tablet types that include delayed-action tablets in which the release of the drug substance is prevented for an interval of time after administration or until certain physiological conditions exist. Repeat action tablets may be formed that periodically release a complete dose of the drug substance to the gastrointestinal fluids. Also, extended release tablets that continuously release increments of the contained drug substance to the gastrointestinal fluids may be formed.

In order for medicinal substances or therapeutic ingredients of the present invention, with or without excipients, to be made into solid dosage forms (*e.g*., tablets) with pressure, using available equipment, it is necessary that the material, in crystalline form, possess a number of physical characteristics. These characteristics can include, for example, the ability to flow freely, as a powder to cohere upon compaction, and to be easily released from tooling. Since many materials have none or only some of these properties, methods of tablet formulation and preparation have been developed to impart these desirable characteristics to the material which is to be compressed into a tablet or similar dosage form.

As noted, in addition to the drugs or therapeutic ingredients, tablets and similar dosage forms may contain a number of materials referred to as excipients or additives. These additives are classified according to the role they play in the formulation of the dosage form such as a tablet, a caplet, a capsule, a troche or the like. One group of additives include, but are not limited to, binders, diluents (fillers), disintegrants, lubricants, and surfactants. In one embodiment, the diluent, binder, disintegrant, and lubricant are not the same.

A binder is used to provide a free-flowing powder from the mix of tablet ingredients so that the material will flow when used on a tablet machine. The binder also provides a cohesiveness to the tablet. Too little binder will give flow problems and yield tablets that do not maintain their integrity, while too much can adversely affect the release (dissolution rate) of the drugs or active ingredients from the tablet. Thus, a sufficient amount of binder should be incorporated into the tablet to provide a free-flowing mix of the tablet ingredients without adversely affecting the dissolution rate of the drug ingredients from the tablet. With lower dose tablets, the need for good compressibility can be eliminated to a certain extent by the use of suitable diluting excipients called compression aids. The amount of binder used varies upon the type of formulation and mode of administration, and is readily discernible to those of ordinary skill in the art.

Binders suitable for use with dosage formulations made in accordance with the present invention include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone (povidone), methyl cellulose, pre-gelatinized starch, hydroxypropyl methyl cellulose, (*e.g*., Nos. 2208, 2906, 2910), microcrystalline cellulose or mixtures thereof. Suitable forms of microcrystalline cellulose can include, for example, the materials sold as AVICEL-PH-101, AVICEL-PH-103 and AVICEL-PH-105 (available from FMC Corporation, American Viscose Division, Avicel Sales, Marcus Hook, Pa., U.S.A.).

Fillers or diluents are used to give the powder (e.g., in the tablet or capsule) bulk so that an acceptable size tablet, capsule or other desirable dosage form is produced. Typically, therapeutic ingredients are formed in a convenient dosage form of suitable size by the incorporation of a diluent therewith. As with the binder, binding of the drug(s) to the filler may occur and affect bioavailability. Consequently, a sufficient amount of filler should be used to achieve a desired dilution ratio without detrimentally affecting release of the drug ingredients from the dosage form containing the filler. Further, a filler that is physically and chemically compatible with the therapeutic ingredient(s) of the dosage form should be used. The amount of filler used varies upon the type of formulation and mode of administration, and is readily discernible to those of ordinary skill in the art. Examples of fillers include, but are not limited to, lactose, glucose, sucrose, fructose, talc, calcium carbonate (*e.g.*, granules or powder), microcrystalline cellulose, powdered cellulose, dextrates, kaolin, mannitol, silicic acid, sorbitol, starch, pre-gelatinized starch, or mixtures thereof.

Disintegrants are used to cause the dose form (*e.g*., tablet) to disintegrate when exposed to an aqueous environment. Too much of a disintegrant will produce tablets which may disintegrate in the bottle due to atmospheric moisture. Too little may be insufficient for disintegration to occur and may thus alter the rate and extent of release of drug(s) or active ingredient(s) from the dosage form. Thus, a sufficient amount of disintegrant that is neither too little nor too much to detrimentally alter the release of the drug ingredients should be used to form the dosage forms made according to the present invention. The amount of disintegrant used varies based upon the type of formulation and mode of administration, and is readily discernible to the skilled artisan. Examples of disintegrants include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, clays, other algins, other celluloses, gums, or mixtures thereof.

When a dose form is desired that dissolves fairly rapidly upon administration to the subject, *e.g*., in the subject's stomach, a super disintegrant can be used, such as, but not limited to, croscarmellose sodium or sodium starch glycolate. The term "super disintegrant," as used herein, means a disintegrant that results in rapid disintegration of drug or active ingredient in the stomach after oral administration. Use of a super disintegrant can facilitate the rapid absorption of drug or active ingredient(s) which may result in a more rapid onset of action.

With regard to tablet dosage forms, adhesion of the dosage form ingredients to the punches of manufacturing machines (*e.g*., a tableting machine) must be avoided. For example, when drug accumulates on the punch surfaces, the tablet surfaces may become pitted and therefore unacceptable. Also, sticking of drug or excipients in this way requires unnecessarily high ejection forces when removing the tablet from the die. Excessive ejection forces may lead to a high breakage rate and increase the cost of production not to mention excessive wear and tear on the dies. In practice, it is possible to reduce sticking by wet-massing or by the use of high levels of lubricants, *e.g*., magnesium stearate. In addition, selection of drug salts and/or excipients with good anti-adhesion properties can also minimize these problems.

As noted, the lubricant is used to enhance the flow of the tableting powder mix to the tablet machine and to prevent sticking of the tablet in the die after the tablet is compressed. Too little lubricant will not permit satisfactory tablets to be made and too much may produce a tablet with a water-impervious hydrophobic coating, which can form because lubricants are usually hydrophobic materials such as stearic acid, magnesium stearate, calcium stearate and the like. Further, a water-impervious hydrophobic coating can inhibit disintegration of the tablet and dissolution of the drug ingredient(s). Thus, a sufficient amount of lubricant should be used that readily allows release of the compressed tablet from the die without forming a water-impervious hydrophobic coating that detrimentally interferes with the desired disintegration and/or dissolution of the drug ingredient(s).

Examples of suitable lubricants for use with the present invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laurate, agar, or mixtures thereof. Additional lubricants include, for example, a syloid silica gel (AEROSIL 200, manufactured by W.R. Grace Co. of Baltimore Md.), a coagulated aerosol of synthetic silica (marketed by Deaussa Co. of Piano, Tex.), CAB-O-SIL (a pyrogenic silicon dioxide product sold by Cabot Co. of Boston, Mass.) or mixtures thereof.

Surfactants are used in dosage forms to improve the wetting characteristics and/or to enhance dissolution, and are particularly useful in pharmaceutical compositions or dosage forms containing poorly soluble or insoluble drug(s) or active ingredients. Examples of surfactants include, but are not limited to, polyoxyethylene sorbitan fatty acid esters, such as those commercially available as TWEENs (*e.g*. Tween 20 and Tween 80), polyethylene glycols, polyoxyethylene stearates, polyvinyl alcohol, polyvinylpyrrolidone, poly(oxyethylene)/ poly(oxypropylene) block co-polyers such as poloxamers (*e.g*., commercially available as PLURONICs), and tetrafunctional block copolymers derived from sequential addition of propylene oxide and ethylene oxide to ethylenediamine, such as polyxamines (*e.g*., commercially as TETRONICs (BASF)), dextran, lecithin, dialkylesters of sodium sulfosuccinic acid, such as Aerosol OT, sodium lauryl sulfate, alkyl aryl polyether sulfonates or alcohols, such as TRITON X-200 or tyloxapol, *p*-isononylphenoxypoly (glycidol) (*e.g*. Olin-10G or Surfactant 10-G (Olin Chemicals), or mixtures thereof. Other pharmaceutically acceptable surfactants are well known in the art, and are described in detail in the Handbook of Pharmaceutical Excipients, 4th ed., Pharmaceutical Press, London, UK and American Pharmaceutical Association, Washington, DC (2003).

Other classes of additives for use with the pharmaceutical compositions or dosage forms of the present invention include, but are not limited to, anti-caking or antiadherent agents, antimicrobial preservatives, coating agents, colorants, desiccants, flavors and perfumes, plasticizers, viscosity increasing agents, sweeteners, buffering agents, humectants and the like.

Examples of anti-caking agents include, but are not limited to, calcium silicate, magnesium silicate, silicon dioxide, colloidal silicon dioxide, talc, or mixtures thereof.

Examples of antimicrobial preservatives include, but are not limited to, benzalkonium chloride solution, benzethonium chloride, benzoic acid, benzyl alcohol, butyl paraben, cetylpyridinium chloride, chlorobutanol, cresol, dehydroacetic acid, ethylparaben, methylparaben, hydroxyphenyl, phenylethyl alcohol, phenylmercuric acetate, phenylmercuric nitrate, potassium sorbate, propylparaben, sodium benzoate, sodium dehydroacetate, sodium propionate, sorbic acid, thimersol, thymol, or mixtures thereof.

Examples of colorants for use with the present invention include, but are not limited to, pharmaceutically acceptable dyes and lakes, caramel, red ferric oxide, yellow ferric oxide or mixtures thereof. Examples of desiccants include, but are not limited to, calcium chloride, calcium sulfate, silica gel or mixtures thereof.

Flavors that may be used include, but are not limited to, acacia, tragacanth, almond oil, anethole, anise oil, benzaldehyde, caraway, caraway oil, cardamom oil, cardamom seed, compound cardamom tincture, cherry juice, cinnamon, cinnamon oil, clove oil, cocoa, coriander oil, eriodictyon, eriodictyon fluidextract, ethyl acetate, ethyl vanillin, eucalyptus oil, fennel oil, glycyrrhiza, pure glycyrrhiza extract, glycyrrhiza fluidextract, lavender oil, lemon oil, menthol, methyl salicylate, monosodium glutamate, nutmeg oil, orange flower oil, orange flower water, orange oil, sweet orange peel tincture, compound orange spirit, peppermint, peppermint oil, peppermint spirit, pine needle oil, rose oil, stronger rose water, spearmint, spearmint oil, thymol, tolu balsam tincture, vanilla, vanilla tincture, and vanillin or mixture thereof.

Examples of sweetening agents include, but are not limited to, aspartame, dextrates, mannitol, saccharin, saccharin calcium, saccharin sodium, acesulfame potassium, sucralose (splenda(R) brand), sorbitol, sorbitol solution, or mixtures thereof.

Exemplary plasticizers for use with the present invention include, but are not limited to, castor oil, diacetylated monoglycerides, diethyl phthalate, glycerin, mono-and diacetylated monoglycerides, polyethylene glycol, propylene glycol, and triacetin or mixtures thereof. Suitable viscosity increasing agents include, but are not limited to, acacia, agar, alamic acid, aluminum monostearate, bentonite, bentonite magma, carbomer 934, carboxymethylcellulose calcium, carboxymethylcellulose sodium, carboxymethylcellulose sodium 12, carrageenan, cellulose, microcrystalline cellulose, gelatin, guar gum, hydroxyethyl cellulose, hydroxypropyl cellulose, hydroxypropyl methylcellulose (Nos. 2208; 2906; 2910), magnesium aluminum silicate, methylcellulose, pectin, polyvinyl alcohol, povidone, silica gel, colloidal silicon dioxide, sodium alginate, tragacanth and xanthan gum or mixtures thereof.

Buffering agents that may be used in the present invention include, but are not limited to, magnesium hydroxide, aluminum hydroxide and the like, or mixtures thereof. Examples of humectants include, but are not limited to, glycerol, other humectants or mixtures thereof.

The dosage forms of the present invention may further include one or more of the following: (1) dissolution retarding agents, such as paraffin; (2) absorption accelerators, such as quaternary ammonium compounds; (3) wetting agents, such as, for example, cetyl alcohol and glycerol monostearate; (4) absorbents, such as kaolin and bentonite clay; (5) antioxidants, such as water soluble antioxidants (*e.g*., ascorbic acid, cysteine hydrochloride, sodium bisulfate, sodium metabisulfate, sodium sulfite and the like), oil soluble antioxidants (*e.g*., ascorbyl palmitate, hydroxyanisole (BHA), butylated hydroxy toluene (BHT), lecithin, propyl gallate, alpha-tocopherol and the like); and (6) metal chelating agents, such as citric acid, ethylenediamine tetracetic acid (EDTA), sorbitol, tartaric acid, phosphoric acid and the like.

Dosage forms of the present invention, such as a tablet or caplet, may optionally be coated. Inert coating agents typically comprise an inert film-forming agent dispersed in a suitable solvent, and may further comprise other pharmaceutically acceptable adjuvants, such as colorants and plasticizers. Suitable inert coating agents, and methods for coating, are well known in the art, including without limitation aqueous or non-aqueous film coating techniques or microencapsulation. Examples of film-forming or coating agents include, but are not limited to, gelatin, pharmaceutical glaze, shellac, sucrose, titanium dioxide, carnauba wax, microcrystalline wax, celluloses, such as methylcellulose, hydroxymethyl cellulose, carboxymethycellulose, cellulose acetate phthalate, hydroxypropyl methylcellulose (*e.g*., Nos.: 2208, 2906, 2910), hydroxypropyl cellulose, hydroxypropyl methyl cellulose phthalate (*e.g*., Nos.: 200731, 220824), hydroxyethylcellulose, methylhydroxyethylcellulose, ethylcellulose which may optionally be crosslinked, and sodium carboxymethyl cellulose; vinyls, such as polyvinyl pyrrolidione, polyvinyl acetate phthalate,; glycols, such as polyethylene glycols; acrylics, such as dimethylaminoethyl methacrylate-methacrylate acid ester copolymer, and ethylacrylate-methylmethacrylate copolymer; and other carbohydrate polymers, such as maltodextrins, and polydextrose, or mixtures thereof. The amount of coating agent and the carrier vehicle (aqueous or non-aqueous) used varies upon the type of formulation and mode of administration, and is readily discernible to those of ordinary skill in the art.

A coating of a film forming polymer may optionally be applied to a tablet or caplet (e.g., a capsule shaped tablet) in accordance with the present invention by using one of several types of equipment such as a conventional coating pan, Accelacota, High-Cola or Worster air suspension column. Such equipment typically has an exhaust-system to remove dust and solvent or water vapors to facilitate quick drying. Spray guns or other suitable atomizing equipment may be introduced into the coating pans to provide spray patterns conducive to rapid and uniform coverage of the tablet bed. Normally, heated or cold drying air is introduced over the tablet bed in a continuous or alternate fashion with a spray cycle to expedite drying of the film coating solution.

The coating solution may be sprayed by using positive pneumatic displacement or peristaltic pump systems in a continuous or intermittent spray-dry cycle. The particular type of spray application is selected depending upon the drying efficiency of the coating pan. In most cases, the coating material is sprayed until the tablets are uniformly coated to the desired thickness and the desired appearance of the tablet is achieved. Many different types of coatings may be applied such as enteric, slow release coatings or rapidly dissolving type coatings for fast acting tablets. Preferably, rapidly dissolving type coatings are used to permit more rapid release of the active ingredients, resulting in hastened onset. The thickness of the coating of the film forming polymer applied to a tablet, for example, may vary. However, it is preferred that the thickness simulate the appearance, feel (tactile and mouth feel) and function of a gelatin capsule. Where more rapid or delayed release of the therapeutic agent(s) is desired, one skilled in the art would easily recognize the film type and thickness, if any, to use based on characteristics such as desired blood levels of active ingredient, rate of release, solubility of active ingredient, and desired performance of the dosage form.

A number of suitable film forming agents for use in coating a final dosage form, such as tablets include, for example, methylcellulose, hydroxypropyl methyl cellulose (PHARMACOAT 606 6 cps), polyvinylpyrrolidone (povidone), ethylcellulose (ETHOCEL 10 cps), various derivatives of methacrylic acids and methacrylic acid esters, cellulose acetate phthalate or mixtures thereof.

The method of preparation and the excipients or additives to be incorporated into dosage form (such as a tablet or caplet) are selected in order to give the tablet formulation the desirable physical characteristics while allowing for ease of manufacture (e.g., the rapid compression of tablets). After manufacture, the dose form preferably should have a number of additional attributes, for example, for tablets, such attributes include appearance, hardness, disintegration ability and uniformity, which are influenced both by the method of preparation and by the additives present in the tablet formulation.

Further, it is noted that tablets or other dosage forms of the pharmaceutical compositions of the invention should retain their original size, shape, weight and color under normal handling and storage conditions throughout their shelf life. Thus, for example, excessive powder or solid particles at the bottom of the container, cracks or chips on the face of a tablet, or appearance of crystals on the surface of tablets or on container walls are indicative of physical instability of uncoated tablets. Hence, the effect of mild, uniform and reproducible shaking and tumbling of tablets should be undertaken to insure that the tablets have sufficient physical stability. Tablet hardness can be determined by commercially available hardness testers. In addition, the *in vitro* availability of the active ingredients should not change appreciably with time.

The tablets, and other dosage forms of the pharmaceutical compositions of the present invention, such as dragees, capsules, pills and granules, may optionally be scored or prepared with coatings and shells, such as enteric coatings and other coatings well known in the pharmaceutical formulating art.

In one embodiment, it is desirable to use a lubricant in pharmaceutical composition and dosage forms of the invention that include an ARB that is poorly soluble or insoluble in water.

### 5.2.3.2 Parenteral Dosage Forms

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein (*i.e.,* the compounds of this invention) can also be incorporated into the parenteral dosage forms of the invention.

### 5.2.3.3 Transdermal, Topical and Mucosal Dosage Forms

Transdermal, topical, and mucosal dosage forms of the invention include, but are not limited to, ophthalmic solutions, sprays, aerosols, creams, lotions, ointments, gels, solutions, emulsions, suspensions, or other forms known to one of skill in the art. *See, e.g.,* Remington's Pharmaceutical Sciences, 16th and 18th eds., Mack Publishing, Easton PA (1980 & 1990); and Introduction to Pharmaceutical Dosage Forms, 4th ed., Lea & Febiger, Philadelphia (1985). Transdermal dosage forms include "reservoir type" or "matrix type" patches, which can be applied to the skin and worn for a specific period of time to permit the penetration of a desired amount of active ingredients.

Suitable excipients (*e.g*., carriers and diluents) and other materials that can be used to provide transdermal, topical, and mucosal dosage forms encompassed by this invention are well known to those skilled in the pharmaceutical arts, and depend on the particular tissue to which a given pharmaceutical composition or dosage form will be applied.

Depending on the specific tissue to be treated, additional components may be used prior to, in conjunction with, or subsequent to treatment with active ingredients of the invention. For example, penetration enhancers can be used to assist in delivering the active ingredients to the tissue.

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different crystal or amorphous forms of the active ingredients can be used to further adjust the properties of the resulting composition.

### 5.2.3.4 Compositions with Enhanced Stability

The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. This invention encompasses pharmaceutical compositions and dosage forms that contain little, if any, lactose other mono- or di-saccharides. As used herein, the term "lactose-free" means that the amount of lactose present, if any, is insufficient to substantially increase the degradation rate of an active ingredient.

Lactose-free compositions of the invention can comprise excipients that are well known in the art and are listed, for example, in the *U.S. Pharmacopeia* (USP) 25-NF20 (2002). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

This invention further encompasses anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. *See, e.g*., Carstensen, Drug Stability: Principles & Practice, 2nd ed., Marcel Dekker, New York, NY, pp. 379-80 (1995). In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers *(e.g*., vials), blister packs, and strip packs.

The invention further encompasses pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients.

### 5.2.3.5 Delayed Release Dosage Forms

Active ingredients of the invention can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566. Such dosage forms can be used to provide slow or controlled-release of one or more active ingredients using, for example, hydropropylmethyl cellulose, eudragit L-100, carnauba wax, magnesium stearate, methocel K4M CR, Surelease, Kollidon SR, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the compounds of this invention. The invention thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects.

Most controlled-release formulations are designed to initially release an amount of drug (active ingredient) that promptly produces the desired therapeutic effect, and gradually and continually release other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.Certain embodiments of the disclosure provide delayed-release formulations comprising (-)-O-desmethylvenlafaxine, including salts thereof. In certain embodiments, the delayed-release formulation comprising (-)-O-desmethylvenlafaxine, including salts thereof, has an advantageous, e.g., slowed, dissolution profile of the (-)-O-desmethylvenlafaxine, including salts and derivatives thereof. In certain embodiments, the formulation comprises the ingredients as described in the "Formulation of Premix" and "Final Formulation" as provided in the Examples section, infra.

### 5.2.3.6 Kits

In some cases, active ingredients of the invention are preferably not administered to a patient at the same time or by the same route of administration. This disclosure encompasses kits which, when used by the medical practitioner, can simplify the administration of appropriate amounts of active ingredients to a patient.

A typical kit of the invention comprises a single unit dosage form of the compounds of this invention, or a pharmaceutically acceptable salt, prodrug, solvate, hydrate, clathrate or stereoisomer thereof, and a single unit dosage form of another agent that may be used in combination with the compounds of this invention. Kits of the invention can further comprise devices that are used to administer the active ingredients. Examples of such devices include, but are not limited to, syringes, drip bags, patches, and inhalers.

Kits of the disclosure can further comprise pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

### 5.2.4 Use

In certain embodiments, utilizing optically pure or substantially optically pure (-)-O-desmethylvenlafaxine or salts thereof in the treatment, prevention and/or management of the conditions described herein results in clearer dose-related definitions of efficacy, diminished adverse effects, and accordingly an improved therapeutic index as compared to venlafaxine itself.

The present invention provides the use of a pharmaceutical composition for the manufacture of a medicament for treating itself. The use of the pharmaceutical composition for preventing or managing one or more diseases, disorders or conditions by administering a accordance therapeutic and/or prophylactic dose of a solid form comprising (-)-O-desmethylvenlafaxine in accordance with claim 1, i.e. a crystal form comprising a hydrochloride salt of (-)-O-desmethylvenlafaxine, to a subject, *e.g.* a human, in need of such treatment, prevention and/or management, wherein said diseases, disorders or conditions include, but are not limited to, affective disorders such as depression, bipolar and manic disorders, attention deficit disorder, attention deficit disorder with hyperactivity, anxiety disorders, panic disorder, social anxiety disorder, post traumatic stress disorder, premenstrual dysphoric disorder, borderline personality disorder, fibromyalgia, agoraphobia, obsessive compulsive disorder, anorexia and bulimia nervosa, obesity, weight gain, Gilles de la Tourette Syndrome, Shy-Drager syndrome, Alzheimer's disease, Parkinson's disease, epilepsy, narcolepsy, smoking cessation, drug craving, neurally mediated sexual dysfunction, pain, including chronic and neuropathic pain, cerebral function disorders, senile dementia, memory loss, amnesia/amnestic syndrome, disturbances of consciousness, coma, speech disorders, Lennox syndrome, autism, hyperkinetic syndrome, schizophrenia, migraine, obesity and weight gain, incontinence, chronic fatigue syndrome, sleep apnea, menopausal vasomotor symptoms such as hot flashes, disorders ameliorated by inhibition of neuronal monoamine uptake, related disorders, and the mental disorders described in the American Psychiatric Association's Diagnostic and Statistical Manual of Mental Disorders, 4th edition (DSM-IV).

The magnitude of a prophylactic or therapeutic dose of (-)-O-desmethylvenlafaxine (herein also referred to as an "active ingredient"), in the acute or chronic management of a disease, will vary with the severity of the condition to be treated and the route of administration. The dose, and in certain embodiments the dose frequency, will also vary according to age, body weight, response, and the past medical history of the individual patient. In certain embodiments of the invention, the recommended daily dose range for the conditions described herein lie within the range of from about 10 mg to about 1000 mg per day. In certain embodiments, the recommended daily dose is given as a single once-a-day dose, e.g. in the morning. In certain embodiments, the recommended daily dose is given as divided doses throughout the day taken with food. In certain embodiments, a daily dose range is from about 50 mg to about 500 mg per day. In certain embodiments, a daily dose range is between about 75 mg and about 300 mg per day. In certain embodiments, a daily dose range is from about 50 mg to about 200 mg per day. In certain embodiments, a daily dose range is from about 25 mg to about 250 mg per day. In managing the patient, the therapy should be initiated at a lower dose, perhaps about 50 mg to about 75 mg, and increased if necessary up to about 250 mg to about 325 mg per day as either a single dose or divided doses, depending on the patient's global response. If a dosage is increased, it is preferably done in intervals of about 75 mg separated by at least 4 days.

Because elimination of (-)-O-desmethylvenlafaxine from the bloodstream is dependant on renal and liver function, it is recommended that the total daily dose be reduced by at least 50% in patients with moderate hepatic impairment, and that it be reduced by 25% in patients with mild to moderate renal impairment. For patients undergoing hemodialysis, it is recommended that the total daily dose be reduced by 5% and that the dose be withheld until the dialysis treatment is completed. Because some adverse reactions have been reported for patients who took venlafaxine concurrently with, or shortly after, a monoamine oxidase inhibitor, it is recommended that (-)-O-desmethylvenlafaxine not be administered to patients currently taking such inhibitors. In general, the concurrent administration of the compounds of this invention with other drugs, particularly other serotonin uptake inhibitors, should be done with care. *See, e.g.,* von Moltke, et al. Biol. Psychiat., 41:377-380 (1997); and Sinclair, J. et al. Rev. Contemp. Pharmacother., 9:333-344 (1998).

The various terms "said amount being sufficient to alleviate the affective disorder," "said amount being sufficient to alleviate depression," "said amount being sufficient to alleviate attention deficit disorder," "said amount being sufficient to alleviate an obsessive-compulsive disorder," "said amount being sufficient to prevent or alleviate substance abuse," "said amount being sufficient to prevent or alleviate pre-menstrual syndrome," "said amount being sufficient to prevent or alleviate anxiety," "said amount being sufficient to prevent or alleviate an eating disorder," "said amount being sufficient to prevent or alleviate or prevent migraine," "said amount being sufficient to alleviate Parkinson's disease," "said amount being sufficient to alleviate epilepsy," "said amount being sufficient to alleviate obesity or weight gain," "an amount sufficient to achieve weight loss," "said amount being sufficient to bring about weight reduction in a human," "said amount being sufficient to alleviate pain," "said amount being sufficient to alleviate dementia," "said amount sufficient to alleviate said disorders ameliorated by inhibition of neuronal monoamine reuptake," "said amount is sufficient to alleviate cerebral function disorders," "said amount being sufficient to alleviate a mental disorder," wherein said disorders are selected from the group consisting of senile dementia, Alzheimer's type dementia, memory loss, amnesia/amnestic syndrome, disturbance of consciousness, coma, lowering of attention, speech disorders, Parkinson's disease, Lennox syndrome, autism, hyperkinetic syndrome, schizophrenia, and cerebrovascular diseases, such as cerebral infarction, cerebral bleeding, cerebral arteriosclerosis, cerebral venous thrombosis, head injuries, mental disorders including those described in the American Psychiatric Association's Diagnostic and Statistical Manual of Mental Disorders, 4th edition (DSM-IV), and the like, "said amount being sufficient to treat, prevent or manage incontinence" wherein said incontinence includes but is not limited to fecal, stress, urinary, urinary exertional, urge, reflex, passive and overflow incontinence, are encompassed by the above described dosage amounts and dose frequency schedule. Similarly, amounts sufficient to alleviate each of the above disorders but insufficient to cause adverse effects associated with venlafaxine are also encompassed by the above described dosage amounts and dose frequency schedule.

The invention is further defined by reference to the following non-limiting examples. It will be apparent to those skilled in the art that many modifications, both to materials and methods, may be practiced without departing from the purpose and interest of this invention.

### 6. EXAMPLES

### 6.1 MATERIALS AND PROCEDURES

Reagents and solvents used below can be obtained from commercial sources such as Aldrich Chemical Co. (Milwaukee, Wis., USA). Routine chemical analyses were conducted using NMR, MS and HPLC. Significant NMR peaks are tabulated by chemical shift and labeled with multiplicity (s, singlet; d, doublet; t, triplet; q, quartet; m, multiplet; br s, broad singlet) and number of protons. Mass spectrometry data is provided in relation to the mass of the parent ion, M. HPLC data is provided as a purity percentage.

X-ray powder diffraction (XRPD) data were obtained by one of the two following methods. In certain experiments, XRPD analyses were performed using an Inel XRG-3000 diffractometer equipped with a CPS (Curved Position Sensitive) detector with a 29 range of 120°. Real time data were collected using Cu-Kα radiation starting at approximately 2.5 °29 at a resolution of 0.03 °2*θ*. The tube voltage and amperage were set to 40 kV and 30 mA, respectively. The slit was set at 5 mm by 130 or 160 µm. Samples were prepared for analysis by packing them into thin-walled glass capillaries. Each capillary was mounted onto a goniometer head that is motorized to permit spinning of the capillary during data acquisition. The samples were analyzed for five or ten minutes. Instrument calibration was performed using a silicon reference standard.

In other experiments, XRPD analyses were performed using a Shimadzu XRD-6000 X-ray powder diffractometer using Cu-Ka radiation. The instrument is equipped with a long fine focus X-ray tube. The tube voltage and amperage were set to 40 kV and 40 mA, respectively. The divergence and scattering slits were set at 1° and the receiving slit was set at 0.15 mm. Diffracted radiation was detected by a Nal scintillation detector. A *θ*-2*θ* continuous scan at 3 °/min (0.4 sec/0.02° step) from 2.5 to 40 °2*θ* was used. A silicon standard was analyzed to check the instrument alignment. Data were collected and analyzed using XRD-6000 v. 4.1. Samples were prepared for analysis by placing them in a silicon sample holder.

In particular embodiments, the experimental error associated with a measured XRPD peak position is about ± 0.1 °2θ.

DSC was performed using a TA Instruments 2920 differential scanning calorimeter. The sample was placed into an aluminum DSC pan, and the weight accurately recorded. The pan was covered with a lid and then crimped, unless otherwise noted. The sample cell was equilibrated at 25 °C and heated under a nitrogen purge at a rate of 10 °C/min, up to a final temperature of 350 °C. Indium metal was used as the calibration standard.

Modulated DSC (MDSC) data were obtained on a TA Instruments 2920 differential scanning calorimeter equipped with a refrigerated cooling system (RCS). The sample was placed into an aluminum DSC pan, and the weight accurately recorded. The pan was covered with a lid and then crimped. MDSC data were obtained using a modulation amplitude of +/- 0.80 °C and a 60 second period with an underlying heating rate of 2 °C/min from -20 to 120 °C. The temperature and the heat capacity were calibrated using indium metal and sapphire as the calibration standards, respectively. The reported glass transition temperature was obtained from the half-height/inflection of the step change in the reversible heat flow versus temperature curve.

TGA analyses were performed using a TA Instruments 2950 thermogravimetric analyzer. Each sample was placed in an aluminum sample pan and inserted into the TGA furnace. Conditions of routine thermal gravimetric analysis involved equilibrating the furnace at 25 °C, followed by heating under nitrogen at a rate of 10 °C/min up to a final temperature of 350 °C. Modifications were made to this procedure in cases involving non-routine analysis, such as not equilibrating prior to heating, heating at different rates and heating to temperatures below 350 °C. Nickel and Alumel™ were used as calibration standards.

Hot stage microscopy was performed using a Linkam hot stage (model FTIR 600) mounted on a Leica DM LP microscope. Samples were observed using a 20x objective and a lambda plate with crossed polarizers. Samples were placed on a coverslip, and another coverslip was then placed over the sample. Each sample was visually observed as the stage was heated. Images were captured using a SPOT Insight™ color digital camera with SPOT Software v. 3.5.8. The hot stage was calibrated using USP melting point standards.

TG-IR analyses were acquired on a TA Instruments 2050 TGA analyzer interfaced to a Magna 560® FT-IR spectrophotometer (Thermo Nicolet) equipped with an Ever-Glo mid/far IR source, a potassium bromide (KBr) beamsplitter, and a deuterated triglycine sulfate (DTGS) detector. The TGA instrument was operated under a flow of helium at 90 and 10 cc/min for the purge and balance, respectively. Each sample was placed in a platinum sample pan, inserted into the TGA furnace, accurately weighed by the instrument, and the furnace was heated from 20 °C to 200 or 250 °C at a rate of 20 °C/min. The TGA instrument was started first, immediately followed by the FT-IR instrument. Each IR spectrum represents 32 co-added scans collected at a spectral resolution of 4 cm⁻¹. A background scan was collected before beginning the experiment. Wavelength calibration was performed using polystyrene. The TGA calibration standards were nickel and Alumel™. Volatiles were identified from a search of the High Resolution Nicolet TGA Vapor Phase spectral library.

Moisture sorption/ desorption data were collected on a VTI SGA-100 Vapor Sorption Analyzer. Sorption and desorption data were collected over a range of 5% to 95% relative humidity (RH) at 10% RH intervals under a nitrogen purge. For some samples, a step at 90% RH was added to the sorption cycle. Samples were not dried prior to analysis. Equilibrium criteria used for analysis were less than 0.0100% weight change in 5 minutes, with a maximum equilibration time of 3 hours if the weight criterion was not met. Data were not corrected for the initial moisture content of the samples. NaCl and PVP were used as calibration standards.

The IR spectra were acquired on a Magna-IR 860® FT-IR spectrophotometer (Thermo Nicolet) equipped with an Ever-Glo mid/far IR source, an extended range potassium bromide (KBr) beamsplitter, and a deuterated triglycine sulfate (DTGS) detector. An attenuated total reflectance (ATR) accessory (the Thunderdome™, ThermoSpectra-Tech), with a germanium (Ge) crystal was used for data acquisition. Each spectrum represents 256 co-added scans collected at a spectral resolution of 4 cm⁻¹. A background data set was acquired with air. A Log 1/*R* (*R* = reflectance) spectrum was acquired by taking a ratio of these two data sets against each other. Wavelength calibration was performed using polystyrene.

In certain experiments, FT-Raman spectra were acquired on a Raman accessory module interfaced to a Magna 860® FT-IR spectrophotometer (Thermo Nicolet). This module used an excitation wavelength of 1064 nm and an indium gallium arsenide (InGaAs) detector. Approximately 0.7 W of Nd:YVO₄ laser power was used to irradiate the sample. The samples were prepared for analysis by placing the material in a glass tube or capillary, which was then positioned in a gold-coated tube or capillary holder in the accessory. A total of 256 sample scans were collected at a spectral resolution of 4 cm⁻¹, using Happ-Genzel apodization. Wavelength calibration was performed using sulfur and cyclohexane.

In other experiments, FT-Raman spectra were acquired on an FT-Raman 960 spectrometer (Thermo Nicolet). This module used an excitation wavelength of 1064 nm and an indium gallium arsenide (InGaAs) detector. Approximately 1 W of Nd:YVO₄ laser power was used to irradiate the sample. The samples were prepared for analysis by placing the material in a glass tube or capillary, which was then positioned in a gold-coated tube or capillary holder in the accessory. A total of 256 sample scans were collected at a spectral resolution of 4 cm⁻¹, using Happ-Genzel apodization. Wavelength calibration was performed using sulfur and cyclohexane.

Coulometric Karl Fisher (KF) analysis for water determination was performed using a Mettler Toledo DL39 Karl Fischer titrator. Approximately 14-32 mg of sample was placed in the KF titration vessel containing Hydranal - Coulomat AD and mixed for 60 seconds to ensure dissolution. The sample was then titrated by means of a generator electrode which produces iodine by electrochemical oxidation: 2 l⁻ => l₂ + 2e. Three replicates were obtained to ensure reproducibility.

### 6.2 EXAMPLE 1: SYNTHESIS

Three different synthetic methods were used to obtain the compounds of this invention. A first method comprises the isolation of (-)-venlafaxine, followed by selective demethylation. A second method comprises separating a racemic mixture of (±)-O-desmethylvenlafaxine into its optically pure components. A third method comprises synthesizing (±)-O-benzyl-O-desmethylvenlafaxine, separating the resulting optically pure components, and debenzylating said components.

### 6.2.1 Synthesis and Resolution of Venlafaxine

### 6.2.1.1 1-[Cyano-(4-methoxyphenyl)methyl]cyclohexanol

A solution of 4-methoxybenzylnitrile (53.5 g, 0.36 mol) in 400 mL THF was cooled to -78 °C. followed by slow addition of a 2.0 M THF solution of lithium diisopropylamide (200 mL, 0.40 mol) maintaining the reaction temperature below -65 °C. The reaction was stirred at -78 °C for 30 minutes. Cyclohexanone (39.5 g, 0.40 mol) was added at a rate such that the reaction temperature did not rise above -65 °C. After the addition reaction was stirred at -78 °C for 2 hours, it was poured into 1 L saturated aqueous NH₄Cl containing ice. The mixture was stirred for 15 minutes and was extracted with ethyl acetate (4×200 mL). Combined ethyl acetate layer was washed with water (3×100 mL), brine (1×100 mL) and dried (Na₂SO₄). Ethyl acetate was evaporated in vacuo to give colorless solid that was trichurated with hexane. The precipitate was filtered, washed with hexane, and dried in vacuo to give a colorless solid (72.0 g, 80.7% yield). ¹H (CDCl₃); 7.30 and 6.90 (q, 4H), 3.80 (s, 3H), 3.75 (s, 1H), 1.55 (m, 10H); ³C (CDCl₃): 159.8, 130.8, 123.8, 120.0, 114.1, 72.9, 55.5, 49.5, 34.9, 25.3, 21.6.

### 6.2.1.2 1-(2-Amino-1-(4-methoxyphenyl)ethyl]cyclohexanol

A 3 L, three-neck flask equipped with a mechanical stirrer and a thermocouple was charged with 1-[cyano(4-methoxyphenyl)methyl]cyclohexanol (40.0 g, 0.16 mol) and 1 L methanol. To the resulting stirred solution was added cobalt chloride (42.4 g, 0.32 mol) and the reaction was stirred until a clear dark blue solution was obtained. Sodium borohydride (62.0 g, 1.63 mol) was added in small lots maintaining the reaction temperature below 35 °C. A dark black precipitate was formed along with vigorous evolution of gas as soon as sodium borohydride was added. After completion of addition the slurry was stirred at room temperature for 2 hours. TLC examination indicated complete disappearance of the starting material. The reaction was cooled in ice/water and 1 L of 3N HCl was added slowly. Reaction temperature was maintained below 25 °C. Reaction was stirred for 30 minutes after completion of the addition. Small amount of black precipitate was still observed. Methanol was removed in vacuo followed by extraction of the aqueous layer with ethyl acetate (3×300 mL). The aqueous layer was cooled in ice/water and was basified (pH paper) by slow addition of concentrated NH₄OH (ca. 600 mL). Reaction temperature was maintained below 25 °C. Reaction was extracted with ethyl acetate (4×200 mL). Combined ethyl acetate layer was washed with water (3×100 mL), brine (1×100 mL), and dried (Na₂SO₄). Ethyl acetate was evaporated in vacuo to give yellow gum (34.0 g, 83.6% yield).¹H (CDCl₃): 7.20 and 6.85 (q, 4H), 3.80 (s, 3H), 3.20 (m, 2H), 2.70 (t, 3H), 2.35 (br s, 3H), 1.40 (m, 10H); ¹³C (CDCl₃): 158.4, 132.6, 130.6, 113.7, 73.7, 56.7, 55.3, 42.4, 37.3, 34.5, 26.0, 21.9.

### 6.2.1.3 (±)-Venlafaxine

1-[2-Amino-1-(4-methoxyphenyl)ethyl]cyclohexanol (33.0 g, 0.13 mol) was dissolved in 88% formic acid (66.0 g, 55 mL, 1.43 mol) and water (330 mL) followed by addition of 37% aqueous formaldehyde (44.4 g, 41 mL, 1.48 mol). The resulting solution was refluxed for 20 hours, cooled to room temperature and was concentrated to 150 mL, adjusted to pH 2.0 with 3N HCl, and extracted with ethyl acetate (ca. 6×50 mL) until pink impurity was removed. The aqueous layer was cooled in ice/water and was basified by slow addition of 50% NaOH. The aqueous layer was extracted with ethyl acetate (3x75 mL). Combined ethyl acetate layer was washed with water (3×25 mL), brine (1×25 mL) and dried (Na₂SO₄). Ethyl acetate was evaporated in vacuo to give yellow gum that turned slowly in to pale yellow solid (34.0 g, 92.6% yield). ¹H (CDCl₃): 7.05 and 6.80 (q, 4H), 3.80 (s, 3H), 3.30 (t, 1 H), 2.95 (dd, 1 H), 2.35 (s, 6 H), 2.30 (dd, 1H), 1.30 (m, 10H); ¹³C (CDCl₃): 158.4, 132.9, 130.3, 113.5, 74.4, 61.4, 55.3, 51.8, 45.6, 38.2,31.3,26.2, 21.8, 21.5. MS (277, M+).

### 6.2.1.4 Tartrate Salts of Venlafaxine

Venlafaxine hydrochloride was prepared from venlafaxine freebase by the addition of hydrochloric acid in an appropriate solvent or according to US patent # 4535186.

A mixture of 2.50 kg of 1-(2-(dimethylamino)-1-(4-methoxyphenyl)ethyl) cyclohexanol hydrochloride, 16.8 kg of ethyl acetate and 14.0 kg of 1 N NaOH (aq) was stirred for 15 min. The stirring was stopped and the lower layer removed. The organic layer was washed twice with 3.5 kg of water. 2.4 kg of methanol and 1.78 kg of di-p-toluoyl-D-tartaric acid in 7.9 kg ethyl acetate was added. The mixture was stirred at reflux (∼65 °C) for 15 min and cooled to 55 °C. The solution was seeded with 43 g of (*R*)-1-(2-(dimethylamino)-1-(4-methoxyphenyl) ethyl)cyclohexanol-hemi-D-di-p-toluoyltartaric acid salt in 0.750 kg of ethyl acetate. The slurry was aged at 55°C for 15 minutes, cooled to 30°C over 110 minutes. The mixture was then cooled to 0°C over 1 hour and filtered. The cake was washed twice with a mixture of 0.23 kg of methanol and 2.3 kg ethyl acetate and dried *in vacuo* at 40-50°C to yield 1.53 kg of (R)-1-(2-(dimethylamino)-1-(4-methoxyphenyl) ethyl)cyclohexanol-hemi-D-di-p-toluoyltartaric acid salt (99.1% ee). ¹H NMR (DMSO-D₆): 0.80 - 1.6 (m, 10H), 2.35 (s, 9H), 2.86 (m, 1H), 2.98 (m, 1H), 3.33 (m, 1 H), 3.72 (s, 3H), 5.62 (s, 2H), 6.81 (d, 2H, J = 8.5 Hz), 7.12 (d, 2H, J = 8.5 Hz), 7.31 (d, 4H, J = 8.3 Hz), 7.85 (d, 4H, J = 8.3 Hz).

### 6.2.1.5 (-)-Venlafaxine

50 mL cold 2N NaOH was added to (R)-(-)-venlafaxine.di-p-toluoyl-D-tartrate salt (5.3 g, 8.0 mmol) and the aqueous layer was extracted with ethyl acetate (3×100 mL). Combined ethyl acetate layer was washed with cold 2N NaOH (1×25 mL) and water until aqueous wash was neutral. Ethyl acetate layer was dried (Na₂SO₄), ethyl acetate evaporated to give (-)-venlafaxine as colorless solid (2.2 g, quantitative yield), e.e. (HPLC): >99.95. ¹H, ¹³C and MS data as in (±)-venlafaxine.

### 6.2.2 Synthesis and Resolution of (-)-O-desmethylvenlafaxine

### 6.2.2.1 (±)-O-desmethylvenlafaxine

A solution of diphenylphosphine (3.0 g, 16.1 mmol) in 20 mL THF was cooled to-10 °C followed by slow addition of a 1.6 M THF solution of n-BuLi (12.7 mL, 20.2 mmol) at a rate such that reaction temperature did not rise above 0 °C. The reaction was stirred at 0 °C for 30 minutes. A solution of (±)-venlafaxine (1.0 g, 3.6 mmol) in 10 mL THF was added slowly at 0 °C. The reaction was stirred at 0° C for 15 minutes and allowed to warm to room temperature and stirred for 1 hour. It was then refluxed overnight. The reaction was cooled to room temperature and was poured slowly into 30 mL cold 3N HCl maintaining the temperature below 15 °C. After stirring for 10 minutes, the aqueous layer was extracted with ethyl acetate (3×30 mL). The aqueous layer was adjusted to pH 6.8-6.9 by slow addition of solid NaHCO₃. It was then saturated by adding NaCl and was extracted with ethyl acetate (6×30 mL). Combined ethyl acetate layer was dried (Na₂SO₄), ethyl acetate was evaporated in vacuo to give colorless solid. The solid was triturated with cold ethyl acetate, filtered, washed with cold ethyl acetate to give colorless solid (0.700 g, 73.8% yield). ¹H (DMSO, d₆): 9.30 (br s, 1 H); 7.10 and 6.80 (q, 4H), 5.60 (br s, 1H), 3.15(dd, 1H), 2.88 (t, 1H), 2.50(dd, 1 H), 2.30 (s, 6H), 1.35 (m, 10H); ¹³C (DMSO, d₆): 155.5, 131.7, 130.1, 114.4, 72.6, 60.4, 51.6, 45.3, 37.2, 32.4, 25.7, 21.2. MS: (264, M+1).

### 6.2.2.2 (-)-O-desmethylvenlafaxine

(-)-O-desmethylvenlafaxine was prepared from (-)-venlafaxine by following the procedure described above. (-)-O-desmethylvenlafaxine: colorless solid, [α]*_{D}*=-35.2 (c=0.25, EtOH), % purity (HPLC): >99% e.e. (HPLC): >99%. ¹H, ¹³C and MS data as in (±)-O-demethylvenlafaxine.

### 6.2.2.3 (-)-O-desmethylvenlafaxine directly from venlafaxine DTTA

(-)-O-desmethylvenlafaxine can also be directly prepared from (-)-venlafaxine-hemi-DTTA salt by following the procedure described below.

### 6.2.2.4 (-)-Venlafaxine

A mixture of 1.95 kg of (R)- 1-(2-(dimethylamino)-1-(4-methoxyphenyl)ethyl) cyclohexanol-hemi- D-di-p-toluoyltartaric acid salt, 12.03 kg of MTBE and 5.85 kg of 1 M NaOH (aq) was stirred for 15 min. The stirring was stopped and the lower layer removed. The organic layer was washed twice with 5.46 kg of water. The organic layer was concentrated to 5 L. 3.90 kg of anhydrous tetrahydrofuran was added and the mixture was distilled to a volume of 4.5 L to yield (-)-venlafaxine as a solution in THF.

A solution of lithium diphenylphosphide was generated by adding 6.2 kg of n-butyllithium, 1.6M (15%wt) to a mixture of 22.9 kg of tetrahydrofuran and 2.2 kg of diphenylphosphine. The THF solution of (-)-venlafaxine was added to the lithium diphenylphosphide. The mixture was stirred at 50 °C and hold until reaction is complete (approximately 24 hr). The mixture was cooled to 22 °C, 11.95 kg of DI water and 3.94 kg of 6N HCl was added. The mixture was stirred for 15 minutes, the stirring stopped and the upper organic phase was removed and discarded. The aqueous layer was washed twice with 7.98 kg of methylene chloride. The pH of the aqueous was adjusted to 9.5 using concentrated ammonium hydroxide. And 19.4 kg of 2-methyltetrahydrofuran was added. The mixture was heated to 65°C and the aqueous phase was removed. The organic was washed at 65 °C with 8 kg of water and the organic was concentrated to 4.5 L. 14.3 kg of ethyl acetate was added and the mixture stirred at 45-55 °C for 30 minutes. The mixture was stirred at 0 °C for 30 min. The slurry was filtered and the cake was washed twice with 2.8 kg of ethyl acetate. The solid was dried under vacuum (>28in Hg) at 40-50°C to yield 0.903 kg of (*R*)-O-desmethyl-1-(2-(dimethylamino)-1-(4-hydroxyphenyl)ethyl)cyclohexanol (99.3% ee). ¹H NMR (DMSO-d₆): 0.80-1.8 (m, 10H), 2.15 (s, 6H), 2.37 (dd, 1H, J = 12.5, 6.5), 2.73 (dd, 1H, J = 8.5, 6.5 Hz), 2.99 (dd, 1 H, J = 12.5, 8.5), 5.42 (br.s 1 H), 6.65 (d, 2H, J = 8.5 Hz), 6.97 (d, 2H, J = 8.5 Hz), 9.16 (br. s, 1 H).

### 6.2.2.5 (-)-O-desmethylvenlafaxine hydrochloride salt

80.4 g of (*R*)-O-desmethyl-1-(2-(dimethylamino)-1-(4-hydroxyphenyl)ethyl) cyclohexanol was charged to a 1 L round bottomed flask fitted with an overhead mechanical stirrer. 326.0 g of methanol and 80.3 g of a 15 % w/w aqueous solution of hydrochloric acid was added. The solution was stirred at 20 °C for 15 minutes and added to 1,797 g of heated (40 °C) methyl tert-butyl ether (MTBE). The mixture was stirred at 40 °C for 20 minutes and cooled to 20 °C. The mixture was stirred at 20 °C for one hour and seeded with 1.6 g of (*R*)-O-desmethyl-1-(2-(dimethylamino)-1-(4-hydroxyphenyl)ethyl)cyclohexanol hydrochloride monohydrate seeds as a slurry in 21 mL of MTBE. The contents of the 5 L flask were mixed at 20 °C for 2 hours to form a slurry. 1.6 L of MTBE was added to the 5 L flask and stirred at 20 °C for 2 hours. The mixture was filtered on a medium fritted funnel to isolate the product and the cake was washed twice (2 x 241.0 g) with MTBE. The filter cake was pulled dry under vacuum on the medium fritted funnel for 1 hour to yield 86.3 g of (*R*)-O-desmethyl 1-(2-(dimethylamino)-1-(4-methoxyphenyl)ethyl)cyclohexanol hydrochloride monohydrate. ¹H NMR (DMSO-d₆): 0.80 - 1.70 (10H, m), 2.60 (3H, s), 2.64 (3H, s), 3.00 (1H, dd, J= 9.3, 3.7 Hz), 3.46 (1H, br.t), 3.63 (1H, br.d), 4.52 (1H, s), 6.75 (2H,d,J= 8.3 Hz), 7.11 (2H, d, J = 8.3 Hz), 9.43 (1 H, br.s), 9.50 ( 1H,s).

### 6.2.3 Resolution of (-)-O-desmethylvenlafaxine

(-)-O-desmethylvenlafaxine was synthesized via the resolution of (±)-O-desmethylvenlafaxine.

1.0 g of (±)-O-desmethylvenlafaxine, 0.89 g (24 mmol) of (R)-1-phenyl-1-cyclohexyl-1-hydroxyacetic acid, 7.9 g of ethanol and 1.05 g of water were charged to a 25 mL flask. The mixture was stirred at 75 °C for 30 min and cooled to room temperature. The resulting solid was collected by filtration and washed with ethanol. The solid was dried to give 790 mg of (R)-1-(2-(dimethylamino)-1-(4-hydroxyphenyl)ethyl)cyclohexanol (R)-1-phenyl-1-cyclohexyl-1-hydroxyacetic aci salt (99.42 % ee). ¹HNMR (CDCl₃): 4.

### 6.2.3.1 (-)-O-desmethylvenlafaxine hydrochloride salt

(R)-1-(2-(Dimethylamino)-1-(4-hydroxyphenyl)ethyl)cyclohexanol (R)-1-phenyl-1-cyclohexyl-1-hydroxyacetic acid salt (2.0 g, 4 mmol) was dissolved into methanol (5.4 mL) and 15% (w/w) HCl in water (1.05 g). The methanol/hydrochloric acid solution was added with stirring to methyl-tert butyl ether (MTBE) (32 mL) at 35-40 °C. Following the addition of the methanol/hydrochloric acid solution the mixture was stirred at 35-40 °C for 60 min and cooled to room temperature. The mixture was seeded with (-)-O-desmethylvenlafaxine monohydrate and stirred at 20 °C for 3 hours. The solid was collected by filtration and washed with MTBE (20 mL). The solid was dried to give 730 mg of 1-(2-(dimethylamino)-1-(4-hydroxyphenyl)ethyl) cyclohexanol hydrochloride. The solid was analyzed (5.49% water, impurities <0.05%, DSC 101.38),¹H NMR (DMSO-d₆): 0.80 - 1.70 (10H, m), 2.60 (3H, s), 2.64 (3H,s), 3.00 (1 H, dd, J = 9.3, 3.7 Hz), 3.46 (1H, br.t), 3.63 (1H, br.d), 4.52 (1H, s), 6.75 (2H, d, J= 8.3 Hz), 7.11 ( 2H, d, J = 8.3 Hz), 9.43 (1H, br.s), 9.50 (1H, s).

(R)-1-Phenyl-1-cyclohexyl-1-hydroxyacetic acid is made according to the procedure outlined in Tetrahedron: Asymmetry 14 (2003) 3593, which is hereby incorporated by reference.

### 6.2.4 Synthesis and resolution of O-benzyl-O-desmethylvenlafaxine

(-)-O-Desmethylvenlafaxine was prepared by synthesizing and resolving (±)-O-desmethylvenlafaxine.

### 6.2.4.1 (±)-O-Benzyl-O-desmethylvenlafaxine

In certain embodiments, the following procedure was employed. 150 g of 2-(4-benzyloxy)phenyl-N,N-dimethylacetamide, 945 g (1062 mL) of THF was charged to a 5 L jacketed reactor. 550 mL of isopropylmagnesium chloride (2.0 M in tetrahydrofuran) was added and the mixture was stirred for 1 h. 115 g of cyclohexanone was added to the reactor and mixed for 1 h. 360 g of RedAI (sodium bis(2-methoxyethoxy)aluminum hydride-65% w/w in toluene) was added to the reactor and stirred for 16 h. When the reaction was complete the mixture was added to 2005 g of 22% w/w aqueous citric acid. 420 g (600 mL) of heptane was charged to the reactor and stirred for 15 min. The stirring was stopped and the top layer was removed. 250 g of 50% NaOH was added to adjust the pH to 9-10, followed by stirring. 1114 g (1500 mL) of MTBE was added to the reactor. The mixture was warmed to 45 ± 5°C to dissolve the solids. The stirring was stopped and the bottom layer was removed. The organic layer was washed twice with 750 g of water at 45 °C. 750 mL of MTBE was removed by distillation and 750 mL of methanol was added. About 750 mL of MTBE/methanol was removed by distillation and 300 g of methanol and 300 g of water were added. The slurry was cooled to 0 °C and stirred for 30 min. The slurry was filtered and the solid washed with 375 g of (4:1 methanol:water). The solid was dried to yield 161 g of 1-(2-(dimethylamino)-1-(4-benzyloxyphenyl)ethyl) cyclohexanol.

In certain embodiments, the following procedure was employed. To a 200 gallon reactor was charged 22.98 kg of 2-(4-benzyloxy)phenyl-N,N-acetamide and 145.1 kg of THF. With agitation, the temperature was adjusted to 5 °C to 10 °C. To the reactor was charged 82.9 kg of isopropylmagnesium chloride, 2.OM in THF, while maintaining the temperature between 5 °C to 35 °C. The lines were rinsed with 2.78 kg of THF. The contents were agitated for 61 minutes at 10 °C to 20 °C. To the reactor was added 9.31 kg of cyclohexanone while maintaining the temperature between 5 °C to 35 °C. The lines were rinsed with 2.77 kg of THF. The temperature was adjusted to 15 °C to 25 °C and the contents were agitated for 17 minutes at this temperature range after which the reaction was complete. To the reactor was charged 55.8 kg of sodium bis(2-methoxyethoxy)aluminum hydride (65 wt% in toluene) while maintaining the temperature at 15 °C to 35 °C. The contents were agitated for 10 h (<3% starting material remained). The reaction mixture was added to 334.1 kg of 22% citric acid solution cooled to 0 °C to 2 °C. THF (22.9 kg) and n-heptane (63.3 kg) were added to the reaction. The mixture was agitated for 15 minutes then the stirring was stopped and the phases were allowed to separate. The top layer was removed and the reactor was charged with 45.4 kg of 50% sodium hydroxide. The reactor was charged 169.9 kg of MTBE and the temperature was adjusted to 40-50 °C. The contents were agitated for 14 minutes and the agitation was stopped to allow the phases to separate for 15 minutes. The aqueous layer was removed and 115 L of USP purified water was added. The temperature was adjusted to 40 °C to 50 °C. The contents were agitated for 15 minutes and the agitation was stopped to allow the phases to separate for 13 minutes. The aqueous bottom layer was removed. The reactor was charged with 115 L of USP purified water and the temperature was adjusted to 40 °C to 50 °C. The contents were agitated for 15 minutes and the agitation was stopped to allow the phases to separate. The aqueous bottom layer was removed. The solution was distilled under vacuum to a final volume of 188 L. To the reactor was charged 115.2 kg of methanol and the solution was distilled under vacuum to a final volume of 131 L. To the reactor was charged 46.0 kg of methanol and 57 L of USP purified water. The temperature was adjusted to 0 °C. The slurry was stirred for 41 minutes at -5 °C to 5 °C and the mixture was filtered. The cake was washed with 46.2 kg of methanol and 11.6 kg of USP purified water (cooled to -5 °C to 5 °C). The wet cake (30.66 kg) was dried at 40-50 °C to yield 24.47 kg of 1-(2-(dimethylamino)-1-(4-benzyloxyphenyl)ethyl)cyclohexanol.

### 6.2.4.2 (-)-O-Benzyl-O-desmethylvenlafaxine-hemi-D-DTTA salt

In certain embodiments, the following procedure was employed. 160 g of 1-(2-(dimethylamino)-1-(4-benzyloxyphenyl)ethyl)cyclohexanol, 100 g of D-di-p-toluoyltartaric acid, 1.6 L of acetone, 150 g of water were added to a 5 L reactor and heated to 50 °C. The mixture was stirred at 50 °C for 15 minutes and cooled to 0 °C. The mixture was stirred at 0 °C for 120 minutes and filtered. The cake was washed with 600 mL of acetone and dried *in vacuo* at 40-50°C to yield 114.3 g of (*R*)-1-(2-(dimethylamino)-1-(4-benzyloxyphenyl)ethyl)cyclohexanol-di-p-toluoyl-D-tartaric acid salt.

In certain embodiments, the following procedure was employed. To a reactor was charged 60.64 kg of 1-(2-(dimethylamino)-1-(4-benzyloxyphenyl)ethyl) cyclohexanol, 42.01 kg of D-di-p-toluoyltartaric acid, 512.7 kg of acetone, and 61 L of USP purified water. The temperature was adjusted to 50 °C to 55 °C, and the contents were agitated at this temperature range for 16 minutes. The mixture was cooled to 36 °C and stirred at 36 °C for a period of 35 minutes. The mixture was cooled to -2 °C to 2 °C over 105 minutes and agitated for 122 minutes. The mixture was filtered and the cake was washed twice with acetone (122.0 kg and 121.8 kg), cooled to -5 °C to 5 °C. The wet cake (47.06 kg) was dried at 40-50 °C to yield 41.50 kg of (*R*)-1-(2-(dimethylamino)-1-(4-benzyloxyphenyl)ethyl) cyclohexanol di-p-toluoyl-D-tartaric acid salt.

### 6.2.4.3 (-)-O-Benzyl-O-desmethylvenlafaxine

In certain embodiments, the following procedure was employed. A 5 L flask was charged with 190 g of (R)-1-(2-(dimethylamino)-1-(4-benzyloxyphenyl)ethyl)cyclohexanol- D-di-p-toluoyltartaric acid salt, 703 g of MTBE and 870 g of 1 N NaOH. The mixture was stirred at 45 °C, the aqueous was removed and the organic layer was washed with water (475 g x 2). The MTBE layer was distilled to 450 mL, 703 g of methanol was added and the mixture was distilled to 450 mL. The slurry was diluted with 450 g of water and the mixture cooled to 0 °C. The mixture was filtered and washed with 435 mL of methanol/water to yield after drying 112 g of (*R*)-1-(2-(dimethylamino)-1-(4-benzyloxyphenyl)ethyl)cyclohexanol. ¹H NMR (DMSO-d₆): 0.8 - 1.6 (10H, m), 2.12 (6H, s), 2.41 (1H, dd, J = 6.9, 12.3 Hz), 2.77 (1H, t, J = 6.9 Hz), 2.94 (1H, dd, J = 7.9, 12.3 Hz), 5.05 (2H, s), 5.23 (1 H, s), 6.89 (2H, d, J = 8.7 Hz), 7.11 (2H, d, J = 8.7 Hz), 7.3-7.5 (5H, m).

In certain embodiments, the following procedure was employed. A reactor was charged 69.49 kg of (*R*)-1-(2-(dimethylamino)-1-(4-benzyloxyphenyl)ethyl) cyclohexanol di-p-toluoyl-D-tartaric acid salt, 265.7 kg of MTBE, and 328.9 kg of 1 N NaOH. The temperature was adjusted to 48-52 °C and the contents were agitated for 17 minutes. The agitation was stopped and the phases were allowed to separate for a period of 8 minutes. The aqueous bottom phase was removed, 180.1 kg of USP purified water was added and the temperature was adjusted to 48 °C to 52 °C. The mixture was agitated at 48 °C to 52 °C for 25 minutes, the agitation was stopped and the phases were allowed to separate for a period of 7 minutes. The aqueous bottom phase was removed and 179.8 kg of USP purified water was added. The mixture was agitated at 48 °C to 52 °C for 17 minutes, the agitation was stopped and the phases were allowed to separate for a period of 7 minutes. The aqueous bottom layer was removed and the solution was distilled to a final volume of 170 L. 265.7 kg of methanol was added and the solution was distilled to a final volume of 170 L. The reaction was cooled to 23 °C to 27 °C over 1 hour and 9 minutes. To the reactor was charged 170 L of USP purified water while maintaining the temperature at 23 °C to 33 °C during the addition. The slurry was cooled to -5 °C to 5 °C over 1 hour and 17 minutes and was agitated at -5 °C to 5 °C for 34 minutes. The mixture was filtered and the cake was washed with 64.7 kg of methanol and 82 L of USP purified water (cooled to -5 °C to 5 °C). The wet cake (46.61 kg) was dried at 40-50 °C to yield 42.47 kg of (*R*)-1-(2-(dimethylamino)-1-(4-benzyloxyphenyl)ethyl) cyclohexanol.

### 6.2.4.4 (-)-O-desmethylvenlafaxine hydrochloride salt

In certain embodiments, the following procedure was employed. 19.5 g of (R)-1-(2-(dimethylamino)-1-(4-benzyloxyphenyl)ethyl) cyclohexanol, 400 mg of 10% palladium on carbon, 58 mL of methanol, 4.5 mL of 37 wt% aqueous hydrochloric acid and 6.8 g of water were charged to a hydrogenation vessel. The mixture was reacted with 50 psi of hydrogen for 3 days. The resulting mixture was filtered and the catalyst was washed with 14 mL of methanol. The combined filtrate and mother liquor was added to 434 mL of MTBE at 40 °C. The mixture was cooled to 20 °C and seeded with (-)-O-desmethylvenlafaxine hydrochloride monohydrate. The mixture was stirred for 1 h at 20 °C and 290 mL of MTBE was added. The mixture was stirred for 2h, filtered and washed with MTBE (2 x 70 mL) to yield after drying 14.4 g of 1-(2-(dimethylamino)-1-(4-hydroxyphenyl)ethyl)cyclohexanol hydrochloride monohydrate. ¹H NMR (DMSO-d₆): 0.80 - 1.70 (10H, m), 2.60 (3H, s), 2.64 (3H, s), 3.00 (1H, dd, J = 9.3, 3.7 Hz), 3.46 (1H, br.t), 3.63 (1H, br.d), 4.52 (1H, s), 6.75 (2H, d, J= 8.3 Hz), 7.11 (2H, d, J = 8.3 Hz), 9.43 (1H, br.s), 9.50 (1 H, s). Solid-state analysis confirmed that this material was the Form A crystal form.

In certain embodiments, the following procedure was employed. 1.0 kg of (R)-1-(2-(dimethylamino)-1-(4-benzyloxyphenyl)ethyl)cyclohexanol, 20 g of 10% palladium on carbon, 1.76 kg of ethanol and 550 g of 20 wt% hydrochloric acid were charged to a hydrogenation vessel. The mixture was reacted with hydrogen until all the starting material had been consumed. The resulting mixture was filtered and the catalyst was washed with 380 g of ethanol. The combined filtrate and mother liquor was added to 4.96 kg of MTBE at 40 °C. The mixture was cooled to 20 °C and seeded with 20 g of 1-(2-(dimethylamino)-1-(4-hydroxyphenyl)ethyl) cyclohexanol hydrochloride monohydrate. The mixture was stirred for 2 h at 40 °C and 6.06 kg of MtBE was added over 8 hours. The mixture was stirred for 2h and cooled to 0 °C. The slurry was filtered, washed with 1.67 kg of MTBE:ethanol (5.4:1) and 1.66 kg of MTBE to yield after drying 1.1 kg of 1-(2-(dimethylamino)-1-(4-hydroxyphenyl)ethyl) cyclohexanol hydrochloride monohydrate. The resulting product was confirmed to be form A.

In certain embodiments, the following procedure was employed. The reactor was charged with 31.80 kg of (*R*)-1-(2-(dimethylamino)-1-(4-benzyloxyphenyl)ethyl) cyclohexanol. A slurry of 0.636 kg of palladium on carbon in 3.96 kg of ethanol (5% denatured with methanol). The atmosphere of the reactor was evacuated and replaced with nitrogen three times to exclude air. The reactor was charged with 56.1 kg of ethanol followed by 17.4 kg of 20 wt% HCl solution. The temperature was adjusted to 20-25 °C. The solids completely dissolved after 35 minutes while bubbling nitrogen through the solution to degas. The reaction was pressurized once with 45 to 55 psig of hydrogen, vented, and then repressurized to 45 to 55 psig with hydrogen. The mixture was agitated at 20 to 30 °C until the reaction was complete. The hydrogen was vented and the reactor was pressurized with nitrogen to 50 to 60 psig three times. The reaction mixture was filtered through a 3 µm filter and the reactor/filter was rinsed with 12.0 kg of ethanol. The combined filtrate/washes were added to 157.0 kg of MTBE at 40 °C to 45 °C. Seeds of the hydrochloride salt of (-)-O-desmethylvenlafaxine (635g) were added and the mixture was mixed for 2 hours and 4 minutes at 35 °C to 45 °C. 191.9 kg of MTBE was added over a period of 8 hours while maintaining the temperature at 35 °C to 45 °C. The mixture was stirred at 35 °C to 45 °C for 2 hours and 3 minutes and then the mixture was cooled to -5 °C to +5 °C over 2 hours and 3 minutes. The slurry was stirred at -5 °C to +5 °C for 37 minutes and filtered. The cake was washed with a mixture of MTBE (43.6 kg) and ethanol (8.2 kg), followed by 100% MTBE (52.3 kg). The wet cake (26.25 kg) was dried at not more than 25 °C to yield 25.42 kg of (R)-1-(2-(dimethylamino)-1-(4-hydroxyphenyl)ethyl) cyclohexanol hydrochloride monohydrate.

These examples provide various exemplary methods of synthesis of (-)-O-desmethylvenlafaxine. Alternate methods of synthesizing (-)-O-desmethylvenlafaxine will be apparent to those of skill in the art.

### 6.3 EXAMPLE 2: DETERMINATION OF POTENCY AND SPECIFICITY

Several methods useful for the determination of the potency and specificity of the compounds of this invention are disclosed in the literature. See, *e.g.,* Haskins, J. T. et al. Euro. J. Pharmacol. 115:139-146 (1985). In some embodiments, methods that have been found particularly useful are disclosed by Muth, E. A. et al. Biochem. Pharmacol. 35:4493-4497 (1986) and Muth, E. A. et al. Drug Develop. Res. 23:191-199 (1991).

### 6.3.1 Receptor Binding

Determination of receptor binding of the compounds of this invention preferably is performed by the methods disclosed by Muth *et al*., and using the protocols summarized in U.S. Patent Nos. 6,342,533 B1, 6,441,048 B1 and 6,911,479 B2.

The tissue homogenates used are preferably whole brain except cerebellum (histamine-1 and opiate binding), cortex (α₁ adrenergic receptor binding, monoamine uptake); and striatum (dopamine-2 and muscarinic cholinergic receptor binding).

### 6.3.2 Synaptosomal Uptake Studies

These studies may be performed using the modified methodology of Wood, M. D., and Wyllie, M. G. J. Neurochem. 37:795-797 (1981) as described in Muth et al. Biochem. Pharmacol. 35:4493-4497 (1986). Briefly, a P2 pellet is prepared from fresh rat brain tissue by sucrose density gradient centrifugation using a vertical rotor. For uptake studies, all components are dissolved in the following buffer: 135 mM NaCl, 5 mM KCl, 1.2 mM MgCl₂, 2.5 mM CaCl₂, 10 mM glucose, 1 mM ascorbic acid, 20 mM Tris, pH 7.4, gassed with O₂ for 30 min prior to use. Various concentrations of test drug are preincubated with 0.1 µM [³H]dopamine or 0.1 µM [³H]norepinephrine (130,000 dpm/tube) and 0.1 µM [¹⁴C]serotonin (7,500 dpm/tube) in 0.9 ml buffer for 5 min at 37 °C. One-tenth milliliter of synaptosomal preparation is added to each tube and incubated for a further 4 min at 37 °C. The reaction is then terminated by the addition of 2.5 ml buffer, after which the mixture was filtered under vacuum using cellulose acetate filters (0.45 µM pore size). The filters are then counted in a scintillation counter, and the results are expressed as pmoles uptake/mg protein/min. The IC50 values for uptake inhibition are calculated by linear regression of log [percent of Na⁺-dependent uptake] vs. log [concentration of test drug].

### 6.3.3 Reversal of Reserpine-Induced Hypothermia

Reversal of reserpine-induced hypothermia in male CF-1 mice (20-25 g., Charles River) may be performed according to an adaptation of the method of Askew, B. Life Sci. 1:725-730 (1963). Test compounds, suspended or solubilized in 0.25% Tween80® in water, are then administered i.p. at several dose levels to male mice (8/dose level) who had been treated 18 hr previously with 45.0 mg/kg reserpine s.c. A vehicle control group is run simultaneously with drug groups. Test compounds, vehicle, and reserpine are administered at a volume of 0.01 ml/g. Reserpine is solubilized by the addition of a small amount (approximately 4 drops) of concentrated acetic acid and then brought to the proper volume by the addition of distilled water. Rectal temperatures are recorded by a Yellow Springs Instruments thermistor probe at a depth of 2 cm. Measurements are taken 18 hr after reserpine pretreatment and at hourly intervals for 3 hr following administration of either test compound or vehicle.

Rectal temperatures for all time periods are subjected to a two-way analysis of variance for repeated measures with subsequent Dunnett's comparison to control values to determine the minimum effective dose (MED) for antagonizing reserpine-induced hypothermia.

### 6.3.4 Induction of Rat Pineal Noradrenergic Subsensitivity

Suitable rats are male Sprague-Dawley rats (250-300 g, Charles River) which should be maintained in continuous light throughout all experiments so as to attenuate the diurnal fluctuation in beta-adrenergic receptor density in the pineal gland and to maintain a consistent supersensitive response to noradrenergic agonists (Moyer, J. A. et al. Soc. Neurosci. Abstract 10:261 (1984)). After 2 days of continuous light exposure, the rats are then injected twice daily with either saline or test compound (10 mg/kg i.p.) for 5 days (total of 9 injections). Another group of rats should receive saline injections twice daily for 4 days followed by a single injection of test compound (10 mg/kg i.p.) on the 5th day. One hour following the final injection of test compound or saline, animals are administered either 0.1 % ascorbic acid (controls), or isoproterenol (2 µmol/kg i.p. in 0.1 % ascorbic acid). Rats are decapitated 2.5 minutes later, the time at which preliminary experiments have shown that the isoproterenol-induced increases in cyclic AMP levels in pineal glands are maximal (Moyer, J. A. et al. Mol. Pharmacol. 19:187-193 (1981)). Pineal glands are removed and frozen on dry ice within 30 seconds to minimize any post-decapitation increase in cAMP concentration.

Prior to radioimmunoassay for cAMP, the pineal glands are placed in 1 ml of ice-cold 2.5% perchloric acid and sonicated for approximately 15 seconds. The sonicate is then centrifuged at 49.000 g for 15 min at 4 °C and then resulting supernatant fluid is removed, neutralized with excess CaCO₃, and centrifuged at 12,000 g for 10 min at 4 °C. The cAMP content of the neutralized extract may be measured by a standard radioimmunoassay using 1251-labeled antigen and antiserum (New England Nuclear Corp., Boston, Mass.; Steiner, A. L. et al. J. Biol. Chem. 247:1106-1113 (1972)). All unknown samples should be assayed in duplicate and compared to standard solutions of cAMP prepared in a 2.5% perchloric acid solution that had been neutralized with CaCO₃. Results are expressed as pmol cAMP/pineal, and statistical analyses are performed by analysis of variance with subsequent Student-Newman-Keuls tests.

### 6.3.5 Single Unit Electrophysiology

The firing rates of individual neurons of the locus coeruleus (LC) or dorsal raphe nucleus (DR) in the chloral-hydrate anesthetized rat are measured using single-barreled glass micro-electrodes as previously described in LC. Haskins, J. T. et al. Eur. J. Pharmacol. 115:139-146 (1985). Using the stereotaxic orientation of Konig, J. F. R., and Klippel, R. A. The rat brain: A stereotaxic atlas of the forebrain and lower parts of the brain stem Baltimore: Williams and Wilkins (1963), the electrode tips should be lowered via a hydraulic microdrive from a point 1.00 mm above the locus coeruleus (AP 2.00 mm caudal to the interaural line and 1.03 mm lateral to midline). Drugs are administered i.v. through a lateral tail vein cannula. Only one cell should be studied in each rat in order to avoid residual drug effects.

### 6.4 EXAMPLE 3: ORAL FORMULATION

The pharmaceutical compositions of this invention may be administered in a variety of ways, including orally.

### 6.4.1 Hard Gelatin Capsule Dosage Forms

The ingredients of suitable capsule forms of the pharmaceutical compositions of this invention may be found in U.S. Patent Nos. 6,342,533 B1, 6,441,048 B1 and 6,911,479 B2.

The active ingredient (optically pure (-)-O-desmethylvenlafaxine, or pharmaceutically acceptable salt thereof) is sieved and blended with the excipients listed. The mixture is filled into suitably sized two-piece hard gelatin capsules using suitable machinery and methods well known in the art. See *Remington's Pharmaceutical Sciences,* 16th or 18th Editions, each incorporated herein in its entirety by reference thereto. Other doses may be prepared by altering the fill weight and, if necessary, by changing the capsule size to suit. Any of the stable hard gelatin capsule formulations above may be formed.

### 6.4.2 Compressed Tablet Dosage Forms

The ingredients of compressed tablet forms of the pharmaceutical compositions of the invention may be found in U.S. Patent Nos. 6,342,533 B1, 6,441,048 B1 and 6,911,479 B2.

The active ingredient is sieved through a suitable sieve and blended with the excipients until a uniform blend is formed. The dry blend is screened and blended with the magnesium stearate. The resulting powder blend is then compressed into tablets of desired shape and size. Tablets of other strengths may be prepared by altering the ratio of the active ingredient to the excipient(s) or modifying the tablet weight.

### 6.4.3 Example of a capsule formulation

| | 50 mg | 100 mg |
|---|---|---|
| | (mg/capsule) | (mg/capsule) |
| (-)-O-desmethylvenlafaxine hydrochloride monohydrate | 60.34 | 120.68 |
| Microcrystalline cellulose (Avicel PH102) | 60.00 | 19.02 |
| Lactose, Anhydrous | 160.16 | 103.40 |
| Sodium Starch Glycolate (Primojel) | 18.00 | 15.60 |
| Magnesium Stearate | 1.50 | 1.30 |
| Total Mass | 300.0 | 260.0 |

### 6.4.4 A delayed release formulation

Several Delayed release formulations have been examined. It was found the addition of more Methocel K4M CR decreased the dissolution rate. Tablets have been manufactured using the formulations outlined below.

### Premix Granulation

| Ingredients | Premix Formula |
|---|---|
| SEP-227162-01 | 605 |
| Avicel pH 102 | 60.5 |
| Surelease (15% w/w) | 21.42 |

### Final Formulations

| Ingredients | Formulation A (mg) | Formulation B (mg) | Formulation C (mg) |
|---|---|---|---|
| Premix | 687.00 | 687.00 | 687.00 |
| Methocel K4M CR | 30.25 | 60.5 | 121.00 |
| Mag. Stearate | 7.00 | 7.00 | 8.00 |
| **Tablet weight** | **724.25** | **754.50** | **816.00** |

### 6.5 EXAMPLE 4: CRYSTALLIZATION AND CHARACTERIZATION OF FORM A OF THE HYDROCHLORIDE SALT OF (-)-O-DESMETHYLVENLAFAXINE

### 6.5.1 Crystallization

(-)-O-desmethylvenlafaxine was crystallized as Form A of the HCl salt of (-)-O-desmethylvenlafaxine. The freebase of (-)-O-desmethylvenlafaxine was prepared according to Example 1. Form A of the HCl salt of (-)-O-desmethylvenlafaxine was prepared from Form B of (-)-O-desmethylvenlafaxine HCl salt, described below, according to the following procedure: A 3.09 gram sample of (-)-O-desmethylvenlafaxine hydrochloride salt (form B) was placed in a 70 X 50 mm crystallization dish and stored at 40°C/75%RH for 3 days. The sample was then dried under vacuum at ambient temperature for 2 days.

The Form A crystal form of the HCl salt of (-)-O-desmethylvenlafaxine prepared according to the procedure above was characterized by analytical techniques including thermal gravimetric analysis, differential scanning calorimetry, X-ray powder diffraction, moisture sorption, infrared spectroscopy and Raman spectroscopy, according to the analytical parameters described supra.

### 6.5.1.1 Single crystal X-ray diffraction data of Form A

Crystals of Form A of the HCl salt of (-)-O-desmethylvenlafaxine suitable for single crystal X-ray diffraction were prepared by solvent/antisolvent techniques from a water/2-methyl-tetrahydrofuran solvent system. Single-crystal X-ray diffraction analysis was performed using a Nonius Kappa CCD diffractometer with Mo *Kα* radiation (λ = 0.71073 A). Refined mosaicity was obtained using DENZO/SCALEPACK (Otwinowski and Minor, Methods Enzymol. 276:307 (1997)). The space group was determined using the program XPREP (Bruker AXS Inc., Madison, Wisconsin, USA, (2002)). Data integration was performed with DENZO-SMN (Otwinowski and Minor, Methods Enzymol. 276:307 (1997)). An empirical absorption correction was applied, obtained using SCALEPACK (Otwinowski and Minor, Methods Enzymol. 276: 307 (1997)). The structure was solved by direct methods using SIR2004 (Burla et al., J. Appl. Cryst., 36:1103 (2003)), and refinements were performed on an LINUX PC using SHELX97 (Sheldrick, University of Göttingen, Germany, (1997)). The absolute configuration of the (-)-O-desmethylvenlafaxine molecule was deduced using information from the structure solution of another crystal form (Form F, described below) obtained using the same (-)-O-desmethylvenlafaxine starting material. Data collection and structure parameter details are shown in Table I.

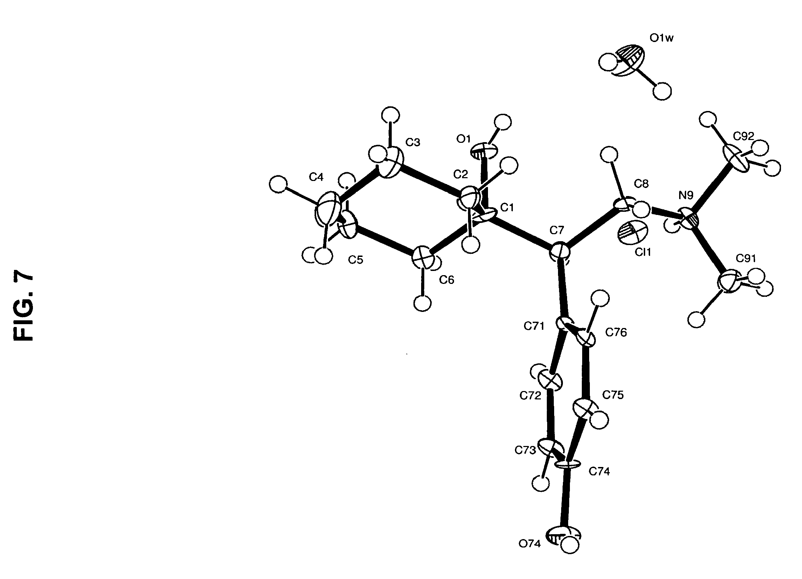

An ORTEP drawing of the asymmetric unit from the single crystal structure solution of the Form A crystal form of the HCl salt of (-)-O-desmethylvenlafaxine is shown in FIG. 7 (*ORTEP-3 for Windows*, v. 1.05. Farrugia, J. Appl. Cryst., 30:565 (1997)). The asymmetric unit shown in the drawing contains one (-)-O-desmethylvenlafaxine cation, one chloride anion and one water molecule.

**Table I. Crystal Data and Data Collection Parameters for Form A of the HCl salt of (-)-O-desmethylvenlafaxine.**

| | |
|---|---|
| formula | C₁₆H₂₈ClNO₃ |
| formula weight | 317.85 |
| space group | *P*2₁2₁2₁ (No. 19) |
| Unit cell dimensions | a = 6.7797(2) A; α = 90°. |
| | b = 9.2896(4) A; β = 90°. |
| | c = 27.6496(15) A; γ = 90°. |
| Volume | 1741.39(13) Å³ |
| Z | 4 |
| d_{calc}, g cm⁻³ | *1.212 |
| crystal dimensions, mm | 0.46 x 0.13 x 0.04 |
| temperature, K | 150 |
| radiation (wavelength, Å) | Mo Kₐ (0.71073) |
| monochromator | graphite |
| linear abs coef, mm⁻¹ | 0.226 |
| absorption correction applied | empirical^{a} |
| transmission factors: min, max | 0.916, 0.992 |
| diffractometer | Nonius Kappa CCD |
| *h, k, l* range | -8 to 7 -11 to 11 -33 to 34 |
| 29 range, deg | 4.38-52.21 |
| mosaicity, deg | 0.38 |
| programs used | SHELXTL |
| *F*₀₀₀ | 688.0 |
| weighting | |
| 1/[σ²(Fₒ²)⁺(0.0000*P*)²+1.9052*P*] where *P*=( *F*ₒ²+2*F*_{c}²)/3 | |
| data collected | 11326 |
| unique data | 2273 |
| *R*ᵢₙₜ | 0.155 |
| data used in refinement | 2273 |
| cutoff used in R-factor calculations | *F*ₒ²>2.0sigma(*F*ₒ²) |
| data with />2.0sigma(*l*) | 2018 |
| number of variables | 208 |
| largest shift/esd in final cycle | 0.00 |
| *R*(*F*ₒ) | 0.071 |
| *R*_{w}(*F*ₒ²) | 0.105 |
| goodness of fit | 1.225 |
| absolute structure determination | Flack parameter (0.1(2)) |

A simulated X-ray powder diffraction pattern was generated for Cu radiation using PowderCell 2.3 (Kraus and Nolze, Federal Institute for Materials Research and Testing, Berlin, Germany, (1999)) with the atomic coordinates, space group, and unit cell parameters from the single crystal data of Form A; see FIG. 8. The Form A experimental X-ray powder diffraction pattern matched the pattern simulated from the single crystal X-ray diffraction data. Slight shifts in XRPD peak location resulted from small changes in the unit cell parameters due to temperature differences: the calculated X-ray powder diffraction pattern was generated from the single crystal data which was collected at 150 K, while the experimental powder pattern was collected at ambient temperature. Collecting data at low temperature is typically used in single crystal analysis to improve the quality of the data.

### REFERENCE

### 6.6 EXAMPLE 5: CRYSTALLIZATION AND CHARACTERIZATION OF FORM B OF THE HYDROCHLORIDE SALT OF (-)-O-DESMETHYLVENLAFAXINE

### 6.6.1 Crystallization

(-)-O-desmethylvenlafaxine was crystallized as Form B of the HCl salt of (-)-O-desmethylvenlafaxine. (-)-O-Desmethylvenlafaxine was prepared according to Example 1. 5.07 g of the HCl salt of (-)-O-desmethylvenlafaxine was dissolved in 400 mL of tetrahydrofuran at 40 °C. The solution was cooled to 25 °C and 10.6 mL of 2.0 M HCl in diethyl ether was added. The mixture was cooled to 0 °C and filtered. The cake was washed with 20 mL of THF and dried in vacuo at ambient temperature to yield 6.09 g of Form B of 1-(2-(dimethylamino)-1-(4-hydroxyphenyl)ethyl) cyclohexanol hydrochloride.

### 6.6.2 Characterization

The Form B crystal form of the HCl salt of (-)-O-desmethylvenlafaxine prepared according to the procedure above was characterized by techniques such as X-ray powder diffraction, differential scanning calorimetry, thermal gravimetric analysis, moisture sorption, infrared spectroscopy and Raman spectroscopy, according to the analytical parameters described above.

### REFERENCE

### 6.7 EXAMPLE 6: CRYSTALLIZATION AND CHARACTERIZATION OF FORM C OF THE HYDROCHLORIDE SALT OF (-)-O-DESMETHYLVENLAFAXINE

### 6.7.1 Crystallization

(-)-O-Desmethylvenlafaxine was crystallized as Form C of the HCl salt of (-)-O-desmethylvenlafaxine. (-)-O-Desmethylvenlafaxine was prepared according to Example 1. 0.18 g of (-)-O-desmethylvenlafaxine and 0.35 mL of 37 wt% aqueous hydrochloric acid were mixed at 60 °C for 1 h. The mixture was cooled to 0 °C, filtered and washed with ethyl acetate. The solid was dried in vacuo at ambient temperature to yield 0.22 g of 1-(2-(dimethylamino)-1-(4-hydroxyphenyl)ethyl) cyclohexanol hydrochloride.

### 6.7.2 Characterization

The Form C crystal form of the HCl salt of (-)-O-desmethylvenlafaxine prepared according to the procedure above was characterized by techniques such as X-ray powder diffraction, differential scanning calorimetry, thermal gravimetric analysis, moisture sorption, infrared spectroscopy and Raman spectroscopy, according to the analytical parameters described above.

### REFERENCE

### 6.8 EXAMPLE 7: CRYSTALLIZATION AND CHARACTERIZATION OF FORM D OF THE HYDROCHLORIDE SALT OF (-)-O-DESMETHYLVENLAFAXINE

### 6.8.1 Crystallization

(-)-O-desmethylvenlafaxine was crystallized as Form D of the HCl salt of (-)-O-desmethylvenlafaxine. (-)-O-desmethylvenlafaxine was prepared according to Example 1. Form A (42.8 mg) of (-)-O-desmethylvenlafaxine HCl salt, obtained as described in Example 4, was weighed into a vial, and 0.5 mL of IPA was added. The sample was sonicated, and became very thick. The solids were isolated by vacuum filtration, and the sample was air dried in a hood. After a day of drying, the sample was stored at ambient conditions for four days, at which point the XRPD analysis was performed.

### 6.8.2 Characterization

The Form D crystal form of the HCl salt of (-)-O-desmethylvenlafaxine prepared according to the procedure above was characterized by techniques such as X-ray powder diffraction, differential scanning calorimetry and thermal gravimetric analysis, according to the analytical parameters described above.

### REFERENCE

### 6.9 EXAMPLE 8: CRYSTALLIZATION AND CHARACTERIZATION OF FORM E OF THE HYDROCHLORIDE SALT OF (-)-O-DESMETHYLVENLAFAXINE

### 6.9.1 Crystallization

(-)-O-Desmethylvenlafaxine was crystallized as Form E of the HCl salt of (-)-O-desmethylvenlafaxine. (-)-O-Desmethylvenlafaxine was prepared according to Example 1. 0.35 mL of 37 wt% aqueous hydrochloric acid was added to 5.0 g of (-)-O-desmethylvenlafaxine in 25 mL of methanol. The resulting solution was stirred at 25 °C for 20 minutes. The methanol/hydrochloric acid solution was added with stirring to 300 mL of methyl-tert butyl ether at 25 °C. Following the addition of the methanol/hydrochloric acid solution the mixture was stirred at 25 °C for 2 hours and then the solid was collected by filtration and washed with 20 mL of MTBE. The solid was air dried at ambient temperature to yield 5.4 g of Form E of 1-(2-(dimethylamino)-1-(4-hydroxyphenyl)ethyl)cyclohexanol hydrochloride.

### 6.9.2 Characterization

The Form E crystal form of the HCl salt of (-)-O-desmethylvenlafaxine prepared according to the procedure above was characterized by techniques such as X-ray powder diffraction, differential scanning calorimetry, thermal gravimetric analysis, moisture sorption, infrared spectroscopy and Raman spectroscopy, according to the analytical parameters described above.

### REFERENCE

### 6.10 EXAMPLE 9: CRYSTALLIZATION AND CHARACTERIZATION OF FORM F OF THE HCL SALT OF (-)-O-DESMETHYLVENLAFAXINE

### 6.10.1 Crystallization

Form A of the HCl salt of (-)-O-desmethylvenlafaxine (19.47 mg) was weighed into a vial, and 3 mL of ethyl acetate was added. Solids remained after sonication. The sample was placed on a hot plate set at 75 °C, and stirred using a magnetic stirrer set at 350 rpm. After approximately 2.5 hours of stirring at 75 °C, the sample was syringe filtered into a warm, 1-dram vial. (Prior to filtering, the filter, syringe, and vial were warmed on the hot plate with the sample.) The sample was capped, set on the bench top, and allowed to cool to ambient temperature. The sample was vacuum filtered and analyzed as Form F.

### 6.10.2 Characterization

The Form F crystal form of the HCl salt of (-)-O-desmethylvenlafaxine prepared according to the procedure above was characterized by techniques such as X-ray powder diffraction, differential scanning calorimetry, thermal gravimetric analysis, moisture sorption, infrared spectroscopy and Raman spectroscopy, according to the analytical parameters described above.

### 6.10.2.1 Single crystal X-ray diffraction data of Form F

Crystals of Form F of the HCl salt of (-)-O-desmethylvenlafaxine suitable for single crystal X-ray diffraction were prepared by a vapor diffusion technique. Three milliliters of 2-butanone were added to 7.71 mg of Form A, obtained as described above. Not all of the solids dissolved. The sample was filtered into a 1 dram vial. The vial was placed into a 20 mL scintillation vial containing toluene. The larger vial was then capped, and the sample was allowed to equilibrate. Single crystals of Form F were isolated, and the structure was solved.

Single-crystal X-ray diffraction analysis was performed using a Bruker D8 APEX II CCD sealed tube diffractometer with Cu *Kα* radiation (λ = 1.54178 A). Data collection, indexing and initial cell refinements were all carried out using the software APEX II (Bruker AXS, Inc., Madison, WI, USA, (2005)). Frame integration and final cell refinements were done using the software SAINT (v. 6.45A, Bruker AXS, Inc., Madison, WI, USA (2003)). The space group was determined by the program XPREP (SHELXTL v. 6.12, Bruker AXS, Inc., Madison, WI, USA). An empirical absorption correction was applied using SADABS (Blessing, Acta Cryst., A51:33 (1995)). The structure was solved by direct methods using SHELXS-97 (Sheldrick, University of Göttingen, Germany, (1997)). Refinements were performed on a PC using SHELXTL (v. 6.12, Bruker AXS, Inc., Madison, WI, USA). The absolute configuration of the (-)-O-desmethylvenlafaxine molecule was deduced by assessing the Flack factor (Flack and Bernardinelli, Acta Cryst., A55: 908 (1999), and J. Appl. Cryst., 33:1143 (2000)). Data collection and structure parameter details are shown in Table 2.

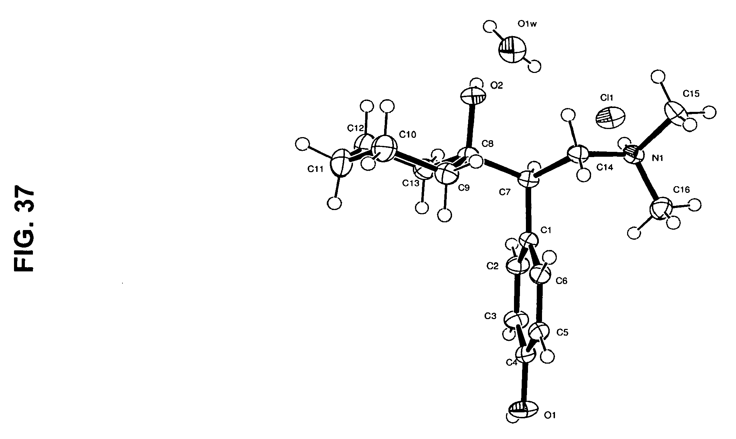

An ORTEP drawing of the asymmetric unit from the single crystal structure solution of the Form F crystal form of the HCl salt of (-)-O-desmethylvenlafaxine is shown in FIG. 37 (ORTEP-3 for Windows, v. 1.05. Farrugia, J. Appl. Cryst., 30:565 (1997)). The asymmetric unit shown in the drawing contains one (-)-O-desmethylvenlafaxine cation, one chloride anion and one water of hydration.

**Table 2. Crystal Data and Data Collection Parameters for Form F of the HCl salt of (-)-O-desmethylvenlafaxine.**

| | | |
|---|---|---|
| Empirical formula | C₁₆H₂₈ClNO₃ | |
| formula weight | 317.85 | |
| Temperature | 173(2) K | |
| Wavelength | 1.54178 Å | |
| Crystal system | Monoclinic | |
| Space group | P2(1) | |
| Unit cell dimensions | a = 9.2881 (2) Å; | α= 90°. |
| | b = 6.8185(2) Å; | β= 92.580(1)° |
| | c = 13.9085(3) Å; | γ = 90°. |
| Volume | 879.95(4) Å³ | |
| Z | 2 | |
| Density (calculated) | 1.200 Mg/m³ | |
| Absorption coefficient | 1.996 mm⁻¹ | |
| F(000) | 344 | |
| Crystal size | 0.43 x 0.25 x 0.18 mm³ | |
| Theta range for data collection | 8.07 to 65.77°. | |
| Index ranges | -10<=h<=10, -6<=k<=6, -16<=l<=15 | |
| Reflections collected | 3464 | |
| Independent reflections | 1722 [R(int) = 0.0131] | |
| Completeness to theta = 65.77° | 76.1 % | |
| Absorption correction | Semi-empirical from equivalents | |
| Max. and min. transmission | 0.7152 and 0.4807 | |
| Refinement method | Full-matrix least-squares on F² | |
| Data / restraints / parameters | 1722 / 1 / 194 | |
| Goodness-of-fit on F² | 1.034 | |
| Final R indices [I>2sigma(I)] | R1 = 0.0265, wR2 = 0.0714 | |
| R indices (all data) | R1 = 0.0268, wR2 = 0.0716 | |
| Absolute structure parameter | 0.034(13) | |
| Largest diff. peak and hole | 0.129 and -0.185 e.Å⁻³ | |

A simulated X-ray powder diffraction pattern was generated for Cu radiation using PowderCell 2.3 (Kraus and Nolze, Federal Institute for Materials Research and Testing, Berlin, Germany, (1999)) and the atomic coordinates, space group, and unit cell parameters from the single crystal data of Form F; see FIG. 33. The Form F experimental X-ray powder diffraction pattern matched the pattern simulated from the single crystal X-ray diffraction data. Differences in intensities may have resulted from preferred orientation. Preferred orientation is the tendency for crystals, usually plates or needles, to align in a non-random manner. Preferred orientation affects peak intensities in X-ray powder diffraction patterns. Slight shifts in peak location may have resulted from experimental temperature differences: the experimental powder pattern was collected at ambient temperature, while the single crystal data was collected at 173 K. Certain Form F samples which were isolated as physical mixtures with Form A exhibited peaks characteristic of Form A in the XRPD pattern, which were not present in the simulated Form F XRPD pattern.

### REFERENCE

### 6.11 EXAMPLE 10: CRYSTALLIZATION AND CHARACTERIZATION OF FORM G OF THE HYDROCHLORIDE SALT OF (-)-O-DESMETHYLVENLAFAXINE

### 6.11.1 Crystallization

(-)-O-desmethylvenlafaxine was crystallized as Form G of the HCl salt of (-)-O-desmethylvenlafaxine. (-)-O-desmethylvenlafaxine was prepared according to Example 1. The Form A crystal form of (-)-O-desmethylvenlafaxine (31.50 mg), prepared according to Example 4, was placed into a 20 mL scintillation vial, which was placed, uncapped, into a P₂O₅ chamber at ambient temperature. After three days, the chamber containing the sample was placed into a 70 °C oven. Analysis performed ten days after the sample was placed in the oven indicated that the sample was Form G.

### 6.11.2 Characterization

The Form G crystal form of the HCl salt of (-)-O-desmethylvenlafaxine prepared according to the procedure above was characterized by techniques such as X-ray powder diffraction, differential scanning calorimetry, thermal gravimetric analysis and moisture sorption, according to the analytical parameters described above.

### REFERENCE

### 6.12 EXAMPLE 11: CRYSTALLIZATION AND CHARACTERIZATION OF FORM H OF THE HYDROCHLORIDE SALT OF (-)-O-DESMETHYLVENLAFAXINE

### 6.12.1 Crystallization

(-)-O-desmethylvenlafaxine was crystallized as Form H of the HCl salt of (-)-O-desmethylvenlafaxine. (-)-O-desmethylvenlafaxine was prepared according to Example 1. Form H was prepared by slurring Form A of the HCl salt of (-)-O-desmethylvenlafaxine in acetone on a hot plate set at 55 °C. The samples were stirred in half dram vials on the hot plate using a magnetic stirrer set at 300 rpm. In each case, 0.5 mL of acetone was used. One sample contained 42.13 mg of the HCl salt of (-)-O-desmethylvenlafaxine, and was slurried for three days prior to isolation of Form H. A second sample was filtered after one day, and contained 48.13 mg of the HCl salt of (-)-O-desmethylvenlafaxine. A third sample, slurried for an unspecified time, contained 41.91 mg of the HCl salt of (-)-O-desmethylvenlafaxine. The solids thus obtained were characterized as the Form H crystal form of the HCl salt of (-)-O-desmethylvenlafaxine.

### 6.12.2 Characterization

The Form H crystal form of the HCl salt of (-)-O-desmethylvenlafaxine prepared according to the procedure above was characterized by techniques such as X-ray powder diffraction, differential scanning calorimetry and thermal gravimetric analysis, according to the analytical parameters described above.

### REFERENCE

### 6.13 EXAMPLE 12: CRYSTALLIZATION AND CHARACTERIZATION OF FORM I OF THE HYDROCHLORIDE SALT OF (-)-O-DESMETHYLVENLAFAXINE

### 6.13.1 Crystallization

(-)-O-desmethylvenlafaxine was crystallized as Form I of the HCl salt of (-)-O-desmethylvenlafaxine. (-)-O-desmethylvenlafaxine was prepared according to Example 1. Form I was precipitated from isopropanol. One sample was prepared by dissolving 46.01 mg of Form A of the HCl salt of (-)-O-desmethylvenlafaxine in 0.5 mL isopropanol using sonication. The sample was prepared in a 1-dram vial. Precipitation was observed after approximately 10-15 minutes. The solids were isolated by vacuum filtration. The second sample was prepared using the procedure described for the first sample, except that 25.86 mg of Form A of the HCl salt of (-)-O-desmethylvenlafaxine was dissolved. Precipitation for this second sample was observed after approximately ten minutes. Following synthesis, solids were isolated and characterized as the Form I crystal form of the HCl salt of (-)-O-desmethylvenlafaxine.

### 6.13.2 Characterization

The Form I crystal form of the HCl salt of (-)-O-desmethylvenlafaxine prepared according to the procedure above was characterized by techniques such as X-ray powder diffraction, differential scanning calorimetry and thermal gravimetric analysis, according to the analytical parameters described above.

### REFERENCE

### 6.14 EXAMPLE 13: CRYSTALLIZATION AND CHARACTERIZATION OF FORM J OF THE HYDROCHLORIDE SALT OF (-)-O-DESMETHYLVENLAFAXINE

### 6.14.1 Crystallization

(-)-O-desmethylvenlafaxine was crystallized as Form J of the HCl salt of (-)-O-desmethylvenlafaxine. (-)-O-desmethylvenlafaxine was prepared according to Example 1. Form J of the HCl salt of (-)-O-desmethylvenlafaxine was prepared by slurring Form A in acetonitrile for approximately one day on a hot plate set at 55 °C. Form A of the HCl salt of (-)-O-desmethylvenlafaxine (43.66 mg) was weighed into a 1-dram vial, and 0.5 mL of acetonitrile was added. Solids remained after sonication. The sample was stirred on the hot plate using a magnetic stirrer set at 300 rpm. After a day, the solvent was decanted. The solids thus obtained were characterized as the Form J crystal form of the HCl salt of (-)-O-desmethylvenlafaxine.

### 6.14.2 Characterization

The Form J crystal form of the HCl salt of (-)-O-desmethylvenlafaxine prepared according to the procedure above was characterized by techniques such as X-ray powder diffraction, and NMR spectroscopy, according to the analytical parameters described above. About 0.2 mole of acetonitrile per mole of the HCl salt of (-)-O-desmethylvenlafaxine was present in a Form J sample, as observed using NMR spectroscopy.

### REFERENCE

### 6.15 EXAMPLE 14: CRYSTALLIZATION AND CHARACTERIZATION OF FORM K OF THE HYDROCHLORIDE SALT OF (-)-O-DESMETHYLVENLAFAXINE.

### 6.15.1 Crystallization

(-)-O-desmethylvenlafaxine was crystallized as Form K of the HCl salt of (-)-O-desmethylvenlafaxine. (-)-O-desmethylvenlafaxine was prepared according to Example 1. Form K of the HCl salt of (-)-O-desmethylvenlafaxine was prepared from a vapor diffusion experiment using ethanol as the solvent and acetone as the antisolvent. The sample was prepared by adding 0.3 mL of ethanol to 22.20 mg of Form A of the HCl salt of (-)-O-desmethylvenlafaxine. The sample dissolved and was filtered into a 1-dram vial. The vial was placed into a 20 mL scintillation vial containing acetone. The larger vial was then capped, and the sample was allowed to equilibrate. Single crystals were isolated from this experiment. The crystals thus obtained were characterized as the Form K crystal form of the HCl salt of (-)-O-desmethylvenlafaxine.

### 6.15.2 Characterization

The Form K crystal form of the HCl salt of (-)-O-desmethylvenlafaxine prepared according to the procedure above was characterized by techniques such as X-ray powder diffraction, and single-crystal X-ray diffraction, according to the analytical parameters described above.

### 6.15.2.1 Single crystal X-ray diffraction data of Form K

Crystals of Form K of the HCl salt of (-)-O-desmethylvenlafaxine suitable for single crystal X-ray diffraction were prepared by the technique described above. Single-crystal X-ray diffraction analysis was performed using a Bruker D8 APEX II CCD sealed tube diffractometer with Cu *Kα* radiation (A = 1.54178 A). Data collection, indexing and initial cell refinements were all carried out using the software APEX II (Bruker AXS, Inc., Madison, WI, USA (2005)). Frame integration and final cell refinements were done using the software SAINT (v. 6.45A, Bruker AXS, Inc., Madison, WI, USA (2003)). The space group was determined by the program XPREP (SHELXTL v. 6.12, Bruker AXS, Inc., Madison, WI, USA). An empirical absorption correction was applied using SADABS (Blessing, Acta Cryst., A51:33 (1995)). The structure was solved by direct methods using SHELXS-97 (Sheldrick, University of Göttingen, Germany, (1997)). Refinements were performed on a PC using SHELXTL (v. 6.12, Bruker AXS, Inc., Madison, WI, USA). The absolute configuration of the (-)-O-desmethylvenlafaxine molecule was deduced by assessing the Flack factor (Flack and Bernardinelli, Acta Cryst., A55:908 (1999), and J. Appl. Cryst., 33:1143 (2000)). Data collection and structure parameter details are shown in Table 3.

The complete contents of the asymmetric unit of the crystal structure of Form K includes two (-)-O-desmethylvenlafaxine cations, two chloride anions and one partially occupied, highly disordered ethanol molecule. Since the ethanol molecule is not fully occupied, Form K is termed a partial ethanol solvate.

**Table 3. Crystal Data and Data Collection Parameters for Form K of the HCl salt of (-)-O-desmethylvenlafaxine.**

| | | |
|---|---|---|
| Empirical formula | C₁₆H₂₆ClNO₂ •0.14(C₂H₆O) | |
| Formula weight | 306.33 | |
| Temperature | 173(2) K | |
| Wavelength | 1.54178 Å | |
| Crystal system | Monoclinic | |
| Space group | C2 | |
| Unit cell dimensions | a = 30.056(3) Å; | α = 90°. |
| | b = 7.7375(8) Å; | β = 134.502(4)°. |
| | c = 21.208(4) Å; | γ = 90°. |
| Volume | 3517.7(8) Å3 | |
| Z | 8 | |
| Density (calculated) | 1.157 Mg/m³ | |
| Absorption coefficient | 1.944 mm⁻¹ | |
| F(000) | 1322 | |
| Crystal size | 0.53 x 0.08 x 0.06 mm³ | |
| Theta range for data collection | 7.37 to 44.67°. | |
| Index ranges | -27<=h<=25, -7<=k<=7, -19<=I<=19 | |
| Reflections collected | 2985 | |
| Independent reflections | 2063 [R(int) = 0.0413] | |
| Completeness to theta = 44.67° | 92.1 % | |
| Absorption correction | Semi-empirical from equivalents | |
| Max. and min. transmission | 0.8923 and 0.4256 | |
| Refinement method | Full-matrix least-squares on F² | |
| Data / restraints / parameters | 2063 / 2 / 378 | |
| Goodness-of-fit on F² | 1.060 | |
| Final R indices [I>2sigma(I)] | R1 = 0.0518, wR2 = 0.1391 | |
| R indices (all data) | R1 = 0.0800, wR2 = 0.1571 | |
| Absolute structure parameter | 0.01(4) | |
| Largest diff. peak and hole | 0.464 and -0.545 e.Å⁻³ | |

A simulated X-ray powder diffraction pattern was generated for Cu radiation using PowderCell 2.3 (Kraus and Nolze, Federal Institute for Materials Research and Testing, Berlin, Germany (1999)) and the atomic coordinates, space group, and unit cell parameters from the single crystal data of Form K; see FIG. 50. The Form K experimental X-ray powder diffraction pattern matched the pattern simulated from the single crystal X-ray diffraction data. Differences in intensities may have resulted from preferred orientation. Slight shifts in peak location may have resulted from experimental temperature differences: the experimental powder pattern was collected at ambient temperature, while the single crystal data was collected at 173 K.

### REFERENCE

### 6.16 EXAMPLE 15: CRYSTALLIZATION AND CHARACTERIZATION OF FORM L OF THE HYDROCHLORIDE SALT OF (-)-O-DESMETHYLVENLAFAXINE

### 6.16.1 Crystallization

(-)-O-desmethylvenlafaxine was crystallized as Form L of the HCl salt of (-)-O-desmethylvenlafaxine. (-)-O-desmethylvenlafaxine was prepared according to Example 1. Form L was prepared from a prolonged ambient temperature slurry in 2-methyl-tetrahydrofuran. The sample was prepared by adding 20 mL of 2-methyl-tetrahydrofuran to 38.75 mg of Form A of the HCl salt of (-)-O-desmethylvenlafaxine. A 20 mL scintillation vial was used for the experiment, and the 2-methyl-tetrahydrofuran was added slowly. Solids were present after the solvent addition, and the sample was capped and placed on a rotating wheel at ambient temperature. After 97 days on the wheel, the sample of Form L was removed, vacuum filtered, and submitted for analysis. The solids thus obtained were characterized as the Form L crystal form of the HCl salt of (-)-O-desmethylvenlafaxine.

### 6.16.2 Characterization

The Form L crystal form of the HCl salt of (-)-O-desmethylvenlafaxine prepared according to the procedure above was characterized by techniques such as X-ray powder diffraction, differential scanning calorimetry, thermal gravimetric analysis and NMR spectroscopy, according to the analytical parameters described above. Between about 0.13 and 0.14 mole of 2-methyl-tetrahydrofuran per mole of the HCl salt of (-)-O-desmethylvenlafaxine was present in a Form L sample, as observed using NMR spectroscopy.

### REFERENCE

### 6.17 EXAMPLE 16: PREPARATION AND CHARACTERIZATION OF A DESOLVATED SOLVATE FORM OF A HYDROCHLORIDE SALT OF (-)-O-DESMETHYLVENLAFAXINE.

### 6.17.1 Preparation

Form C of the HCl salt of (-)-O-desmethylvenlafaxine was prepared as described above. Form C was heated to 100 °C in a TGA furnace, according to the procedure described above, and a weight loss of 5.4% was observed. The material was removed from the furnace; analysis confirmed that the material was a desolvated solvate.

### 6.17.2 Characterization

The desolvated solvate was analyzed by X-ray powder diffraction. The locations of the peaks in the X-ray powder diffraction pattern of the desolvated solvate were similar to the locations of the XRPD peaks in the Form C starting material. This data, in conjunction with the TGA weight loss data, indicated that the solvent evacuated the crystal lattice of Form C while maintaining structural features of Form C in the form of a desolvated solvate.

### REFERENCE

### 6.18 EXAMPLE 17: PREPARATION AND CHARACTERIZATION OF AN AMORPHOUS FORM OF A HYDROCHLORIDE SALT OF (-)-O-DESMETHYLVENLAFAXINE

### 6.18.1 Preparation

(-)-O-desmethylvenlafaxine was prepared as an amorphous form of the HCl salt of (-)-O-desmethylvenlafaxine. An aqueous solution of the HCl salt of (-)-O-desmethylvenlafaxine was prepared, filtered and frozen. The sample was then placed under vacuum on a freeze dryer and lyophilized until all solvent had been removed.

### 6.18.2 Characterization

The resulting product was characterized by X-ray powder diffraction and modulated differential scanning calorimetry. XRPD data confirmed that the material was amorphous. Based on modulated differential scanning calorimetry data, the glass transition temperature of the amorphous form of the HCl salt of (-)-O-desmethylvenlafaxine was approximately 24 °C.

### 6.19 EXAMPLE 18: COMPOSITION OF A DELAYED-RELEASE FORMULATION COMPRISING A HYDROCHLORIDE SALT OF (-)-O-DESMETHYLVENLAFAXINE

(-)-O-Desmethylvenlafaxine HCl and Avicel were mixed inside the vertical granulator. Pharmacoat 606 was slowly added to the blend. The wet mass was then tray dried at 45 °C for 2 hours and the semidried blend was then passed through a Fitzmill using screen size 0109 @ 2000 rpm. The particles were again put back to the dryer. The dried granules were screened through mesh # 14 and retain on screen #14 was passed through Fitzmill. The milled particles were mixed with finer screened particles. The Premix Formulation is summarized in Table 4. Using this Premix, the Final Formulation was developed, summarized in Table 5.

**Table 4. Formulation of Premix**

| Ingredient | Quantity (mg) |
|---|---|
| API: (-)-O-Desmethylvenlafaxine HCl Form A | 605 |
| Avicel 105 | 60.5 |
| Pharmacoat 606 (8% Solution) | 11.5 |

**Table 5. Final Formulation**

| Ingredient | Formula |
|---|---|
| Premix | 677 mg |
| Magnesium Stearate | 8 mg |
| Methocel K4M CR | 60.5 mg |

In another embodiment, API and Avicel were added to a high shear granulator and blended briefly. Surelease suspension was added drop-wise with the high shear process operating. The wet granulation was removed from the high shear granulator, dried in a fluid bed dryer, blended with Methocel and magnesium stearate, and compressed on a suitable tablet machine.

**Table 6. Formulation of Premix**

| Ingredient | Quantity (mg) |
|---|---|
| API: (-)-O-Desmethylvenlafaxine HCl form A | 484 |
| Avicel pH 102 | 320 |
| Surelase Suspension 20% w/w (dry wt/susp wt) | 80/400 |
| Total | 884 |

**Table 7. Matrix Tablets**

| | |
|---|---|
| 50 mg tablet | |
| | |

| Ingredient | Quantity (mg) |
|---|---|
| Premix | 110.5 |
| Magnesium Stearate | 1.5 |
| Methocel K15M CR | 213.0 |
| Total | 325.0 |
| | |
| 100 mg tablet | |
| | |

| Ingredient | Quantity (mg) |
|---|---|
| Premix | 221.0 |
| Magnesium Stearate | 3.0 |
| Methocel K 15M CR | 276.0 |
| Total | 500.0 |
| | |
| 150 mg tablet | |
| | |

| Ingredient | Quantity (mg) |
|---|---|
| Premix | 331.5 |
| Magnesium Stearate | 4.5 |
| Methocel K15M CR | 164.0 |
| Total | 500.0 |

## Claims

1. A crystal form of a hydrochloride salt of the stereomerically pure compound of formula (I): 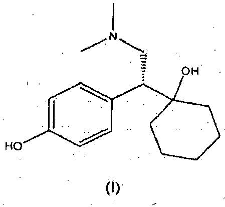 which contains water and which is Form A, wherein the molar ratio of water to (-)-O-desmethylvenlafaxine hydrochloride salt of Formula (I) is approximately one to one, and which Form A has an X-ray powder diffraction pattern comprising peaks at approximately 12.7, 14.5, 19.1, 21.4, 23.0, 25.5, and 27.3 °2θ using CuK_{α} radiation.

2. The crystal form of claim 1, wherein the water is present in an amount of about 6% by weight.

3. The crystal form of claim 1, which has a thermal gravimetric analysis thermogram comprising a weight loss of about 5.6% when heated from 25°C to 110°C.

4. The crystal form of claim 1, which has a differential scanning calorimetry thermogram comprising an endotherm with an onset temperature of between 50°C and 125°C.

5. The crystal form of claim 4, wherein the onset temperature is about 93°C.

6. The crystal form of claim 1, which has the following approximate unit cell parameters when measured at approximately 150 K; a = 6.78 Å; *b* = 9.29 Å; c = 27.65 Å; *α* = 90°; *β* = 90°; *γ* = 90°.

7. A pharmaceutical composition comprising the crystal form of any of claims 1 to 6, and a pharmaceutically acceptable diluent, excipient, or carrier.

8. The pharmaceutical composition of claim 7 for use in treating, preventing, or managing a condition selected from depression, pain, anxiety, and incontinence.

## Patentansprüche

1. Kristallform eines Hydrochloridsalzes der stereomer reinen Verbindung der Formel (I): 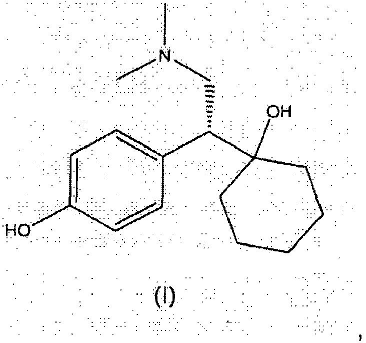 die Wasser enthält und welche Form A ist, wobei das molare Verhältnis von Wasser zu (-)-O-Desmethylvenlafaxin-Hydrochloridsalz der Formel (I) etwa 1:1 beträgt, und wobei die Form A ein Röntgenpulverbeugungsmuster aufweist, umfassend Peaks bei etwa 12,7, 14.5, 19,1, 21,4, 23,0, 25,5 und 27.3 °2θ unter Verwendung von CuK_{α}-Strahlung.

2. Kristallform nach Anspruch 1, wobei das Wasser in einer Menge von etwa 6 Gew% vorhanden ist.

3. Kristallform nach Anspruch 1, welche ein Thermogramm einer thermischen gravimetrischen Analyse aufweist, umfassend einen Gewichtsverlust von etwa 5,6% bei Erwärmung von 25 °C bis 110 °C.

4. Kristallform nach Anspruch 1, die ein Thermogramm einer Differential-Scanning-Kalorimetrie aufweist, welche eine Endotherme mit einer Onset-Temperatur zwischen 50 °C und 125 °C aufweist.

5. Kristallform nach Anspruch 4, wobei die Onset-Temperatur etwa 93 °C beträgt.

6. Kristallform nach Anspruch 1, welche die folgenden angenäherten Elementarzellenparameter aufweist, wenn bei etwa 150 K gemessen wird; a = 6,78 Å; b = 9,29 Å; c = 27,65 Å; α = 90°; β = 90°; γ = 90°.

7. Pharmazeutische Zusammensetzung, die die Kristallform nach einem der Ansprüche 1 bis 6 und ein pharmazeutisch verträgliches Verdünnungsmittel, einen Exzipient oder einen Träger umfasst.

8. Pharmazeutische Zusammensetzung nach Anspruch 7 zur Verwendung bei der Behandlung, Verhinderung oder zum Bewältigen eines Zustands ausgewählt aus Depression, Schmerz, Angst und Inkontinenz.

## Revendications

1. Forme cristalline d'un sel chlorhydrate d'un composé stéréomorphiquement pur de formule (I) : 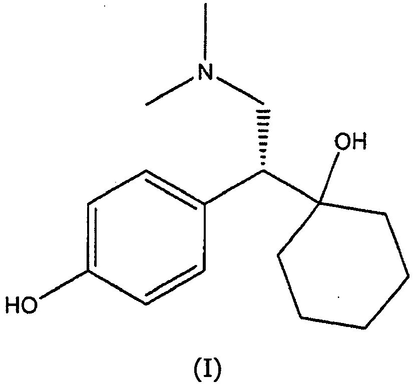 qui contient de l'eau et qui est la forme A, dans laquelle le rapport molaire de l'eau au sel chlorhydrate de (-)-O-desméthylvenlafaxine de formule (I) est d'environ un pour un, laquelle forme A a un motif de diffraction des rayons X par la technique des poudres comprenant des pics à environ 12,7, 14,5, 19,1, 21,4, 23,0, 25,5 et 27,3 °2θ, par utilisation du rayonnement CuKα.

2. Forme cristalline selon la revendication 1, dans laquelle l'eau est présente en une quantité d'environ 6 % en poids.

3. Forme cristalline selon la revendication 1, qui a un thermogramme d'analyse thermogravimétrique comprenant une perte de poids d'environ 5,6 % lors d'un chauffage de 25°C à 110°C.

4. Forme cristalline selon la revendication 1, qui a un thermogramme de calorimétrie à balayage différentiel comprenant un endotherme avec une température de début comprise entre 50°C et 125°C.

5. Forme cristalline selon la revendication 4, dans laquelle la température de début est d'environ 93°C.

6. Forme cristalline selon la revendication 1, qui a les paramètres de cellule unitaire approximatifs suivants lors d'une mesure à environ 150 K ; a = 6,78 Å ; b = 9,29 Å ; c = 27,65 Å ; α = 90° ; β = 90° ; γ = 90°.

7. Composition pharmaceutique comprenant la forme cristalline de l'une quelconque des revendications 1 à 6, et un diluant, excipient ou véhicule pharmaceutiquement acceptable.

8. Composition pharmaceutique selon la revendication 7, pour utilisation dans le traitement, la prévention ou la gestion d'un état choisi parmi la dépression, la douleur, l'anxiété, et l'incontinence.
